(19) Europäisches Patentamt
European Patent Office
Office européen des brevets

(11) Veröffentlichungsnummer: **0 250 725 B1**

(12) **EUROPÄISCHE PATENTSCHRIFT**

(45) Veröffentlichungstag der Patentschrift: **04.11.92**

(51) Int. Cl.[5]: **C07D 401/12**, C07D 413/14, A61K 31/44, //C07D211/90

(21) Anmeldenummer: **87104601.7**

(22) Anmeldetag: **27.03.87**

Die Akte enthält technische Angaben, die nach dem Eingang der Anmeldung eingereicht wurden und die nicht in dieser Patentschrift enthalten sind.

# (54) 1,4-Dihydropyridinderivate, Verfahren zu ihrer Herstellung und diese Verbindungen enthaltende Arzneimittel.

(30) Priorität: **24.06.86 DE 3621104**

(43) Veröffentlichungstag der Anmeldung: **07.01.88 Patentblatt 88/01**

(45) Bekanntmachung des Hinweises auf die Patenterteilung: **04.11.92 Patentblatt 92/45**

(84) Benannte Vertragsstaaten: **AT BE CH DE ES FR GB GR IT LI LU NL SE**

(56) Entgegenhaltungen:
**EP-A- 0 041 359**
**EP-A- 0 106 462**
**EP-A- 0 119 050**
**DE-A- 2 407 115**
**DE-B- 2 211 454**

**Chem. Soc. Rev. 14, 375 (1985)**

**Pharmacie heute 104, 139 (1983)**

**B.J.R. Nicolaus in "Decision making in Drug Research" (Ed. F. Gross)Raven Press, New York, 1983, S. 173-188**

(73) Patentinhaber: **HEUMANN PHARMA GMBH & CO**
**Heideloffstrasse 18 - 28**
**W-8500 Nürnberg 1(DE)**

(72) Erfinder: **Schickaneder, Helmut Dr.**
**Moosäcker 25**
**W-8501 Eckental(DE)**
Erfinder: **Mörsdorf, Peter Dr.**
**Ziegelstrasse 64**
**W-8506 Langenzehn(DE)**
Erfinder: **Buschauer, Armin Dr.**
**Bachestrasse 5**
**W-1000 Berlin 41(DE)**
Erfinder: **Schunack, Walter, Prof.Dr.Dr.**
**Spanische Allee 95**
**W-1000 Berlin 38(DE)**
Erfinder: **Engler, Heidrun, Dr.**
**Ringstrasse 23**
**W-8501 Cadolzburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

Erfinder: **Vergin, Hartmut, Dr.**
**Ebenreuther Strasse 32**
**W-8500 Nürnberg 30(DE)**
Erfinder: **Ahrens, Kurt Hennings, Dr.**
**Praterstrasse 9**
**W-8500 Nürnberg(DE)**

(74) Vertreter: **Kraus, Walter, Dr. et al**
**Patentanwälte Kraus, Weisert & Partner**
**Thomas-Wimmer-Ring 15**
**W-8000 München 22(DE)**

## Beschreibung

Die Erfindung betrifft neue herz-kreislaufaktive Verbindungen und im besonderen 1,4-Dihydropyridine, die calciumantagonistische und/oder $H_2$-rezeptoragonistische Aktivitäten Zeigen und deshalb bestens geeignet sind, bei Erkrankungen des Herzens, bei bestimmten Formen der Hypertonie sowie bei arteriellen Verschlußkrankheiten eingesetzt werden zu können.

1,4-Dihydropyridincalciumantagonisten, wie zum Beispiel Nifedipin, sind bekannte Therapeutika, welche zum Beispiel den Calciumeinstrom in die Zelle hemmen.

So senkt das aus der Reihe der 1,4-Dihydropyridine bekannte Nifedipin (1,4-Dihydro-2,6-dimethyl-4-(2-nitrophenyl)-3,5-pyridindicarbonsäuredimethylester) den arteriellen Widerstand bei besonders ausgeprägter koronardilatierender Wirkung und hat in therapeutischer Dosierung keine Hemmwirkung auf das Herz.

Aus Pharmazie heute 104, 139 (1983) ist bekannt, daß zahlreiche 1,4-Dihydropyridine Calciumantagonisten sind.

Calciumantagonisten zeigen u.a. jedoch eine direkte negativ chronotrope und negativ inotrope Herzwirkung, was in der Therapie den Nachteil hat, daß möglicherweise Tachykardien auftreten können.

In Chem. Soc. Rev. 14, 375 (1985) werden Imidazolderivate sowie die entsprechenden Isosteren und Analogen beschrieben, welche am Histamin-Rezeptor angreifen. In B.J.R. Nicolaus in Decision Making in Drug Research (Ed. F. Gross), Raven Press, New York, 1983, Seite 173 - 188 schließlich ist beschrieben, daß durch die Kombination zweier pharmakophorer Gruppen Hybridmoleküle mit sehr unterschiedlicher Aktivität erhalten werden.

Es wäre vorteilhaft, bei der Therapie von Herzerkrankungen einen Wirkstoff zu verwenden, der zusätzlich zu einer negativ inotropen Herzwirkung eine direkte positiv inotrope Wirkung besitzt.

Der Erfindung liegt daher die Aufgabe zugrunde, neue Wirkstoffe zur Verfügung zu stellen, die in einem einzigen Molekül die Kombination einer calciumantagonistischen und einer $H_2$-agonistischen Wirkung bei einer vorteilhaften, klinisch relevanten Gesamtaktivität vereinigen.

Diese Aufgabe wird durch die Erfindung gelöst.

Gegenstand der Erfindung sind daher 1,4-Dihydropyridinderivate der allgemeinen Formel I

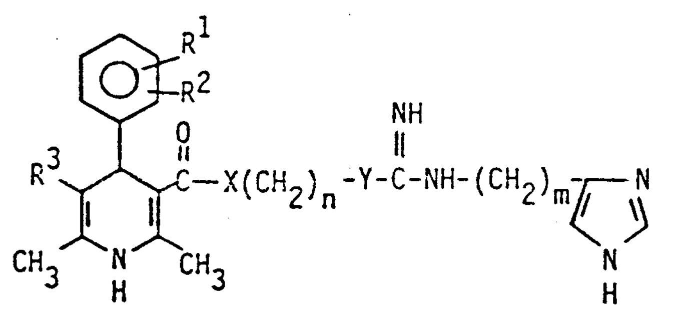

(I)

in der $R^1$ und $R^2$ unabhängig voneinander für ein Wasserstoffatom, eine gegebenenfalls durch Halogen substituierte Methylgruppe, ein Halogenatom, eine Nitrogruppe oder eine Trifluormethylgruppe stehen oder zusammen für einen 1,2,5-Oxadiazolrest stehen,

$R^3$ eine Nitrogruppe oder den Rest

$$-\overset{O}{\overset{\|}{C}}-OR^4$$

bedeutet, worin $R^4$ eine geradoder verzweigtkettige, gegebenenfalls durch eine $C_1$-$C_4$-Alkoxygruppe substituierte $C_1$-$C_4$-Alkylgruppe ist,

X für ein Sauerstoffatom oder die Gruppe NH steht,

Y für die Gruppe NH oder eine Bindung steht,

n eine ganze Zahl von 1 bis 16 bedeutet und

m den Wert 2 oder 3 hat,

sowie die physiologisch annehmbaren Salze davon.

In der allgemeinen Formel I bedeuten $R^1$ und $R^2$ unabhängig voneinander eine Methylgruppe. Weiterhin

können $R^1$ und $R^2$ ein Halogenatom, zum Beispiel ein Chlor-, Brom- oder Jodatom, eine Nitrogruppe oder eine Trifluormethylgruppe sein. Vorzugsweise stehen $R^1$ und $R^2$ für ein Halogenatom, ganz besonders bevorzugt für ein Chloratom. Weiterhin bevorzugt ist die Nitrogruppe. Die Substituenten $R^1$ und $R^2$ sind vorzugsweise in 2- und/oder 3-Stellung des Phenylrings gebunden. Die Methylgruppe kann unsubstituiert oder durch Halogen, vorzugsweise Chlor, substituiert sein, wobei die Einfachsubstitution bevorzugt wird. Die Gruppen $R^1$ und $R^2$ können weiterhin zusammen für einen 1,2,5-Oxadiazolrest stehen, der mit seinen C-Atomen 3 und 4 an den Phenylrest, vorzugsweise bei dessen Positionen 2 und 3, ankondensiert ist.

$R^3$ bedeutet eine Nitrogruppe oder den Rest

$$-\overset{\overset{\displaystyle O}{\|}}{C}-OR^4.$$

In diesem steht $R^4$ für eine gerad- oder verzweigtkettige $C_1$-$C_4$-Alkylgruppe. Beispiele für solche $C_1$-$C_4$-Alkylgruppen sind die Methyl-, Ethyl-, n-Propyl-, i-Propyl-, n-Butyl, i-Butyl-, sec.-Butyl- und tert.-Butylgruppe, wobei die Methyl- und die Ethylgruppe bevorzugt werden. Ganz besonders bevorzugt wird die Methylgruppe. Diese $C_1$-$C_4$-Alkylgruppe kann unsubstituiert oder einfach, substituiert sein. Als Substituenten kommen $C_1$-$C_4$-Alkoxygruppen in Betracht. Beispiele für solche $C_1$-$C_4$-Alkoxygruppen sind die Methoxy-, Ethoxy-, i-Propoxy-, n-Propoxy-, n-Butoxy- oder i-Butoxy- bzw. sec.-Butoxy- oder tert.-Butoxygruppe.

X steht für ein Sauerstoffatom oder die Gruppe NH, während Y die Gruppe NH oder eine Einfachbindung repräsentiert. n ist eine ganze Zahl von 1 bis 16, vorzugsweise 2 bis 6, während m den Wert 2 oder 3 hat, wobei der Wert 3 bevorzugt wird.

Eine bevorzugte Gruppe der erfindungsgemäßen Verbindungen ist dadurch gekennzeichnet, daß $R^1$ und $R^2$ unabhängig voneinander für ein Wasserstoffatom, eine Methylgruppe, die gegebenenfalls durch ein Halogenatom substituiert ist, ein Halogenatom, eine Nitrogruppe oder eine Trifluormethylgruppe stehen, wobei die Substituenten $R^1$ und $R^2$ in 2- und/oder 3-Stellung des Phenylrings gebunden sind, oder $R^1$ und $R^2$ zusammen für einen 1,2,5-Oxadiazolrest stehen, der in Positionen 3 und 4 an den Phenylrest in den Positionen 2 und 3 ankondensiert ist,

$R^3$ für eine Nitrogruppe oder den Rest

$$-\overset{\overset{\displaystyle O}{\|}}{C}-OR^4$$

steht, worin $R^4$ eine gerad- oder verzweigtkettige $C_1$-$C_4$-Alkylgruppe bedeutet, die gegebenenfalls durch eine $C_1$-$C_4$-Alkoxygruppe substituiert ist,
X für ein Sauerstoffatom oder die Gruppe NH steht,
Y für die Gruppe NH oder eine Bindung steht, n eine ganze Zahl von 1 bis 16 ist und m den Wert 3 hat.

Eine weitere bevorzugte Gruppe der erfindungsgemäßen Verbindungen ist dadurch gekennzeichnet, daß $R^1$ und $R^2$ unabhängig voneinander für ein Wasserstoffatom, ein Chloratom oder eine Nitrogruppe stehen, wobei die Substituenten $R^1$ und $R^2$ in 2- und/oder 3-Stellung des Phenylrings gebunden sind,
$R^3$ den Rest

$$-\overset{\overset{\displaystyle O}{\|}}{C}-OR^4$$

darstellt, worin $R^4$ für eine gerad- oder verzweigtkettige $C_1$-$C_4$-Alkylgruppe, die gegebenenfalls durch eine $C_1$-$C_4$-Alkoxygruppe substituiert ist, steht,
X für ein Sauerstoffatom oder die Gruppe NH steht,
Y für die Gruppe NH oder eine Bindung steht,
n eine ganze Zahl von 2 bis 6 bedeutet und
m den Wert 3 hat.

Schließlich ist eine weitere Gruppe der erfindungsgemäßen Verbindungen dadurch gekennzeichnet, daß $R^1$ für ein Wasserstoffatom steht, $R^2$ für eine in 2- oder 3-Position des Phenylrings gebundene Nitrogruppe

steht.

$R^3$ für den Rest

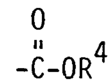

steht, worin $R^4$ eine gerad- oder verzweigtkettige $C_1$-$C_4$-Alkylgruppe ist, die gegebenenfalls durch eine $C_1$-$C_4$-Alkoxygruppe substituiert ist,
X für ein Sauerstoffatom steht,
Y für die Gruppe NH steht,
n eine ganze Zahl von 1 bis 16 bedeutet und
m den Wert 3 hat.

Die erfindungsgemäßen Verbindungen können mit geeigneten Säuren in ihre physiologisch annehmbaren Salze überführt werden. Diese Salze können zum Beispiel mit Mineralsäuren, wie Chlor-, Brom- oder Jodwasserstoffsäure, Phosphorsäure, Salpetersäure oder Schwefelsäure, oder mit organischen Säuren, wie Ameisensäure, Essigsäure, Propionsäure, Weinsäure, Zitronensäure, Fumarsäure, Methansulfonsäure, Embonsäure etc., in bekannter Weise gebildet werden. Die Erfindung umfaßt daher auch alle physiologisch annehmbaren Salze der oben beschriebenen Verbindungen der allgemeinen Formel I.

Die Erfindung umfaßt auch alle stereisomeren sowie alle tautomeren Formen und Hydrate der oben beschriebenen Verbindungen der allgemeinen Formel I.

Die erfindungsgemäßen Verbindungen können wie folgt hergestellt werden:

(a) Verbindungen der allgemeinen Formel I, bei denen $R^1$, $R^2$, $R^3$, X, m und n wie oben definiert sind und Y für NH steht, können hergestellt werden

a1) durch Umsetzung eines Isothiuroniumsalzes der allgemeinen Formel II

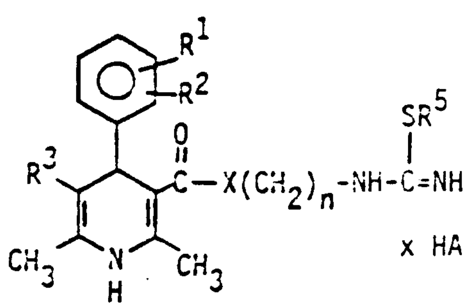

(II)

in der $R^1$, $R^2$, $R^3$, X und n die im Zusammenhang mit der allgemeinen Formel I angegebenen Bedeutungen besitzen, $R^5$ für einen gegebenenfalls substituierten Alkylrest und A für ein Halogenatom oder die Gruppe $-OSO_2OR^5$ stehen, mit einem m-(1H-Imidazol-4-yl)-alkylamin der Formel III

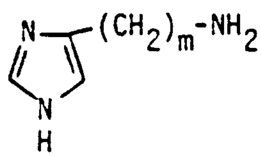

(III)

worin m den Wert 2 oder 3 hat. In der allgemeinen Formel II steht $R^5$ für einen gegebenenfalls substituierten Alkylrest, vorzugsweise einen gegebenenfalls substituierten $C_1$-$6_4$-Alkylrest, wie oben im Zusammenhang mit der allgemeinen Formel I definiert. Der durch $R^5$ angegebene Alkylrest kann unsubstituiert oder substituiert sein. Im Falle der Substitution kommt die Substitution mit einem Phenyl- oder Benzylrest, vorzugsweise Benzylrest, in Frage. A steht für ein Halogenatom, wie oben im Zusammenhang mit der allgemeinen Formel I definiert, vorzugsweise ein Jodatom, oder die Gruppe $-OSO_2OR^5$, wobei $R^5$ die vorstehend angegebene Bedeutung hat. Die Umsetzung der Reaktanten

erfolgt vorzugsweise in äquimolaren Mengen und in einem Lösungsmittel bei Temperaturen zwischen Raumtemperatur und dem Siedepunkt des Lösungsmittels. Als Lösungsmittel werden Pyridin, Acetonitril oder Alkohole, insbesondere n-Butanol, bevorzugt.

a2) durch Umsetzung eines Amins der allgemeinen Formel IV

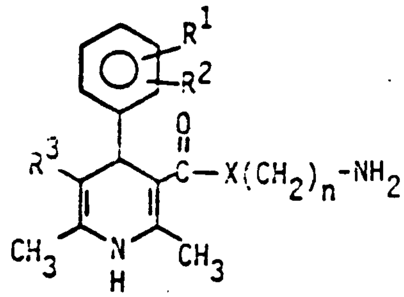

(IV)

in der $R^1$, $R^2$, $R^3$, X und n die im Zusammenhang mit der allgemeinen Formel I angegebenen Bedeutungen haben, mit einer Verbindung der allgemeinen Formel V

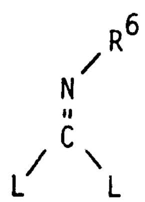

(V)

in der $R^6$ eine Cyangruppe oder einen Benzoylrest bedeutet und L für eine Abgangsgruppe, wie $-SR^7$ oder $-OR^7$, steht, in welcher $R^7$ einen Alkylrest, vorzugsweise einen $C_1$-$C_4$-Alkylrest, wie im Zusammenhang mit der allgemeinen Formel I definiert, oder einen Arylrest, vorzugsweise einen Phenylrest, bedeutet, zu einer Zwischenverbindung der allgemeinen Formel VI

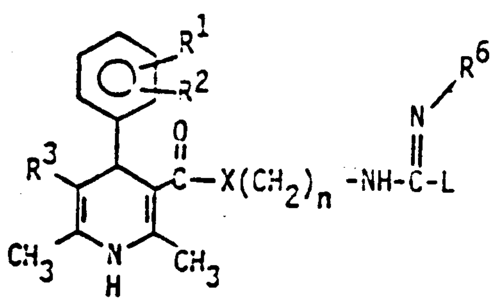

(VI)

Umsetzung der Zwischenverbindung VI mit einem m-(1H-Imidazol-4-yl)-alkylamin der Formel III

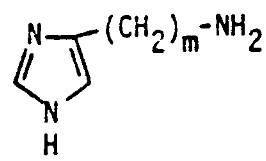

(III)

worin m den Wert 2 oder 3 hat,
zu einer Verbindung der allgemeinen Formel VII

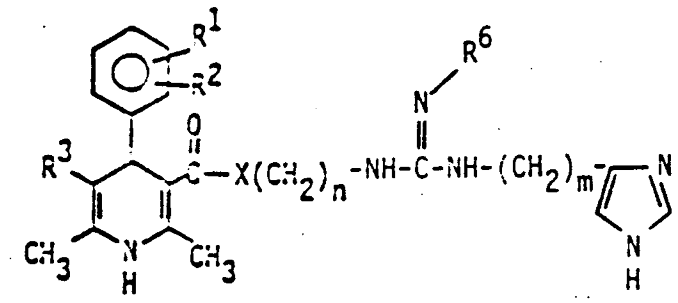

(VII)

und anschließende säurekatalysierte Hydrolyse zu einer Verbindung der allgemeinen Formel I.

In der ersten Stufe dieses Verfahrens werden die Reaktanten der Formeln IV und V vorzugsweise in äquimolaren Mengen eingesetzt. Als Lösungsmittel kommen die üblichen inerten organischen Lösungsmittel in Frage. Bevorzugt werden chlorierte Kohlenwasserstoffe, wie Dichlormethan oder Chloroform, Ether, wie Tetrahydrofuran oder Dioxan, sowie Acetonitril, Aceton, Pyridin oder Dimethylformamid. Die Reaktionstemperaturen liegen im allgemeinen zwischen 0 ° C und dem Siedepunkt des verwendeten Lösungsmittels.

Auch in der zweiten Stufe dieses Verfahrens werden die Reaktanten VI und III vorzugsweise in äquimolaren Mengen eingesetzt. Es werden die gleichen Lösungsmittel und Temperaturen angewandt wie in der ersten Stufe dieses Verfahrens.

Die dritte Stufe dieses Verfahrens, d.h. die Hydrolyse der Zwischenverbindung VII zu dem Endprodukt der allgemeinen Formel I, wird in verdünnten anorganischen Säuren, in verdünnten organischen Säuren oder in Gemischen von beiden bei Rückflußtemperaturen durchgeführt. Als Säuren werden bevorzugt: Chlorwasserstoffsäure, Bromwasserstoffsäure, Jodwasserstoffsäure, Schwefelsäure, Phosphorsäure, Essigsäure sowie Mischungen aus den genannten anorganischen Säuren und Essigsäure.

oder

a3) durch Umsetzung eines Amins der allgemeinen Formel IV

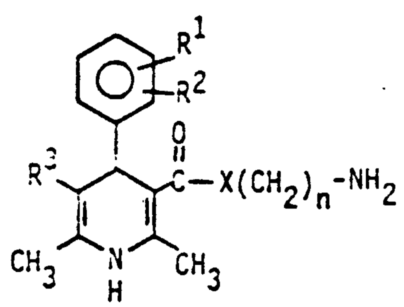

(IV)

in der $R^1$, $R^2$, $R^3$, X und n die im Zusammenhang mit der allgemeinen Formel I angegebenen Bedeutungen haben, mit einer Verbindung der allgemeinen Formel VIII

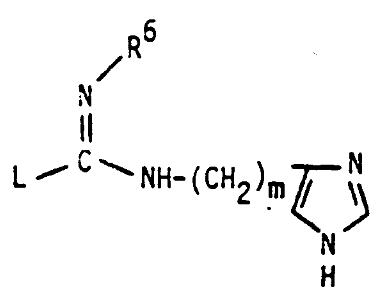

(VIII)

in der $R^6$ und L die im Zusammenhang mit der allgemeinen Formel V angegebenen Bedeutungen besitzen und m den Wert 2 oder 3 hat, zu einer Verbindung der allgemeinen Formel VII

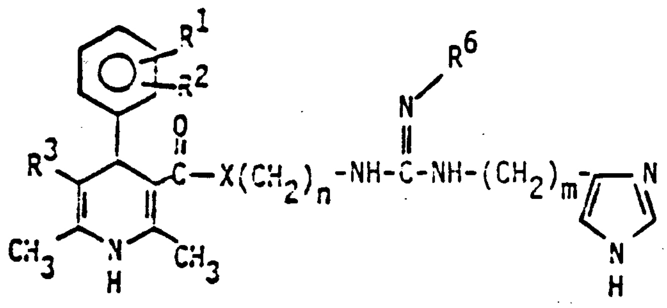

(VII)

und anschließende Hydrolyse zu einer Verbindung der allgemeinen Formel I. Die Hydrolyse erfolgt wie im Zusammenhang mit der Verfahrensvariante a2) beschrieben.

oder

a4) durch Umsetzung eines Amins der allgemeinen Formel IV

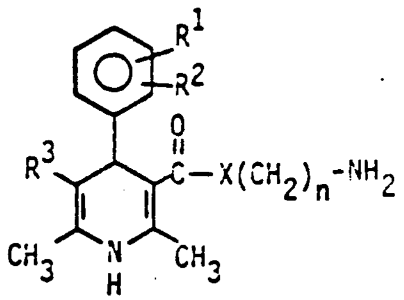

(IV)

in der $R^1$, $R^2$, $R^3$, X und n die im Zusammenhang mit der allgemeinen Formel I angegebenen Bedeutungen haben, mit einer Verbindung der allgemeinen Formel IX

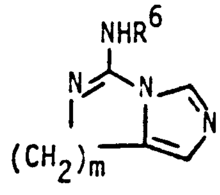

(IX)

in der $R^6$ die im Zusammenhang mit der allgemeinen Formel V angegebene Bedeutung hat und m den Wert 2 oder 3 hat, zu einer Zwischenverbindung der allgemeinen Formel VII

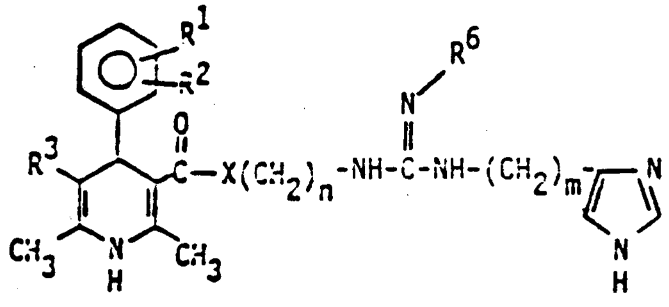

(VII)

und anschließende Hydrolyse der so erhaltenen Zwischenverbindung VII zu einer Verbindung der allgemeinen Formel I.

Das Verfahren dieser Variante erfolgt bei den gleichen Bedingungen, wie sie oben im Zusammenhang

mit den Verfahrensvarianten a2) und a3) beschrieben wurden.
(b) Verbindungen der allgemeinen Formel I, bei denen $R^1$, $R^2$, $R^3$, X, m und n die im Zusammenhang mit der allgemeinen Formel I angegebenen Bedeutungen haben und Y für eine Einfachbindung steht, werden hergestellt, indem man einen Imidoester der allgemeinen Formel X

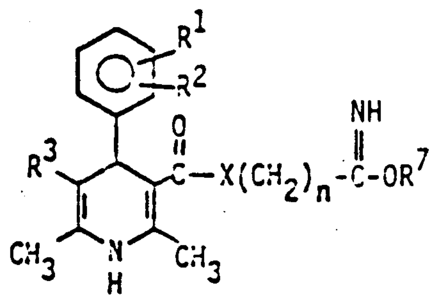

(X)

in der $R^1$, $R^2$, $R^3$, X und n die im Zusammenhang mit der allgemeinen Formel I angegebenen Bedeutungen haben und $R^7$ für einen $C_1$-$C_4$-Alkylrest, wie im Zusammenhang mit der allgemeinen Formel I definiert, steht, mit einem m-(1H-Imidazol-4-yl)-alkylamin der Formel III

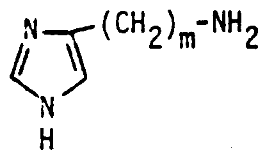

(III)

worin m den Wert 2 oder 3 hat, umsetzt.
Die Umsetzung wird in einem geeigneten Lösungsmittel und bei Temperaturen von Raumtemperatur bis zum Siedepunkt des verwendeten Lösungsmittels, vorzugsweise bei Rückflußtemperatur, durchgeführt.
Geeignete Lösungsmittel sind die üblichen inerten organischen Lösungsmittel, wobei Alkohole, insbesondere die dem Rest $R^7$ entsprechenden Alkohole, bevorzugt werden.
Das Molverhältnis der Reaktanten III und X kann zwischen 3:1 und 1:1 variieren, bevorzugt wird ein Verhältnis von 1:1 bis 1,2:1.

Die nach den Verfahrensvarianten a1) bis b) erhaltenen Verbindungen werden in üblicher Weise isoliert und gereinigt, beispielsweise durch chromatographische Methoden, Umkristallisation, Extraktion etc.

Die als Ausgangsstoffe verwendeten Amine der allgemeinen Formel IV sind bekannt oder können nach an sich bekannten Methoden hergestellt werden (vgl. DE-OS 21 17 571, DE-OS 34 20 784 und EP-OS 0 151 006).

Die Isothiuroniumsalze der allgemeinen Formel II erhält man nach literaturbekannten Verfahren, beispielsweise durch Acylierung der Amine der allgemeinen Formel IV mit Benzoylisothiocyanat, basische Hydrolyse des Benzoylrests und Alkylierung des erhaltenen Thioharnstoffs mit den bekannten Alkylierungsmitteln, wie Alkylhalogeniden, -sulfaten etc.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutischen Trägern oder Verdünnungsmitteln hergestellt werden.

Die erfindungsgemäßen Verbindungen können daher für die orale, bukkale, topische, parenterale oder rektale Verabreichung formuliert werden. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen wie Suspensionen, Lösungen oder Emulsionen in öligen oder

wäßrigen Trägern einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten. Eine Dosiseinheit des erfindungsgemäßen Arzneimittels enthält 0,1 mg bis 30 mg Wirkstoff, vorzugsweise 1 mg bis 20 mg.

Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die erfindungsgemäßen Verbindungen zeichnen sich durch eine neuartige, bisher nicht bekannte und beschriebene pharmakologische Gesamtaktivität aus. Die erfindungsgemäße neue Strukturklasse zeigt sowohl eine calciumantagonistische als auch eine $H_2$-agonistische Wirkkomponente.

Die calciumantagonistische Aktivität der erfindungsgemäßen Verbindungen wurde am isolierten, mit Bariumchlorid stimulierten Meerschweinchen-Ileum bestimmt (leicht modifizierte Methode nach A. Fleckenstein u.a., Arzneim.-Forsch. 29, 230-246 (1971)).

Zur Bestimmung der $H_2$-agonistischen Aktivität ($pD_2$-Werte) wird die Methode nach van Rossum, J.M. (1963) Cumulative dose-response Curves, II. Technique for the making of dose-response curves in isolated organs and the evaluation of drug parameters, Arch. Intern. Pharmacodyn. Therap. 143, 299-307, herangezogen.

## Pharmakologische Daten

| | $pA_2$-Wert ($BaCl_2$) | $pD_2$-Wert |
|---|---|---|
| Verbindung des Beispiels 1 | 7,42 | 7,32 |
| Verbindung des Beispiels 7 | 6,96 | 7,41 |
| Verbindung des Beispiels 8 | 7,52 | 6,48 |
| Nifedipin (Vergleich) | 8,02 | - |
| Impromidin (Vergleich) | - | 7,67 |

## Herstellungsbeispiele

### Beispiel 1

N[1]-[2-[1,4-Dihydro-2,6-dimethyl-3-methoxycarbonyl-4-(2-nitrophenyl)-pyridin-5-carboxy]ethyl]-N[2]-[3-(1H-imidazol-4-yl) propyl]-guanidin-hydrojodid

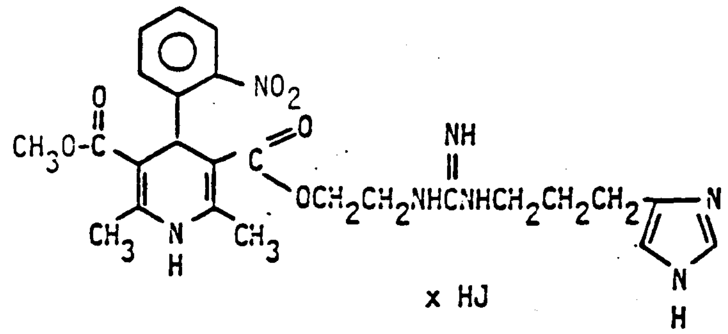

a) 1,4-Dihydro-2,6-dimethyl-3-methoxycarbonyl-4-(2-nitrophenyl)-5-(2-phthalimido-ethoxy)carbonyl-pyridin

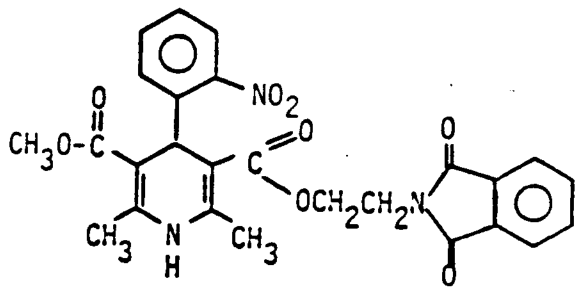

14,95 g (60 mmol) 2-(2-Nitrobenzyliden)-acetessigsäuremethylester und 16,46 g (60 mmol) 3-Aminocrotonsäure-(2-phthalimidoethyl)ester werden in 150 ml Ethanol 16 Stunden gekocht. Nach Abkühlen auf Raumtemperatur wird der ausgefallene Niederschlag abgesaugt, mit etwas kaltem Ethanol gewaschen und aus Ethanol umkristallisiert.

Man erhält 21,53 g (71 %) gelbe Kristalle vom Schmp. 229-230 ° C.

$C_{26}H_{23}N_3O_8$ (505,48)

Rf ($CH_2Cl_2/CH_3OH$ 95:5) : 0,35

b) 1,4-Dihydro-2,6-dimethyl-3-methoxycarbonyl-4-(2-nitrophenyl)-5-(2-amino-ethoxy)carbonyl-pyridin

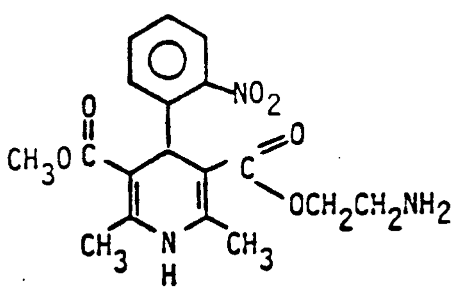

Zu einer siedenden Lösung von 5,05 g (10 mmol) 1,4-Dihydro-2,6-dimethyl-3-methoxycarbonyl-4-(2-nitrophenyl)-5-(2-phthalimidoethoxy)carbonyl-pyridin in 50 ml Ethanol werden 1,5 ml (30 mmol) Hydrazinhydrat in 7,5 ml Ethanol getropft. Es wird 2 Std. weitergekocht, abgekühlt und i. Vak. eingeengt. Der Rückstand wird mit 25 ml 2N Salzsäure 30 Minuten bei Raumtemperatur gerührt, der ausgefallene Feststoff wird abgesaugt und das Filtrat mit konz. Ammoniak auf pH 10 eingestellt. Nach dreimaliger Extraktion mit je 30 ml Methylenchlorid werden die organischen Phasen getrocknet und eingeengt. Es verbleiben 2,91 g (77 %) eines gelben amorphen Feststoffs, der ohne weitere Reinigung weiter umgesetzt wird.

$C_{18}H_{21}N_3O_6$ (375,38)

Rf ($CH_2Cl_2/CH_3OH$ 90 : 10) : 0,3

c) N[1]-Benzoyl-N[2]-[2-[1,4-dihydro-2,6-dimethyl-3-methoxycarbonyl-4-(2-nitrophenyl)-pyridin-5-carboxy] ethyl]-thioharnstoff

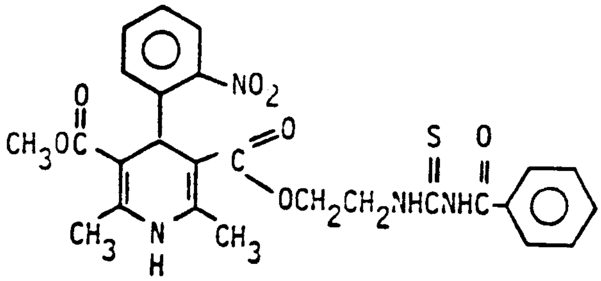

1,50 g (4 mmol) 1,4-Dihydro-2,6-dimethyl-3-methoxycarbonyl-4-(2-nitrophenyl)-5-(2-aminoethoxy)-carbonyl-pyridin und 0,65 g (4 mmol) Benzoylisothiocyanat werden in 15 ml Dichlormethan 2 Std. gekocht. Nach Abdampfen des Lösungsmittels i. Vak. wird der Rückstand mit tert.-Butyl-methylether kristallisiert. Man erhält 2,0 g (93 %) eines gelben Feststoffs vom Schmp. 96-98 ° C.

$C_{26}H_{26}N_4O_7S$ (538,58)

Rf ($CH_2Cl_2/CH_3OH$ 95 : 5) : 0,57

d) S-Methyl-N-[2-[1.4-dihydro-2,6-dimethyl-3-methoxy-carbonyl-4-(2-nitrophenyl)-pyridin-5-carboxy]ethyl]-isothiuroniumjodid

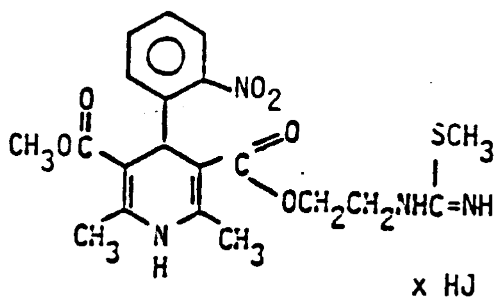

2,00 g (3,7 mmol) N[1]-Benzoyl-N[2]-[2-[1,4-dihydro-2,6-dimethyl-3-methoxycarbonyl-4-(2-nitrophenyl)-pyridin-5-carboxy]ethyl]-thioharnstoff werden mit 0,51 g (3,7 mmol) Kaliumcarbonat in 50 ml Methanol und 15 ml Wasser 20 Minuten gekocht. Die Mischung wird mit 100 ml Wasser verdünnt und mit 4 x 50 ml Dichlormethan extrahiert. Nach Trocknen über Natriumsulfat werden die organischen Phasen filtriert und i. Vak. eingedampft. Der Rückstand wird in 40 ml Ethanol aufgenommen und mit 0,23 ml (3,7 mmol) Methyljodid versetzt. Nach 20 Stunden bei Raumtemperatur wird die Lösung i. Vak. eingeengt und der Rückstand mit tert.-Butylmethylether verrieben, wobei 1,35 g (63 %) gelbe Kristalle vom Schmp. 96-98 ° C erhalten werden.

$C_{20}H_{25}JN_4O_6S$ (576,41)

Rf ($CH_2Cl_2/CH_3OH$ 90 : 10) : 0,4

e) N[1]-[2-[1,4-Dihydro-2,6-dimethyl-3-methoxycarbonyl-4-(2-nitrophenyl)-pyridin-5-carboxy]ethyl]-N[2]-[3-(1H-imidazol-4-yl)propyl]-guanidin-hydrojodid

1,00 g (1.73 mmol) S-Methyl-N-[2-[1,4-dihydro-2,6-dimethyl-3-methoxycarbonyl-4-(2-nitrophenyl)-pyridin -5-carboxy]ethyl] isothiuronium-jodid und 0,22 g (1.73 mmol) 3-(1H-Imidazol-4-yl)propylamin werden in 15 ml Acetonitril 3 Stunden gekocht. Nach Abdampfen des Lösungsmittels i. Vak. wird das Rohprodukt

chromatographisch an Kieselgel mit Ethylacetat/Ethanol (80:20) als Laufmittel gereinigt. Die Hauptfraktion ergibt nach Eindampfen i.Vak. 0,82 g (72 %) eines gelben, amorphen Feststoffs.

$C_{25}H_{32}JN_7O_6$ (653,47)

Rf ($CH_3COOC_2H_5/CH_3OH/NH_4Cl/NH_3$-Puffer 50:47,5:2,5) : 0,66

| | | | | |
|---|---|---|---|---|
| $^1$H-NMR-Daten ($CD_3OD$, TMS als interner Standard) | δ = 1,8 - 2,2 | (m) | 2 H | |
| | 2,3 | (s) | 3 H | |
| | 2,4 | (s) | 3 H | |
| | 2,6 - 2,9 | (t) | 2 H | |
| | 3,1 - 4,6 | (m) | 6 H | |
| | 3,6 | (s) | 3 H | |
| | 5,1 | (breit) | 6 H | austauschbar mit $D_2O$ |
| | 5,85 | (s) | 1 H | |
| | 7,1 | (s) | 1 H | |
| | 7,3 - 8,1 | (m) | 5 H | ppm |

Beispiel 2

N[1]-[3-[1,4-Dihydro-2,6-dimethyl-3-methoxycarbonyl-4-(2-nitrophenyl)-pyridin-5-carboxy]propyl]-N[2]-[3-(1H-imidazol-4-yl)propyl]-guanidin-hydrojodid

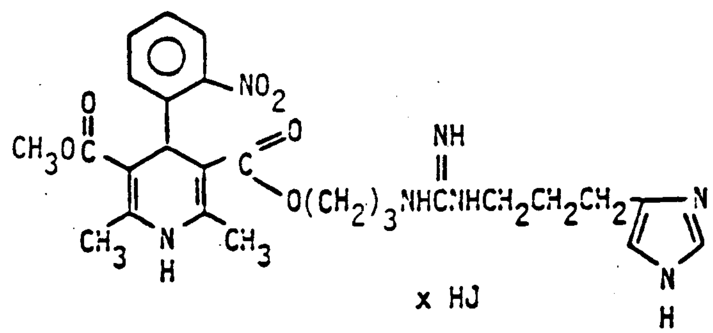

a) 1,4-Dihydro-2,6-dimethyl-3-methoxycarbonyl-4-(2-nitrophenyl)-5-(3-phthalimido-propoxy)carbonyl-pyridin

Analog zu Beispiel 1a) werden aus 12,5 g (50 mmol) 2-(2-Nitrobenzyliden)-acetessigsäuremethylester und 14,4 g (50 mmol) 3-Amino-crotonsäure-(3-phthalimidopropyl)ester 12,2 g (47 %) gelbe Kristalle vom Schmp. 174-175 °C erhalten.

$C_{27}H_{25}N_3O_8$ (519,51)

Rf ($CH_2Cl_2/CH_3OH$ 95 : 5) : 0,6

b) 1,4-Dihydro-2,6-dimethyl-3-methoxycarbonyl-4-(2-nitrophenyl)-5-(3-aminopropoxy)carbonyl-pyridin

Entsprechend Beispiel 1b) aus 12,0 g (23 mmol) 1,4-Dihydro-2,6-dimethyl-3-methoxycarbonyl-4-(2-nitrophenyl)-5-(3-phthalimidopropoxy)carbonyl-pyridin und 4,2 ml (69 mmol) Hydrazinhydrat in Ethanol.

Das Rohprodukt wird an Kieselgel mit Dichlormethan/Methanol (80 : 20) als Laufmittel gereinigt. Die gelbe Hauptfraktion gibt nach Eindampfen i. Vak. 6,93 g (77 %) eines braungelben, zähen Öls.

$C_{19}H_{23}N_3O_6$ (389,41)

Rf ($CH_2Cl_2/CH_3OH$ 80:20) : 0,2

c) N[1]-Benzoyl-N[2]-[3-[1,4-dihydro-2,6-dimethyl-3-methoxycarbonyl - 4- (2-nitrophenyl)-pyridin-5-carboxy]

propyl]-thioharnstoff

Aus 6,93 g (17,8 mmol) 1,4-Dihydro-2,6-dimethyl-3-methoxycarbonyl-4-(2-nitrophenyl)-5-(3-aminopropoxy)-carbonyl-pyridin und 2,90 g (17,8 mmol) Benzoylisothiocyanat analog zu Beispiel 1c). 7,6 g (77 %) dunkelgelbes Öl.

$C_{27}H_{28}N_4O_7S$ (552,60)

Rf ($CH_2Cl_2/CH_3OH$) 95:5) : 0,5

d) S-Methyl-N-[3-[1,4-dihydro-2,6-dimethyl-3-methoxycarbonyl-4-(2-nitrophenyl)-pyridin-5-carboxy]propyl] isothiuronium-jodid

Hergestellt analog zu Beispiel 1d) aus 7,6 g (14 mmol) N[1]-Benzoyl-N[2]-[3-[1,4-dihydro-2,6-dimethyl-3-methoxycarbonyl-4-(2-nitrophenyl)-pyridin-5-carboxy]propyl]-thioharnstoff. 6,1 g (75 %) gelber, amorpher Schaum.

$C_{21}H_{27}J\ N_4O_6S$ (590,24)

Rf ($CH_2Cl/CH_3OH$ 90:10) : 0,4

e) N[1]-[3-[1,4-Dihydro-2,6-dimethyl-3-methoxycarbonyl-4-(2-nitrophenyl)-pyridin-5-carboxy]propyl]-N[2]-[3-(1H-imidazol-4-yl)propyl]-guanidin-hydrojodid

Erhalten analog zu Beispiel 1e) aus 1,00 g (1,69 mmol) S-Methyl-N-[3-[1,4-dihydro-2,6-dimethyl-3-methoxycarbonyl-4-(2-nitrophenyl)-pyridin-5-carboxy]propyl]-isothiuronium-jodid und 0,21 g (1,69 mmol) 3-(1H-Imidazol-4-yl)propylamin in Acetonitril. Nach chromatographischer Reinigung des Rohproduktes an Kieselgel mit Ethylacetat/Methanol (80:20) als als Laufmittel erhält man 0,80 g (71 %) eines gelben, amorphen Feststoffs.

$C_{26}H_{34}JN_7O_6$ (667,50)

Rf ($CH_3COOC_2H_5/CH_3OH/NH_4Cl/NH_3$-Puffer 50:47,5:2,5) : 0,5

| | | | |
|---|---|---|---|
| $^1$H-NMR-Daten ($CD_3OD$, TMS als interner Standard) $\delta$= | 1,7 - 2,1 | (m) | 4H |
| | 2,3 | (s) | 3H |
| | 2,35 | (s) | 3H |
| | 2,7 | (t) | 2H |
| | 3,0 - 3,4 | (m) | 4H |
| | 3,6 | (s) | 3H |
| | 3,9 - 4,4 | (m) | 2H |
| | 4,9 | (breit) | 6H, austauschbar mit $D_2O$ |
| | 5,8 | (s) | 1H |
| | 6,95 | (s) | 1H |
| | 7,3 - 7,9 | (m) | 5H ppm. |

Beispiel 3

N[1]-[2-[1,4-Dihydro-2,6-dimethyl-3-methoxycarbonyl-4-(3-nitrophenyl)-pyridin-5-carboxy]ethyl]-N[2]-[3-(1H-imidazol-4-yl)propyl]-guanidin-hydrojodid

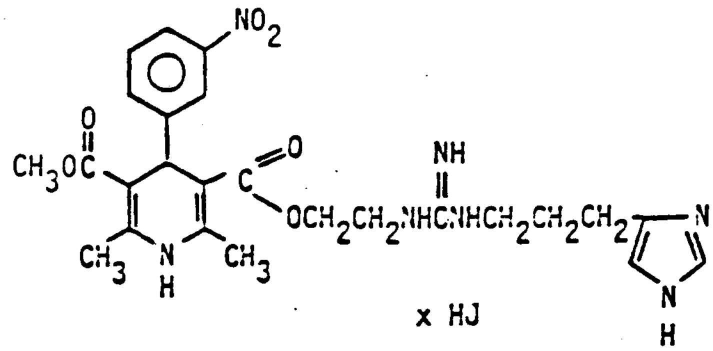

a) 1,4-Dihydro-2,6-dimethyl-3-methoxycarbonyl-4-(3-nitrophenyl)-5-(2-phthalimidoethoxy)carbonyl-pyridin

Hergestellt analog zu Beispiel 1a) aus 14,95 g (60 mmol) 2-(3-Nitro-benzyliden)-acetessigsäuremethylester und 16,46 g (60 mmol) 3-Amino-crotonsäure-(2-phthalimidoethyl)ester in Ethanol. Es werden 25,25 g (83%) gelbe Kristalle vom Schmp. 181-182 °C erhalten.

$C_{26}H_{23}N_3O_8$ (505,48)

Rf ($CH_2Cl_2/CH_3OH$ 95:5) : 0,5

b) 1,4-Dihydro-2,6-dimethyl-3-methoxycarbonyl-4-(3-nitrophenyl)-5-(2-aminoethoxy)carbonyl-pyridin

Aus 20,22 g (40 mmol) 1,4-Dihydro-2,6-dimethyl-3-methoxycarbonyl-4-(3-nitrophenyl)-5-(2-phthalimidoethoxy)carbonyl-pyridin und 5,8 ml (120 mmol) Hydrazinhydrat erhält man analog zu Beispiel 1b) 13,48 g (93 %) eines gelben Feststoffs.

$C_{18}H_{21}N_3O_6$ (375,38)

Rf ($CH_2Cl_2/CH_3OH$ 90:10) : 0,33

c) $N^1$-Benzoyl-$N^2$-[2-[1,4-dihydro-2,6-dimethyl-3-methoxycarbonyl-4-(3-nitrophenyl)-pyridin-5-carboxy]ethyl] thioharnstoff

Hergestellt analog zu Beispiel 1c) aus 11,26 g (30 mmol) 1,4-Dihydro-2,6-dimethyl-3-methoxycarbonyl-4-(3-nitrophenyl)-5-(2-aminoethoxy)carbonyl-pyridin und 4,89 g (30 mmol) Benzoylisothiocyanat. Nach chromatographischer Reinigung an Kieselgel mit Dichlormethan/Methanol (97:3) als Laufmittel 7,41 g (46 %) eines gelben, amorphen Feststoffs.

$C_{26}H_{26}N_4O_7S$ (538,38)

Rf ($CH_2Cl_2/CH_3OH$ 90:10) : 0,6

d) S-Methyl-N-[2-[1,4-dihydro-2,6-dimethyl-3-methoxycarbonyl-4-(3-nitrophenyl)-pyridin-5-carboxy]ethyl]-isothiuronium-jodid

Analog zu Beispiel 1d) aus 3,80 g (7 mmol) $N^1$-Benzoyl-$N^2$-[2-[1,4-dihydro-2,6-dimethyl-3-methoxycarbonyl-4-(3-nitrophenyl)-pyridin-5-carboxy]ethyl]-thioharnstoff 1,09 g /27 %) gelblicher, amorpher Feststoff.

$C_{20}H_{25}JN_4O_6S$ (576,41)

Rf ($CH_2Cl_2/CH_3OH$ 90:10) : 0,32

e) $N^1$-[2-[1,4-Dihydro-2,6-dimethyl-3-methoxycarbonyl-4-(3-nitrophenyl)-pyridin-5-carboxy]ethyl]-$N^2$-[3-(1H-imidazol-4-yl)propyl]-guanidin-hydrojodid

Hergestellt analog zu Beispiel 1e) aus 1,00g (1,7 mmol) S-Methyl-N-[2-[1,4-dihydro-2,6-dimethyl-3-methoxycarbonyl-4-(3-nitrophenyl)-pyridin-5-carboxy]ethyl]-isothiuronium-jodid und 0,22 g (1,7 mmol) 3-(1H-Imidazol-4-yl)propylamin. Nach chromatographischer Reinigung an Aluminiumoxid mit Essigester/Ethanol (60:40) 0,73 g (65 %) gelber, amorpher Feststoff.

$C_{25}H_{32}JN_7O_6$ (653,47)

Rf ($CH_3COOC_2H_5/C_2H_5OH$ 60:40) : 0,36

$^1$H-NMR-Daten ($CD_3OD$, TMS als interner Standard): δ =

| | | |
|---|---|---|
| 1,7-2,1 | (m) | 2H |
| 2,3 | (s) | 3H |
| 2,35 | (s) | 3H |
| 2,7 | (t) | 2H |
| 3,1-3,8 | (m) | 4H |
| 3,65 | (s) | 3H |
| 4,1-4,4 | (m) | 2H |
| 4,9 | (breit) | 6H, austauschbar mit $D_2O$ |
| 5,1 | (s) | 1H |
| 7,0 | (s) | 1H |
| 7,4-8,2 | (m) | 5H ppm |

Beispiel 4

N[1]-[2-[1,4-Dihydro-2,6-dimethyl-3-ethoxycarbonyl-4-(3-nitrophenyl)-pyridin-5-carboxy]ethyl]-N[2]-[3-(1H-imidazol-4-yl)propyl]-guanidin-hydrojodid

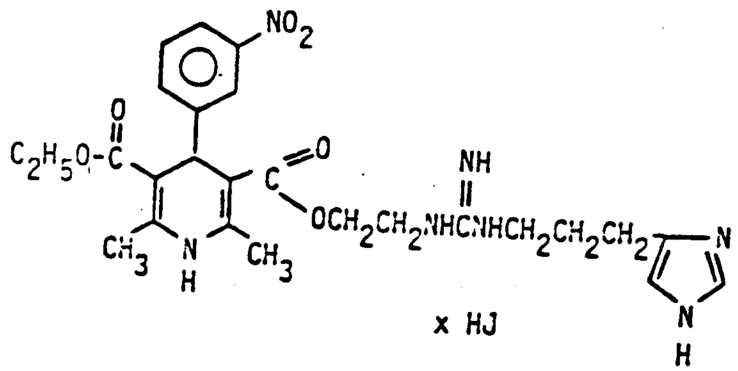

a) 1,4-Dihydro-2,6-dimethyl-3-ethoxycarbonyl-4-(3-nitrophenyl)-5-(2-phthalimidoethoxy)carbonyl-pyridin

Hergestellt analog zu Beispiel 1a) aus 15,80 g (60 mmol) 2-(3-Nitrobenzyliden)-acetessigsäureethylester und 16,46 g (60 mmol) 3-Amino-crotonsäure-(2-phthalimidoethyl)ester in Ethanol. Man erhält 27,75 g (89 %) gelbe Kristalle vom Schmp. 194-195 ° C.

$C_{27}H_{25}N_3O_8$ (519,51)

Rf ($CH_2Cl_2$/$CH_3OH$ 95:5) : 0,48

b) 1,4-Dihydro-2,6-dimethyl-3-ethoxycarbonyl-4-(3-nitrophenyl)-5-(2-aminoethoxy)carbonyl-pyridin

Hergestellt analog zu Beispiel 1b) aus 20,78 g (40 mmol) 1,4-Dihydro-2,6-dimethyl-3-ethoxycarbonyl-4-(3-nitrophenyl)-5-(2-phthalimidoethoxy)carbonyl-pyridin und 5,83 ml (120 mmol) Hydrazinhydrat. 13,39 g (86 %) gelbe Kristalle vom Schmp. 137 - 139 ° C.

$C_{19}H_{23}N_3O_6$ (389,41)

Rf ($CH_2Cl_2$/$CH_3OH$ 90:10) : 0,36

c) N[1]-Benzoyl-N[2]-[2-[1,4-dihydro-2,6-dimethyl-3-ethoxycarbonyl-4-(3-nitrophenyl)-pyridin-5-carboxy]ethyl]-thioharnstoff

Hergestellt analog zu Beispiel 1c) aus 9,74 g (25 mmol) 1,4-Dihydro-2,6-dimethyl-3-ethoxycarbonyl-4-(3-nitrophenyl)-5-(2-aminoethoxy)carboxyl-pyridin und 4,08 g (25 mmol) Benzoylisothiocyanat. Aus tert.-Butylmethylether 12,52 g (91 %) gelbe Kristalle mit Schmp. 80 - 82° C.

$C_{27}$ $H_{28}$ $N_4$ $O_7$ S (552,60)

Rf ($CH_2Cl_2/CH_3OH$ 95:5) : 0,62

d) S-Methyl-N-[2-[1,4-dihydro-2,6-dimethyl-3-ethoxycarbonyl-4-(3-nitrophenyl)-pyridin-5-carboxy]ethyl]-isothiuronium-jodid

Analog zu Beispiel 1d) aus 11,05 g (20 mmol) $N^1$-Benzoyl-$N^2$-[2-[1,4-dihydro-2,6-dimethyl-3-ethoxycarbonyl-4-(3-nitrophenyl)-pyridin-5-carboxy] ethyl]-thioharnstoff und 2,84 g (20 mmol) Methyljodid. 8,94 g (76 %) gelber, amorpher Schaum.

$C_{21}$ $H_{27}$ J $N_4$ $O_6$ S (590,24)

Rf ($CH_2Cl_2/CH_3OH$ 90:10) : 0,45

e) $N^1$-[2-[1,4-Dihydro-2,6-dimethyl-3-ethoxycarbonyl-4-(3-nitrophenyl)-pyridin-5-carboxy]ethyl]-$N^2$-[3-(1H-imidazol-4-yl)propyl]-guanidin-hydrojodid

1,00 g (1,69 mmol) S-Methyl-N-[2-[1,4-dihydro-2,6-dimethyl-3-ethoxycarbonyl-4-(3-nitrophenyl)-pyridin-5-carboxy]ethyl]isothiuronium-jodid und 0,21 g (1,69 mmol) 3-(1H-Imidazol-4-yl)propylamin werden in 15 ml Acetonitril 3 Std. unter Rückfluß gekocht. Nach Abdampfen des Lösungsmittels i.Vak. wird der Rückstand mit Dichlormethan kristallisiert. Man erhält 0,67 g (59 %) eines gelben Feststoffs vom Schmp. 104-105° C.

$C_{26}$ $H_{34}$ J $N_7$ $O_6$ (667,50)

Rf ($CH_2Cl_2/CH_3OH$ 80:20) : 0,46

| | | | | |
|---|---|---|---|---|
| $^1$H-NMR-Daten ($CD_3OD$, TMS als interner Standard) | $\delta$ = | 1,2 | (t) | 3H |
| | | 1,7-2,1 | (m) | 2H |
| | | 2,3 | (s) | 3H |
| | | 2,35 | (s) | 3H |
| | | 2,7 | (t) | 2H |
| | | 3,1-3,6 | (m) | 4H |
| | | 4,1 | (q) | 2H |
| | | 4,1-4,4 | (m) | 2H |
| | | 4,8 | (breit) | 6H, austauschbar mit $D_2O$ |
| | | 5,1 | (s) | 1H |
| | | 7,0 | (s) | 1H |
| | | 7,4-8,2 | (m) | 5H ppm |

Beispiel 5

$N^1$-[2-[1,4-Dihydro-2,6-dimethyl-3-(2-methoxyethoxy)carbonyl-4-(3-nitrophenyl)-pyridin-5-carboxy]ethyl]-$N^2$-[3-(1H-imidazol-4-yl)propyl]-guanidin-hydrojodid

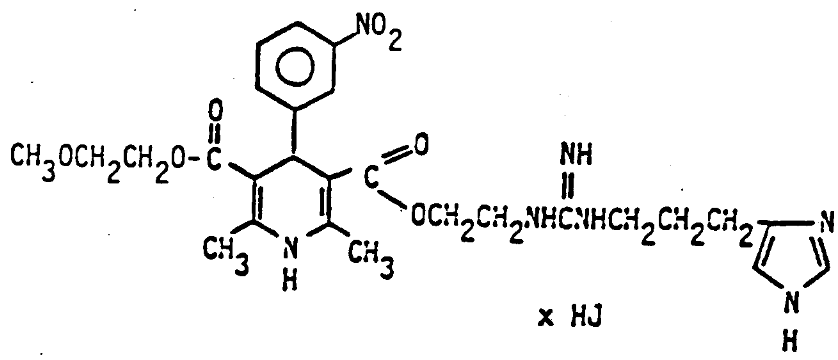

a) 1,4-Dihydro-2,6-dimethyl-3-(2-methoxyethoxy)carbonyl-4-(3-nitrophenyl)-5-(2-phthalimidoethoxy)carbonyl-pyridin

Hergestellt analog zu Beispiel 1a) aus 7,0 g (24 mmol) 2-(3-Nitrobenzyliden)-acetessigsäure-(2-methoxyethyl) ester und 6,55 g (24 mmol) 3-Amino-crotonsäure-(2-phthalimidoethyl)ester in Ethanol. 11,08 g (84 %) hellgelbe Kristalle vom Schmp. 182-184 ° C.

$C_{28}H_{27}N_3O_9$ (549,54)

Rf ($CH_2Cl_2/CH_3OH$ 95:5) : 0,48

b) 1,4-Dihydro-2,6-dimethyl-3-(2-methoxyethoxy)carbonyl-4-(3-nitrophenyl)-5-(2-aminoethoxy)carbonyl-pyridin

Hergestellt analog zu Beispiel 1b) aus 10,0 g (18,2 mmol) 1,4-Dihydro-2,6-dimethyl-3-(2-methoxyethoxy)carbonyl-4-(3-nitrophenyl)-5-(2-phthalimidoethoxy)carbonyl-pyridin und 2,65 ml (54,6 mmol) Hydrazinhydrat.

Nach chromatographischer Reinigung an Kieselgel mit Dichlormethan/Methanol (93:7) 4,0 g (52 %) gelbe Kristalle vom Schmp. 136-137 ° C.

$C_{20}H_{25}N_3O_7$ (419,43)

Rf ($CH_2Cl_2/CH_3OH$ 90:10) : 0,33

c) N[1]-Benzoyl-N[2]-[2-[1,4-dihydro-2,6-dimethyl-3-(2-methoxyethoxy)carbonyl-4-(3-nitrophenyl)-pyridin-5-carboxy] ethyl]-thioharnstoff

Hergestellt analog zu Beispiel 1c) aus 3,50 g (8,3 mmol) 1,4-Dihydro-2,6-dimethyl-3-(2-methoxyethoxy)-carbonyl-4-(3-nitrophenyl)-5-(2-aminoethoxy)carbonyl-pyridin und 1,36 g (8,3 mmol) Benzoylisothiocyanat. 4,65 g (96 %) hellgelbes Öl.

$C_{28}H_{30}N_4O_8S$ (582,43)

Rf ($CH_2Cl_2/CH_3OH$ 95:5) : 0,29

d) S-Methyl-N-[2-[1,4-dihydro-2,6-dimethyl-3-(2-methoxyethoxy) carbonyl-4-(3-nitrophenyl)-pyridin-5-carboxy]ethyl]-isothiuronium-jodid

Hergestellt aus 4,58 g (7,9 mmol) N[1]-Benzoyl-N[2]-[2-[1,4-dihydro-2,6-dimethyl-3-(2-methoxyethoxycarbonyl)-4-(3-nitrophenyl)-pyridin-5-carboxy]ethyl]-thioharnstoff analog zu Beispiel 1d). 2,60 g (53 %) gelber, amorpher Feststoff.

$C_{22}H_{29}JN_4O_7S$ (620,43)

Rf ($CH_2Cl_2/CH_3OH$ 90:10) : 0,55

e) N[1]-[2-[1,4-Dihydro-2,6-dimethyl-3-(2-methoxyethoxycarbonyl-4-(3-nitrophenyl)-pyridin-5-carboxy]ethyl]-N[2]-[3- (1H-imidazol-4-yl)propyl]-guanidin-hydrojodid

Hergestellt analog zu Beispiel 1e) aus 1,00 g (1,6 mmol) S-Methyl-N-[2-[1,4-dihydro-2,6-dimethyl-3-(2-methoxyethoxy) carbonyl-4-(3-nitrophenyl)-pyridin-5-carboxy]ethyl] isothiuronium-jodid und 0,20 g (1,6

mmol) 3-(1H-Imidazol-4-yl)propylamin. 0,51 g (46 %) gelber Feststoff vom Schmp. 94-96 °C.

$C_{27}$ $H_{36}$ J $N_7$ $O_7$ (697,53)

Rf ($CH_2Cl_2$/$CH_3OH$ 80:20) : 0,3

| | | | | | |
|---|---|---|---|---|---|
| $^1$H-NMR-Daten ($CD_3OD$, TMS als interner Standard) | δ = | 1,7-2,1 | (m) | 2H | |
| | | 2,3 | (s) | 3H | |
| | | 2,35 | (s) | 3H | |
| | | 2,65 | (t) | 2H | |
| | | 3,1-3,8 | (m) | 8H | |
| | | 3,3 | (s) | 3H | |
| | | 4,0-4,4 | (m) | 2H | |
| | | 4,8 | (breit) | 6H, | austauschbar mit $D_2O$ |
| | | 5,1 | (s) | 1H | |
| | | 6,9 | (s) | 1H | |
| | | 7,3-8,2 | (m) | 5H | ppm |

Beispiel 6

N[1]-[2-[1,4-Dihydro-2,6-dimethyl-3-isobutoxycarbonyl-4-(2-nitrophenyl)-pyridin-5-carboxy]ethyl]-N[2]-[3-(1H-imidazol-4-yl)propyl]-guanidin-hydrojodid

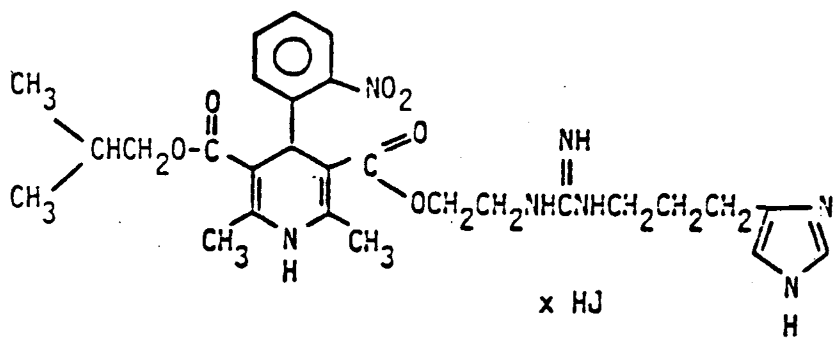

a) 1,4-Dihydro-2,6-dimethyl-3-isobutoxycarbonyl-4-(2-nitrophenyl)-5-(2-phthalimidoethoxy)carbonyl-pyridin

Hergestellt analog zu Beispiel 1a) aus 36,1 g (124 mmol) 2-(2-Nitrobenzyliden)-acetessigsäure-i-butylester und 34,0 g (124 mmol) 3-Amino-crotonsäure-(2-phthalimidoethyl)-ester. 44,1 g (69 %) gelbe Kristalle vom Schmp. 103-105 °C.

$C_{29}$ $H_{29}$ $N_3$ $O_8$ (547,56)

Rf ($CH_2Cl_2$/$CH_3OH$ 95:5) : 0,4

b) 1,4-Dihydro-2,6-dimethyl-3-isobutoxycarbonyl-4-(2-nitrophenyl)-5-(2-aminoethoxy)carbonyl-pyridin

Hergestellt nach Beispiel 1b) aus 10,94 g (20 mmol) 1,4-Dihydro-2,6-dimethyl-3-isobutoxycarbonyl-4-(2-nitrophenyl)-5-(2-phthalimidoethoxycarbonyl)-pyridin und 3,0 ml (60 mmol) Hydrazinhydrat. Man erhält 8,06 g (96 %) eines zähen gelben Öls, das ohne weitere Reinigung weiter umgesetzt wird.

$C_{21}$ $H_{27}$ $N_3$ $O_6$ (417,46)

Rf ($CH_2Cl_2$/$CH_3OH$ 95:5) : 0,2

c) N[1]-Benzoyl-N[2]-[2-[1,4-dihydro-2,6-dimethyl-3-isobutoxycarbonyl-4-(2-nitrophenyl)-pyridin-5-carboxy]-

ethyl]-thioharnstoff

Hergestellt analog zu Beispiel 1c) aus 7,86 g (18,8 mmol) 1,4-Dihydro-2,6-dimethyl-3-isobutoxycarbonyl-4-(2-nitrophenyl)-5-(2-aminoethoxy)carbonyl-pyridin und 3,07 g (18,8 mmol) Benzoylisothiocyanat in Essigester. 9,90 g (91 %) gelber, amorpher, Feststoff.

$C_{29}H_{32}N_4O_7S$ (580,66)

Rf ($CH_2Cl_2/CH_3OH$ 95:5) : 0,5

d) S-Methyl-N-[2-[1,4 dihydro-2,6-dimethyl-3-isobutoxycarbonyl-4-(2-nitrophenyl)-pyridin-5-carboxy]ethyl]-isothiuronium-jodid

Hergestellt analog zu Beispiel 1d) aus 3,05 g (5,3 mmol) $N^1$-Benzoyl-$N^2$-[2-[1,4-dihydro-2,6-dimethyl-3-isobutoxycarbonyl-4-(2-nitrophenyl)-pyridin-5-carboxy]ethyl]-thioharnstoff. Aus tert.-Butyl-methylether 3,10 g (94 %) gelbe Kristalle vom Schmp. 107-109 ° C

$C_{23}H_{31}JN_4O_6S$ (618,49)

Rf ($CH_2Cl_2/CH_3OH$ 90:10) : 0,4

e) $N^1$-[2-[1,4-Dihydro-2,6-dimethyl-3-isobutoxycarbonyl-4-(2-nitrophenyl)-pyridin-5-carboxy]ethyl]-$N^2$-[3-(1H-imidazol-4-yl)propyl]-guanidin-hydrojodid

1,00 g (1,62 mmol) S-Methyl-N-[2-[1,4-dihydro-2,6-dimethyl-3-isobutoxycarbonyl-4-(2-nitrophenyl)-pyridin-5-carboxy]ethyl]-isothiuronium-jodid und 0,20 g (1,62 mmol) 3-(1H-Imidazol-4-yl)propylamin werden in 20 ml Acetonitril 3 Std. gekocht. Nach Abdampfen des Lösungsmittels i.Vak. wird der erhaltene gelbe Schaum an Kieselgel mit Dichlormethan/Methanol (85:15) als Laufmittel chromatographiert. Nach Eindampfen der Hauptfraktion i.Vak. erhält man 0,68 g (60 %) der Titelverbindung als gelben, amorphen Feststoff.

$C_{28}H_{38}JN_7O_6$ (695,56)

Rf ($CH_2Cl_2/CH_3OH$ 80:20) : 0,5

| | | | |
|---|---|---|---|
| $^1$H-NMR-Daten ($CD_3OD$, TMS als interner Standard) $\delta$ = | 0,65-0,85 | (2d) | 6H |
| | 1,7-2,1 | (m) | 3H |
| | 2,35 | (s) | 6H |
| | 2,7 | (t) | 2H |
| | 3,1-4,5 | (m) | 6H |
| | 3,8 | (d) | 2H |
| | 4,9 | (breit) | 6H, austauschbar mit $D_2O$ |
| | 5,8 | (s) | 1H |
| | 6,95 | (s) | 1H |
| | 7,3-7,9 | (m) | 5H ppm |

Beispiel 7

$N^1$-[6-[1,4-Dihydro-2,6-dimethyl-3-methoxycarbonyl-4-(3-nitrophenyl)-pyridin-5-carboxy]hexyl]-$N^2$-[3-(1H-imidazol-4-yl)propyl]-guanidin-hydrojodid

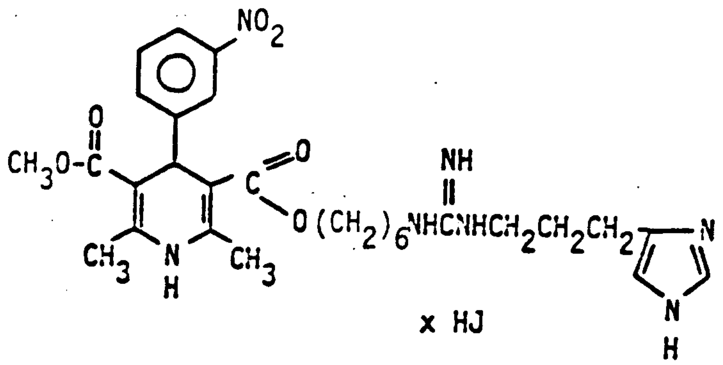

a) 1,4-Dihydro-2,6-dimethyl-3-methoxycarbonyl-4-(3-nitrophenyl)-5-(6-phthalimidohexyloxy)carbonyl-pyridin

12,0 g (26 mmol) 2-(3-Nitrobenzyliden)-acetessigsäure-(6-phthalidmidohexyl)ester und 3,0 g (26 mmol) 3-Aminocrotonsäuremethylester werden in 100 ml Isopropanol 4 Std. unter Rückfluß gekocht. Nach Abdampfen des Lösungsmittels i.Vak. werden 15,0 g eines braungelben Öls erhalten, das ohne weitere Reinigung weiter umgesetzt wird.

$C_{30}H_{31}N_3O_8$ (561,59)

Rf ($CH_2Cl_2/CH_3OH$ 97:3) : 0,5

b) 1,4-Dihydro-2,6-dimethyl-3-methoxycarbonyl-4-(3-nitrophenyl)-5-(6-aminohexyloxy)carbonyl-pyridin

Hergestellt analog zu Beispiel 1b) aus 15,0 g (26 mmol) 1,4-Dihydro-2,6-dimethyl-3-methoxycarbonyl-4-(3-nitrophenyl)-5-(6-phthalimidohexyloxy)carbonyl-pyridin und 3,9 ml (80 mmol) Hydrazinhydrat. 11,2 g (97 %) orangefarbenes Öl.

$C_{22}H_{29}N_3O_6$ (431,49)

Rf ($CH_2Cl_2/CH_3OH/N(C_2H_5)_3$ 90:10:1) : 0,2

c) N[1]-Benzoyl-N[2]-[6-[1,4-dihydro-2,6-dimethyl-3-methoxycarbonyl-4-(3-nitrophenyl)-pyridin-5-carboxy] hexyl]-thioharnstoff

Hergestellt analog zu Beispiel 1c) aus 10,0 g (23 mmol) 1,4-Dihydro-2,6-dimethyl-3-methoxycarbonyl-4-(3-nitrophenyl)-5-(6-aminohexyloxy)carbonyl-pyridin und 3,8 g (23 mmol) Benzoylisothiocyanat. Nach chromatographischer Reinigung an Kieselgel mit Dichlormethan/Methanol (95:5) als Laufmittel 10,5 g (76 %) orangefarbenes Öl.

$C_{30}H_{34}N_4O_7S$ (594,69)

Rf ($CH_2Cl_2/CH_3OH$ 99:1) : 0,2

d) S-Methyl-N-[6-[1,4-dihydro-2,6-dimethyl-3-methoxycarbonyl-4-(3-nitrophenyl)-pyridin-5-carboxy] hexyl]-isothiuronium-jodid

Hergestellt analog zu Beispiel 1d) aus 10,5 g (18 mmol) N[1]-Benzoyl-N[2]-[6-[1,4-dihydro-2,6-dimethyl-3-methoxycarbonyl-4-(3-nitrophenyl)-pyridin-5-carboxy]hexyl]-thioharnstoff. 3,66 g (33 %) gelber, amorpher Feststoff aus tert.-Butyl-methylether.

$C_{24}H_{33}JN_4O_6S$ (632,48)

Rf ($CH_2Cl_2/CH_3OH/N(C_2H_5)_3$ 90:10:1) : 0,3

e) N[1]-[6-[1,4-Dihydro-2,6-dimethyl-3-methoxycarbonyl-4-(3-nitrophenyl)-pyridin-5-carboxy]hexyl]-N[2]-[3-(1H-imidazol-4-yl)propyl]-guanidin-hydrojodid

Hergestellt analog zu Beispiel 1e) aus 1,00 g (1,6 mmol) S-Methyl-N-[6-[1,4-dihydro-2,6-dimethyl-3-methoxycarbonyl-4-(3-nitrophenyl)-pyridin-5-carboxy]hexyl]-isothiuronium-jodid und 0,20 g (1,6 mmol) 3-(1H-Imidazol-4-yl)propylamin. 1,1 g (97 %) blaßgelber, amorpher Feststoff.

$C_{29}H_{40}JN_7O_6$ (709,59)

Rf ($CH_3COOC_2H_5/CH_3OH/NH_4Cl/NH_3$-Puffer 50:47,5:2,5) : 0,7

| | | | |
|---|---|---|---|
| $^1$H-NMR-Daten ($CD_3OD$, TMS als interner Standard) δ = | 1,1-2,1 | (m) | 10H |
| | 2,3 | (s) | 3H |
| | 2,35 | (s) | 3H |
| | 2,7 | (t) | 2H |
| | 3,1-3,5 | (m) | 4H |
| | 3,7 | (s) | 3H |
| | 3,9-4,2 | (m) | 2H |
| | 4,9 | (breit) | 6H, austauschbar mit $D_2O$ |
| | 5,2 | (s) | 1H |
| | 7,0 | (s) | 1H |
| | 7,4-8,3 | (m) | 5H ppm |

Beispiel 8

N[1]-[2-[1,4-Dihydro-2,6-dimethyl-3-methoxycarbonyl-4-(2,3-dichlorphenyl)-pyridin-5-carboxy]ethyl]-N[2]-[3-(1H-imidazol-4-yl)propyl]-guanidin-hydrojodid

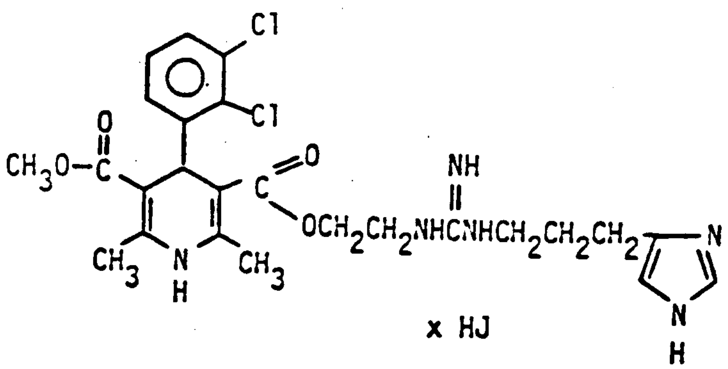

a) 1,4-Dihydro-2,6-dimethyl-3-methoxycarbonyl-4-(2,3-dichlorphenyl)-5-(2-phthalimidoethoxy)carbonyl-pyridin

Hergestellt analog zu Beispiel 1a) aus 13,0 g (48 mmol) 2-(2,3-Dichlorbenzyliden)-acetessigsäure-methylester und 13,1 g (48 mmol) 3-Amino-crotonsäure-(2-phthalimido-ethyl)ester. 14,2 g (57 %) blaßgelbe Kristalle vom Schmp. 195-196 ° C.

$C_{26}H_{22}Cl_2N_2O_6$ (529,38)

Rf ($CH_2Cl_2/CH_3OH$ 95:5) : 0,5

b) 1,4-Dihydro-2,6-dimethyl-3-methoxycarbonyl-4-(2,3-dichlorphenyl)-5-(2-aminoethoxy)carbonyl-pyridin

Hergestellt analog zu Beispiel 1b) aus 10,0 g (19 mmol) 1,4-Dihydro-2,6-dimethyl-3-methoxycarbonyl-4-(2,3-dichlorphenyl)-5-(2-phthalimidoethoxy)carbonyl-pyridin und 2,84 ml (57 mmol) Hydrazinhydrat. Aus tert.-Butylmethylether 3,85 g (51 %) blaßgelbe Kristalle vom Schmp. 166-167 ° C.

$C_{18}H_{20}Cl_2N_2O_4$ (399,27)

Rf ($CH_2Cl_2/CH_3OH$ 80:20) : 0,4

c) N[1]-Benzoyl-N[2]-[2-[1,4-dihydro-2,6-dimethyl-3-methoxycarbonyl-4-(2,3-dichlorphenyl)-pyridin-5-carboxy]-ethyl]-thioharnstoff

Hergestellt analog zu Beispiel 1c) aus 2,00 g (5mmol) 1,4-Dihydro-2,6-dimethyl-3-methoxycarbonyl-4-(2,3-dichlorphenyl)-5-(2-aminoethoxy)carbonyl-pyridin und 0,82 g (5 mmol) Benzoylisothiocyanat. 2,63 g (93 %) amorpher Feststoff.

$C_{26}H_{25}Cl_2N_3O_5S$ (562,47)

Rf ($CH_2Cl_2/CH_3OH$ 90:10) : 0,8

d) S-Methyl-N-[2-[1,4-dihydro-2,6-dimethyl-3-methoxycarbonyl-4-(2,3-dichlorphenyl)-pyridin-5-carboxy]-ethyl]-isothiuronium-jodid

Hergestellt analog zu Beispiel 1d) aus 2,50 g (4,4 mmol) $N^1$-Benzoyl-$N^2$-[2-[1,4-dihydro-2,6-dimethyl-3-methoxycarbonyl-4-(2,3-dichlorphenyl)-pyridin-5-carboxy]ethyl]-thioharnstoff. 2,26 g (86 %) gelber, amorpher Feststoff

$C_{20}H_{24}Cl_2JN_3O_4S$ (600,30)

Rf ($CH_2Cl_2/CH_3OH$ 90:10) : 0,3

e) $N^1$-[2-[1,4-Dihydro-2,6-dimethyl-3-methoxycarbonyl-4-(2,3-dichlorphenyl)-pyridin-5-carboxy]ethyl]-$N^2$-[3-(1H-imidazol-4-yl)propyl]-guanidin-hydrojodid

Hergestellt analog zu Beispiel 1e) aus 1,00 g (1,67 mmol) S-Methyl-N-[2-[1,4-dihydro-2,6-dimethyl-3-methoxycarbonyl-4-(2,3-dichlorphenyl-pyridin-5-carboxyl]ethyl]-isothiuronium-jodid und 0,21 g (1,67 mmol) 3-(1H-Imidazol-4-yl)propylamin. 0,98 g (87 %) gelber, amorpher Schaum.

$C_{25}H_{31}Cl_2JN_6O_4$ (677,42)

Rf ($CH_3COOC_2H_5/CH_3OH$ 80:20) : 0,2

| | | | | |
|---|---|---|---|---|
| $^1$H-NMR-Daten ($CD_3OD$, TMS als interner Standard) | $\delta$ = 1,7-2,1 | (m) | 2H | |
| | 2,25 | (s) | 3H | |
| | 2,3 | (s) | 3H | |
| | 2,7 | (t) | 2H | |
| | 3,1-3,7 | (m) | 4H | |
| | 3,6 | (s) | 3H | |
| | 4,0-4,4 | (m) | 2H | |
| | 4,9 | (breit) | 6H, | austauschbar mit $D_2O$ |
| | 5,5 | (s) | 1H | |
| | 6,95 | (s) | 1H | |
| | 7,1-7,6 | (m) | 3H | |
| | 7,7 | (s) | 1H | ppm |

Beispiel 9

$N^1$-[2-[1,4-Dihydro-2,6-dimethyl-3-nitro-4-(2-methylphenyl)-pyridin-5-carboxy] ethyl]-$N^2$-[3-(1H-imidazol-4-yl)-propyl]-guanidin-hydrojodid

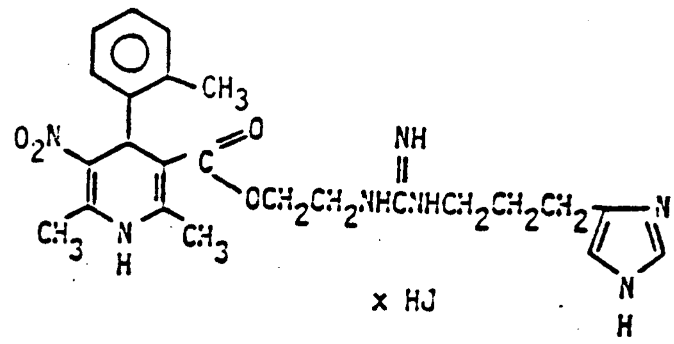

a) N[1]-Benzoyl-N[2]-[2-[1,4-dihydro-2,6-dimethyl-3-nitro-4-(2-methylphenyl)-pyridin-5-carboxy]ethyl]-thioharnstoff

Hergestellt nach Beispiel 1c) aus 2,49 g (7,5 mmol) 1,4-Dihydro-2,6-dimethyl-3-nitro-4-(2-methylphenyl)-5-(2-aminoethoxy)carbonyl-pyridin und 1,22 g (7,5 mmol) Benzoylisothiocyanat. Aus tert.-Butylmethylether 3,57 g (96 %) gelbe Kristalle vom Schmp. 85-87 ° C.
$C_{25}$ $H_{26}$ $N_4$ $O_5$ S (494,57)
Rf ($CH_2Cl_2$/$CH_3OH$ 90:10) : 0,7

b) S-Methyl-N-[2-[1,4-dihydro-2,6-dimethyl-3-nitro-4-(2-methylphenyl)-pyridin-5-carboxy]ethyl]-isothiuronium-jodid

Hergestellt analog zu Beispiel 1d) aus 2,52 g (5,1 mmol) N[1]-Benzoyl-N[2]-[2-[1,4-dihydro-2,6-dimethyl-3-nitro-4-(2-methylphenyl)-pyridin-5-carboxy]ethyl]-thioharnstoff. Aus tert.-Butylmethylether/Dichlormethan (9:1) 1,68 g (62 %) gelber, amorpher Feststoff.
$C_{19}$ $H_{25}$ J $N_4$ $O_4$ S (532,40)
Rf ($CH_2Cl_2$/$CH_3OH$ 90:10) : 0,25

c) N[1]-[2-[1,4-Dihydro-2,6-dimethyl-3-nitro-4-(2-methylphenyl)-pyridin-5-carboxy]ethyl]-N[2]-[3-(1H-imidazol-4-yl)propyl]-guanidin-hydrojodid

Hergestellt analog zu Beispiel 1e) aus 1,00 g (1,88 mmol) S-Methyl-N-[2-[1,4-dihydro-2,6-dimethyl-3-nitro-4-(2-methylphenyl)-pyridin-5-carboxy]ethyl]isothiuronium-jodid und 0,23 g (1,88 mmol) 3-(1H-Imidazol-4-yl)propylamin in Acetonitril 0,65 g (56 %) gelbe Kristalle vom Schmp. 85-87 ° C.
$C_{24}$ $H_{32}$ J $N_7$ $O_4$ (609,47)
Rf ($C_2H_5OH$, $Al_2O_3$ neutral) : 0,35

| | | | | |
|---|---|---|---|---|
| $^1$H-NMR-Daten ($CD_3OD$, TMS als interner Standard) $\delta$ = | 1,7-2,1 | (m) | 2H | |
| | 2,3 | (s) | 3H | |
| | 2,5 | (s) | 3H | |
| | 2,6 | (s) | 3H | |
| | 2,65 | (t) | 2H | |
| | 3,1-3,6 | (m) | 4H | |
| | 4,0-4,4 | (m) | 2H | |
| | 5,0 | (breit) | 6H, | austauschbar mit $D_2O$ |
| | 5,5 | (s) | 1H | |
| | 6,9 | (s) | 1H | |
| | 6,9-7,3 | (m) | 4H | |
| | 7,6 | (s) | 1H | ppm |

Beispiel 10

$N^1$-[2-[1,4-Dihydro-2,6-dimethyl-3-nitro-4-(2-trifluormethylphenyl)-pyridin-5-carboxy]ethyl]-$N^2$-[3-(1H-imidazol-4-yl) propyl]-guanidin-hydrojodid

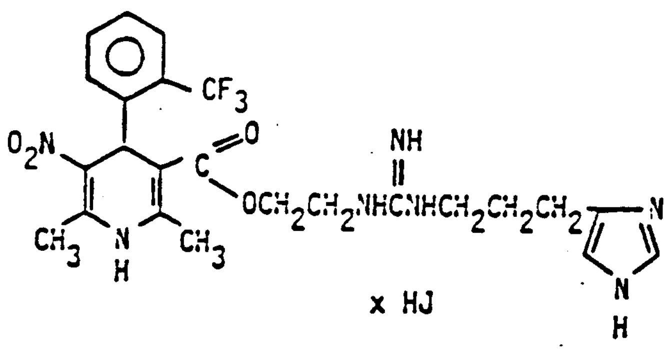

a) $N^1$-Benzoyl-$N^2$-[2-[1,4-dihydro-2,6-dimethyl-3-nitro-4-(2-trifluormethylphenyl)-pyridin-5-carboxy]ethyl]-thioharnstoff

Hergestellt analog zu Beispiel 1c) aus 3,00 g (7,8 mmol) 1,4-Dihydro-2,6-dimethyl-3-nitro-4-(2-trifluormethylphenyl)-5-(2-aminoethoxy)carbonyl-pyridin und 1,27 g (7,8 mmol) Benzoylisothiocyanat. 4,01 g (94 %) gelbe Kristalle vom Schmp. 83-85 ° C.

$C_{25}H_{23}F_3N_4O_5S$ (548,35)

Rf ($CH_2Cl_2/CH_3OH$ 95:5) : 0,55

b) S-Methyl-N-[2-[1,4-dihydro-2,6-dimethyl-3-nitro-4-(2-trifluormethylphenyl)-pyridin-5-carboxy]ethyl]-isothiuronium-jodid

Hergestellt analog zu Beispiel 1d) aus 3,9 g (7,1 mmol) $N^1$-Benzoyl-$N^2$-[2-[1,4-dihydro-2,6-dimethyl-3-nitro-4-(2-trifluormethylphenyl)-pyridin-5-carboxy]ethyl]-thioharnstoff.

Aus Dichlormethan 2,23 g (54 %) amorpher, tiefgelber Feststoff.

$C_{19}H_{22}F_3JN_4O_4S$ (586,37)

Rf ($CH_2Cl_2/CH_3OH$ 90:10) : 0,43

c) N[1]-[2-[1,4-Dihydro-2,6-dimethyl-3-nitro-4-(2-trifluormethylphenyl)-pyridin-5-carboxy]ethyl]-N[2]-[3-(1H-imidazol-4-yl)propyl]-guanidin-hydrojodid

Hergestellt analog zu Beispiel 1e) aus 1,00 g (1,7 mmol) S-Methyl-N-[2-[1,4-dihydro-2,6-dimethyl-3-nitro-4-(2-trifluormethylphenyl)-pyridin-5-carboxy]ethyl]-isothiuronium-jodid und o,21 g (1,7 mmol) 3-(1H-Imidazol-4-yl)propylamin. 0,40 g (35 %) orangegelber, amorpher Feststoff.

$C_{24}H_{29}F_3JN_7O_4$ (663,44)

Rf ($CH_3COOC_2H_5/C_2H_5OH$ 50:50 auf $Al_2O_3$ neutral) : 0,28

| | | | | |
|---|---|---|---|---|
| $^1$H-NMR-Daten ($CD_3OD$, TMS als interner Standard) $\delta$ = | 1,7-2,1 | (m) | 2H | |
| | 2,35 | (s) | 3H | |
| | 2,5 | (s) | 3H | |
| | 2,7 | (t) | 2H | |
| | 3,2 | (t) | 2H | |
| | 3,45 | (t) | 2H | |
| | 3,9-4,5 | (m) | 2H | |
| | 5,0 | (breit) | 6H, | austauschbar mit $D_2O$ |
| | 5,95 | (s) | 1H | |
| | 6,9 | (s) | 1H | |
| | 7,2-7,8 | (m) | 5H | ppm |

Beispiel 11

N[1]-[6-[1;4-Dihydro-2,6-dimethyl-3-methoxycarbonyl-4-(2-nitrophenyl)-pyridin-5-carboxy]hexyl]-N[2]-[3-(1H-imidazol-4-yl) propyl]-guanidin-hydrojodid

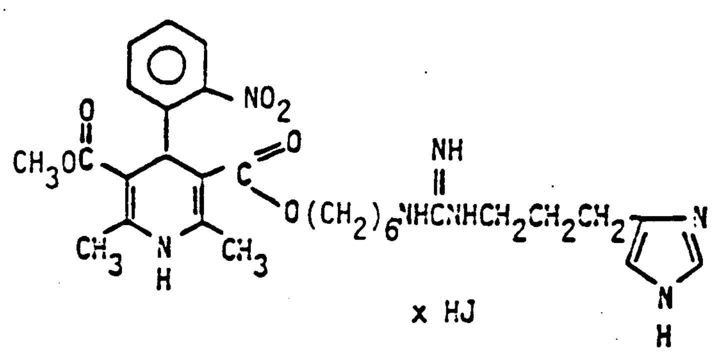

a) 1,4-Dihydro-2,6-dimethyl-3-methoxycarbonyl-4-(2-nitrophenyl)-5-(6-phthalimidohexyloxy)carbonyl-pyridin

Hergestellt analog zu Beispiel 7a) aus 23,0 g (50 mmol) 2-(2-Nitrobenzyliden)-acetessigsäure-(6-phthalimidohexyl) ester und 5,7 g (50 mmol) 3-Amino-crotonsäuremethylester. Nach säulenchromatographischer Reinigung an Kieselgel mit Dichlormethan/Methanol (98:2) als Laufmittel 9,7 g (35 %) dunkelgelbes Öl.

$C_{30}H_{31}N_3O_8$ (561,59)

Rf ($CH_2Cl_2/CH_3OH$ 98:2) : 0,3

b) 1,4-Dihydro-2,6-dimethyl-3-methoxycarbonyl-4-(2-nitrophenyl)-5-(6-aminohexyloxy)carbonyl-pyridin

Hergestellt analog zu Beispiel 1b) aus 9,1 g (16 mmol) 1,4-Dihydro-2,6-dimethyl-3-methoxycarbonyl-4-(2-nitrophenyl)-5-(6-phthalimidohexyloxy)carbonyl-pyridin und 2,4 ml (49 mmol) Hydrazinhydrat. 6,2 g (89 %) orangefarbenes Öl.

$C_{22}$ $H_{29}$ $N_3$ $O_6$ (431,49)

Rf ($CH_2Cl_2/CH_3OH/N(C_2H_5)_3$ 90:10:1) : 0,2

c) N[1]-Benzoyl-N[2]-[6-[1,4-dihydro-2,6-dimethyl-3-methoxycarbonyl-4-(2-nitrophenyl)-pyridin-5-carboxy]hexyl]-thioharnstoff

Hergestellt aus 5,0 g (11,6 mmol) 1,4-Dihydro-2,6-dimethyl-3-methoxycarbonyl-4-(2-nitrophenyl)-5-(6-aminohexyloxy) carbonyl-pyridin und 1,9 g (11,6 mmol) Benzoylisothiocyanat analog zu Beispiel 1c). Nach chromatographischer Reinigung an Kieselgel mit Dichlormethan/Methanol (95:5) als Laufmittel erhält man 4,7 g (68 %) eines orangegelben, amorphen Feststoffs.

$C_{30}$ $H_{34}$ $N_4$ $O_7$ S (594,69)

Rf ($CH_2Cl_2/CH_3OH$ 95:5) : 0,7

d) S-Methyl-N-[6-[1,4-dihydro-2,6-dimethyl-3-methoxycarbonyl-4-(2-nitrophenyl)-pyridin-5-carboxy]hexyl]-isothiuronium-jodid

Hergestellt analog zu Beispiel 7d) aus 6,9 g (11,6 mmol) N[1]-Benzoyl-N[2]-[6-[1,4-dihydro-2,6-dimethyl-3-methoxycarbonyl-4-(2-nitrophenyl)-pyridin-5-carboxy]hexyl]-thioharnstoff. 2,7 g (37 %) gelber, amorpher Feststoff.

$C_{24}$ $H_{33}$ J $N_4$ $O_6$ S (632,48)

Rf ($CH_2Cl_2/CH_3OH/N(C_2H_5)_3$ 90:10:1) : 0,4

e) N[1]-[6-[1,4-Dihydro-2,6-dimethyl-3-methoxycarbonyl-4-(2-nitrophenyl)-pyridin-5-carboxy]hexyl]-N[2]-[3-(1H-imidazol-4-yl)propyl]-guanidin-hydrojodid

Hergestellt analog zu Beispiel 1e) aus 1,00 g (1,6 mmol) S-Methyl-N-[6-[1,4-dihydro-2,6-dimethyl-3-methoxycarbonyl-4-(2-nitrophenyl)-pyridin-5-carboxy]hexyl]-isothiuronium-jodid und 0,20 g (1,6 mmol) 3-(1H-Imidazol-4-yl)-propylamin. 0,98 g (87 %) gelber amorpher Feststoff.

$C_{29}$ $H_{40}$ J $N_7$ $O_6$ (709,59)

Rf ($CH_3COOC_2H_5/CH_3OH/NH_4Cl/NH_3$-Puffer 50:47,5:2,5) : 0,7

| | | | | |
|---|---|---|---|---|
| $^1$H-NMR-Daten ($CD_3OD$, TMS als interner Standard) | $\delta$ = | 1,1-1,8 | (m) | 8H |
| | | 1,8-2,1 | (m) | 2H |
| | | 2,3 | (s) | 3H |
| | | 2,4 | (s) | 3H |
| | | 2,7 | (t) | 2H |
| | | 3,1-3,4 | (m) | 4H |
| | | 3,6 | (s) | 3H |
| | | 3,9-4,2 | (m) | 2H |
| | | 4,8 | (breit) | 6H, austauschbar mit $D_2O$ |
| | | 5,8 | (s) | 1H |
| | | 6,95 | (s) | 1H |
| | | 7,2-7,9 | (m) | 5H ppm |

Beispiel 12

3-[1,4-Dihydro-2,6-dimethyl-3-methoxycarbonyl-4-(2-nitrophenyl)-pyridin-5-carboxy]-N-[3-(1H-imidazol-4-yl)-

propyl]-propionamidin-hydrochlorid

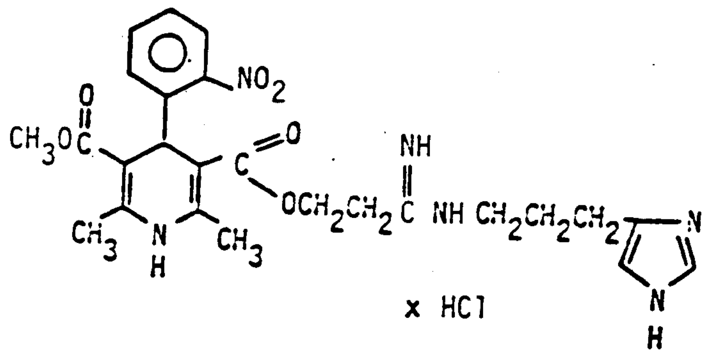

a) 1,4-Dihydro-2,6-dimethyl-3-methoxycarbonyl-4-(2-nitrophenyl)-5-(2-cyanethoxy)carbonyl-pyridin

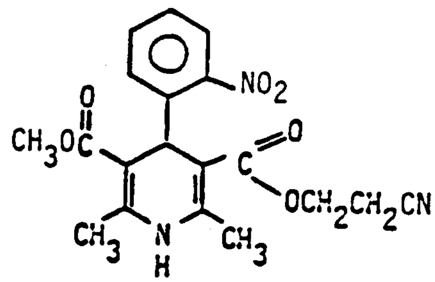

Hergestellt analog zu Beispiel 1a) aus 24,9 g (100 mmol) 2-(2-Nitrobenzyliden)-acetessigsäuremethylester und 15,4 g (100 mmol) 3-Amino-crotonsäure-(2-cyanethyl)ester in Ethanol. Nach Umkristallisieren aus Methanol 23,8 g (62 %) hellgelbe Kristalle vom Schmp. 153-154 °C.

$C_{19}H_{19}N_3O_6$ (385,38)

Rf ($CH_2Cl_2/C_2H_5OH$ 97:3) : 0,42

b) 3-[1,4-Dihydro-2,6-dimethyl-3-methoxycarbonyl-4-(2-nitrophenyl)-pyridin-5-carboxy]-propionimidsäure-methylester-dihydrochlorid

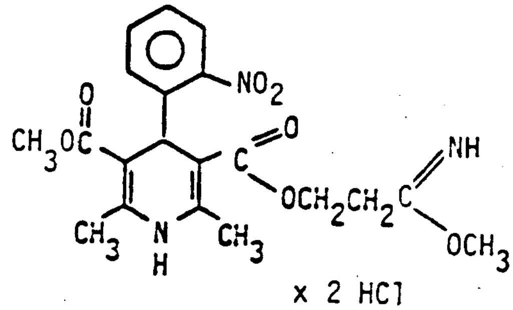

5,0 g (13 mmol) 1,4-Dihydro-2,6-dimethyl-3-methoxycarbonyl-4-(2-nitrophenyl)-5-(2-cyanethoxy)-carbonyl-pyridin werden in 15 ml trockenem Methanol und 20 ml Dichlormethan gelöst und auf 0 °C gekühlt. Bei einer Innentemperatur von 0-5 °C wird dann trockenes Chlorwasserstoffgas bis zur Sättigung eingeleitet (ca. 3 Std.). Die erhaltene Lösung wird nach 20-stündigem Aufbewahren bei 0 °C i.Vak. bei einer Badtemperatur von max. 20 °C eingedampft. Der Rückstand wird zweimal mit 20 ml Dichlormethan

aufgenommen und i. Vak. eingedampft. Es verbleiben 6,4 g eines gelben, hygroskopischen Schaums, der ohne Reinigung weiter umgesetzt wird.

$C_{20}H_{25}Cl_2N_3O_7$ (490,34)

Rf ($CH_2Cl_2/CH_3OH/N(C_2H_5)_3$ 90:10:1) : 0,6

c) 3-[1,4-Dihydro-2,6-dimethyl-3-methoxycarbonyl-4-(2-nitrophenyl)-pyridin-5-carboxy]-N-[3-(1H-imidazol-4-yl)-propyl]-propionamidin-hydrochlorid

5,9 g (12 mmol) 3-[1,4-Dihydro-2,6-dimethyl-3-methoxycarbonyl-4-(2-nitrophenyl)-pyridin-5-carboxy]-propionimidsäuremethylester-dihydrochlorid und 1,51 g (12 mmol) 3-(1H-Imidazol-4-yl)propylamin werden in 50 ml Methanol 30 Std. bei Raumtemperatur gerührt. Das nach Abdampfen des Lösungsmittels i. Vak. erhaltene, amorphe Rohprodukt wird durch Chromatographie an Kieselgel mit Ethylacetat/Methanol (70:30) als Laufmittel gereinigt. Die Hauptfraktion ergibt nach Eindampfen i.Vak. 2.05 g (31%) eines gelben, amorphen Feststoffs.

$C_{25}H_{31}ClN_6O_6$ (547,01)

Rf ($CH_3COOC_2H_5/CH_3OH$ 70:30) : 0,3

| | | | | |
|---|---|---|---|---|
| $^1$H-NMR-Daten ($CD_3OD$, TMS als interner Standard) | δ = | 1,8-2,2 | (m) | 2H |
| | | 2,3 | (s) | 3H |
| | | 2,35 | (s) | 3H |
| | | 2,6-3,0 | (m) | 4H |
| | | 3,2-3,5 | (t) | 2H |
| | | 3,55 | (s) | 3H |
| | | 4,0-4,8 | (m) | 2H |
| | | 5,1 | (breit) | 5H, austauschbar mit $D_2O$ |
| | | 5,7 | (s) | 1H |
| | | 6,95 | (s) | 1H |
| | | 7,2-7,9 | (m) | 5H ppm |

Beispiel 13

3-[1,4-Dihydro-2,6-dimethyl-3-methoxycarbonyl-4-(2-nitrophenyl)-pyridin-5-carboxy]-N-[2-(1H-imidazol-4-yl) ethyl]-propionamidin-hydrochlorid

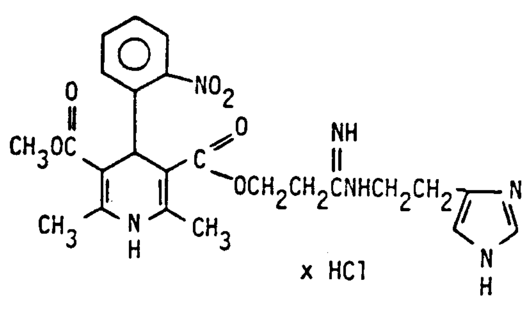

Hergestellt analog zu Beispiel 12 c) aus 0,65 g (1,3 mmol) 3-[1,4-Dihydro-2,6-dimethyl-3-methoxycarbonyl-4-(2-nitrophenyl)-pyridin-5-carboxy]-propionimidsäuremethylesterdihydrochlorid und 0,15 g (1,3 mmol) 2-(1H-Imidazol-4-yl) ethylamin. Nach chromatographischer Reinigung an Kieselgel mit Ethylacetat/Methanol (70:30) als Laufmittel 0,20 g (28 %) eines gelben, amorphen Feststoffs.

$C_{24}H_{29}ClN_6O_6$ (533,00)
Rf ($CH_3COOC_2H_5/CH_3OH$ 50:50) : 0,5

| | | | | |
|---|---|---|---|---|
| $^1$H-NMR-Daten ($CD_3OD$, TMS als interner Standard) | δ = 2,3 | (s) | 3H | |
| | 2,35 | (s) | 3H | |
| | 2,7-3,1 | (m) | 4H | |
| | 3,5 | (t) | 2H | |
| | 3,55 | (s) | 3H | |
| | 4,0-4,7 | (m) | 2H | |
| | 5,0 | (breit) | 5H, | austauschbar mit $D_2O$ |
| | 5,7 | (s) | 1H | |
| | 7,0 | (s) | 1H | |
| | 7,2-7,8 | (m) | 5H | ppm |

Beispiel 14

4-[1,4-Dihydro-2,6-dimethyl-3-methoxycarbonyl-4-(2-nitrophenyl)-pyridin-5-carboxy]-N-[3-(1H-imidazol-4-yl)-propyl]-butyramidin-hydrochlorid

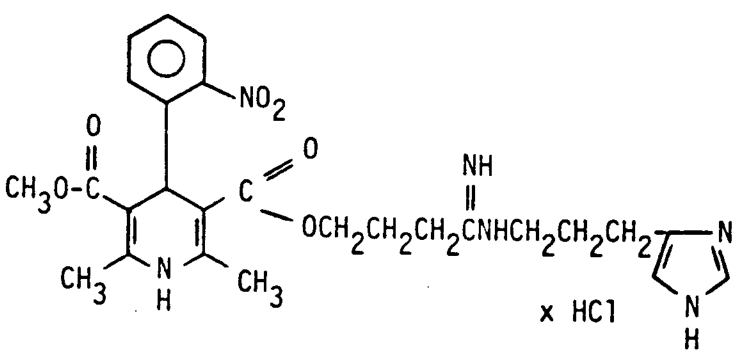

a) 1,4-Dihydro-2,6-dimethyl-3-methoxycarbonyl-4-(2-nitrophenyl)-5-(3-chlorpropoxy)carbonyl-pyridin

8,93 g (50 mmol) Acetessigsäure-(3-chlorpropyl)ester und 4,00 g (50 mmol) Ammoniumacetat werden in 50 ml Ethanol 1,5 Std. unter Rückfluß und Stickstoffatmosphäre gekocht. Nach Zugabe von 12,50 g (50 mmol) 2-(2-Nitrobenzyliden)-acetessigsäuremethylester wird 12 Std. weitergekocht. Die Lösung wird i. Vak. eingeengt und der erhaltene Rückstand an Kieselgel mit Dichlormethan/Methanol (95:5) als Laufmittel chromatographisch gereinigt. Die polare gelbe Hauptfraktion ergibt nach Eindampfen i. Vak. 4,10 g (20 %) eines gelben Öls.
$C_{19}H_{21}ClN_2O_6$ (408,84)
Rf ($CH_2Cl_2/CH_3OH$ 95:5): 0,7

b) 1,4-Dihydro-2,6-dimethyl-3-methoxycarbonyl-4-(2-nitrophenyl)-5-(3-cyanpropoxy)carbonyl-pyridin

Zu einer Lösung von 4,09 g (10 mmol) 1,4-Dihydro-2,6-dimethyl-3-methoxycarbonyl-4-(2-nitrophenyl)-5-(3-chlorpropoxy)carbonyl-pyridin in 10 ml Dimethylsulfoxid werden bei 60° C Innentemperatur 0,55 g (11 mmol) Natriumcyanid in 15 ml Dimethylsulfoxid getropft. Das Reaktionsgemisch wird dann 2,5 Std. bei 120° C gerührt und nach Abkühlen in 150 ml Eiswasser gegossen. Die Mischung wird mit 3 x 50 ml

Essigester extrahiert. Nach Trocknen und Einengen der organischen Phasen i. Vak. werden 5,7 g eines braunen Öls erhalten, das an Kieselgel mit Dichlormethan/Methanol (99:1) chromatographiert wird. Die Hauptfraktion ergibt nach Eindampfen i. Vak. und Umkristallisation des Rückstands aus Methanol 2,7 g (68 %) gelbe Kristalle vom Schmp. 48 - 51 ° C.

$C_{20}H_{21}N_3O_6$ (399,40)
Rf ($CH_2Cl_2/CH_3OH$ 95:5): 0,5

c) 4-[1,4-Dihydro-2,6-dimethyl-3-methoxycarbonyl-4-(2-nitrophenyl)-pyridin-5-carboxy]-butyrimidsäuremethylester-dihydrochlorid

Analog zu Beispiel 12 b) werden aus 2,30 g (5,8 mmol) 1,4-Dihydro-2,6-dimethyl-3-methoxycarbonyl-4-(2-nitrophenyl)-5-(3-cyanpropoxy)carbonyl-pyridin 2,6 g (89 %) eines gelben Schaums erhalten, die ohne weitere Reinigung umgesetzt werden.

$C_{21}H_{27}Cl_2N_3O_7$ (504,36)
Rf ($CH_2Cl_2/CH_3OH/N(C_2H_5)_3$ 90:10:1): 0,6

d) 4-[1,4-Dihydro-2,6-dimethyl-3-methoxycarbonyl-4-(2-nitrophenyl)-pyridin-5-carboxy]-N-[3-(1H-imidazol-4-yl)propyl]-butyramidin-hydrochlorid

Hergestellt analog zu Beispiel 12 c) aus 1,00 g (2 mmol) 4-[1,4-Dihydro-2,6-dimethyl-3-methoxycarbonyl-4-(2-nitrophenyl)-pyridin-5-carboxy] -butyrimidsäuremethylester-dihydrochlorid und 0,25 g (2 mmol) 3-(1H-Imidazol-4-yl)propylamin. Nach chromatographischer Reinigung an Kieselgel mit Ethylacetat/Methanol (70:30) als Laufmittel 0,30 (27 %) gelber, erstarrter Schaum.

$C_{26}H_{33}ClN_6O_6$ (561,03)
Rf ($CH_3COOC_2H_5/CH_3OH$ 1:1): 0,4

$^1$H-NMR-Daten (CD$_3$OD, TMS als interner Standard): $\delta$ = 1,8 - 2,2 (m) 4 H,
2,3 (s) 3 H,
2,35 (s) 3 H,
2,4 - 2,9 (m) 4 H,
3,4 (t) 2 H,
3,6 (s) 3 H,
3,8 - 4,5 (m) 2 H,
5,0 (breit) 5 H, austauschbar mit $D_2O$
5,8 (s) 1 H,
7,0 (s) 1 H,
7,2 - 7,9 (m) 5 H ppm.

Beispiel 15

N[1]-[4-[1,4-Dihydro-2,6-dimethyl-3-methoxycarbonyl-4-(2-nitrophenyl)-pyridin-5-carboxy]butyl]-N[2]-[3-(1H-imidazol-4-yl)propyl]-guanidin-hydrojodid

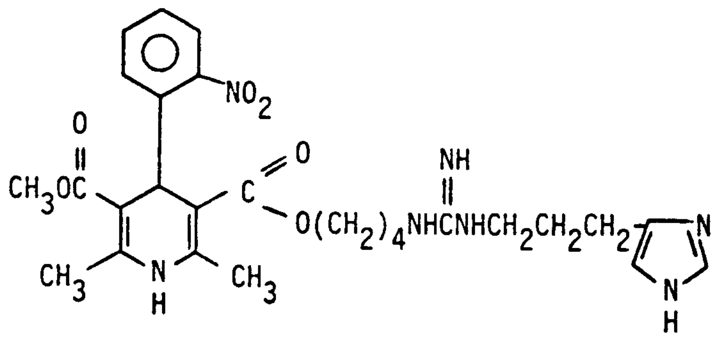

x HJ

a) 1,4-Dihydro-2,6-dimethyl-3-methoxycarbonyl-4-(2-nitrophenyl)-5-(4-phthalimido-butoxy)carbonyl-pyridin

13,7 g (55 mmol) 2-(2-Nitrobenzyliden)-acetessigsäuremethylester und 16,6 g (55 mmol) 3-Amino-crotonsäure-(4-phthalimido-butyl)ester werden in 150 ml Ethanol 18 Std. gekocht. Das nach Abdampfen des Lösungsmittels i. Vak. erhaltene orangegelbe Öl (28,7 g - 98 %) wird ohne weitere Reinigung weiter umgesetzt.

$C_{28}H_{27}N_3O_8$ (533,54)

Rf ($CH_2Cl_2$/$CH_3OH$ 97:3): 0,44

b) 1,4-Dihydro-2,6-dimethyl-3-methoxycarbonyl-4-(2-nitrophenyl)-5-(4-aminobutoxy)carbonyl-pyridin

Hergestellt analog zu Beispiel 1 b) aus 27,0 g (51 mmol) 1,4-Dihydro-2,6-dimethyl-3-methoxycarbonyl-4-(2-nitrophenyl)-5-(4-phthalimido-butoxy) carbonyl-pyridin und 7,4 ml (152 mmol) Hydrazinhydrat. Nach chromatographischer Reinigung an Kieselgel mit Dichlormethan/Methanol (95:5) als Laufmittel 16,0 g (78 %) orangegelbes Öl.

$C_{20}H_{25}N_3O_6$ (403,44)

Rf ($CH_2Cl_2$/$CH_3OH$/$NH_3$ konz. 50:50:1): 0,38

c) $N^1$-Benzoyl-$N^2$-[4-[1,4-dihydro-2,6-dimethyl-3-methoxycarbonyl -4-(2-nitrophenyl)-pyridin-5- carboxy]-butyl]-thioharnstoff

Analog zu Beispiel 1 c) aus 6,50 g (16 mmol) 1,4-Dihydro-2,6-dimethyl-3-methoxycarbonyl-4-(2-nitrophenyl)-5-(4-aminobutoxy)carbonyl-pyridin und 2,63 g (16 mmol) Benzoylisothiocyanat. Nach Säulenchromatographie an Kieselgel mit Dichlormethan/Methanol (98:2) als Laufmittel 4,38 g (48 %) eines hellgelben, amorphen Feststoffs.

$C_{28}H_{30}N_4O_7S$ (566,44)

Rf ($CH_2Cl_2$/$CH_3OH$ 95:5): 0,75

d) S-Methyl-N-[4-[1,4-dihydro-2,6-dimethyl-3-methoxycarbonyl-4-(2-nitrophenyl)-pyridin-5-carboxy]butyl]-isothiuronium-jodid

Aus 3,5 g (6,2 mmol) $N^1$-Benzoyl-$N^2$-[4-[1,4-dihydro-2,6-dimethyl-3-methoxycarbonyl-4-(2-nitrophenyl)-pyridin-5-carboxy]butyl]-thioharnstoff analog zu Beispiel 1 d). 3,15 g (84 %) hellgelber, amorpher Feststoff.

$C_{22}H_{29}JN_4O_6S$ (604,46)

Rf ($CH_3COOC_2H_5$/$CH_3OH$ 70:30): 0,73

e) $N^1$-[4-[1,4-Dihydro-2,6-dimethyl-3-methoxycarbonyl-4-(2-nitrophenyl)-pyridin-5-carboxy]butyl]-$N^2$-[3-(1H-imidazol-4-yl)propyl] -guanidin-hydrojodid

Hergestellt analog zu Beispiel 1 e) aus 1,00 g (1,65 mmol) S-Methyl-N-[4-[1,4-dihydro-2,6-dimethyl-3-methoxycarbonyl-4-(2-nitrophenyl)-pyridin -5-carboxy]butyl]-isothiuronium-jodid und 0,21 g (1.65 mmol) 3-(1H-Imidazol-4-yl)propylamin. 0,36 g (32 %) gelber, amorpher Feststoff.

$C_{27}H_{36}JN_7O_6$ (681,53)
Rf ($CH_3COOC_2H_5$/$CH_3OH$ 70:30): 0,46

| | |
|---|---|
| $^1H$-NMR-Daten | δ = 1,3 - 1,7 (m) 4 H, |
| ($CD_3OD$, TMS als | 1,8 - 2,2 (m) 2 H, |
| interner Standard): | 2,3 (s) 3 H, |
| | 2,4 (s) 3 H, |
| | *2,6 - 2,9 (t) 2 H, |
| | 3,1 - 3,5 (m) 4 H, |
| | 3,6 (s) 3 H, |
| | 3,9 - 4,3 (m) 2 H, |
| | 4,9 (breit) 6 H, austauschbar mit $D_2O$ |
| | 5,75 (s) 1 H, |
| | 7,05 (s) 1 H, |
| | 7,2 - 8,0 (m) 5 H ppm. |

Beispiel 16

N[1]-[4-[1,4-Dihydro-2,6-dimethyl-3-methoxycarbonyl-4-(3-nitrophenyl) -pyridin-5-carboxy]butyl]-N[2]-[3-(1H-imidazol-4-yl)propyl]-guanidin-hydrojodid

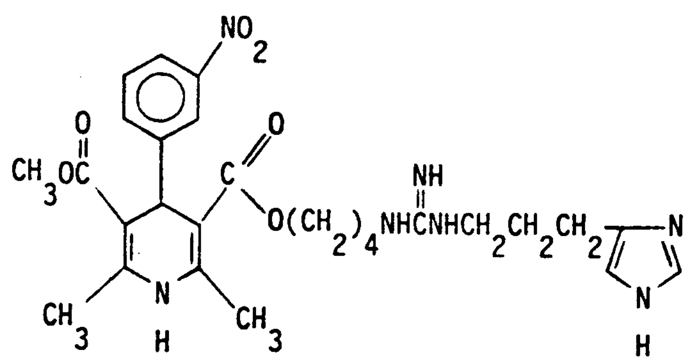

x HJ

a) 1,4-Dihydro-2,6-dimethyl-3-methoxycarbonyl-4-(3-nitrophenyl)-5-(4-phthalimido-butoxy)carbonyl-pyridin

Aus 8,25 g (33 mmol) 2-(3-Nitrobenzyliden)-acetessigsäuremethylester und 10,00 g (33 mmol) 3-Amino-crotonsäure-(4-phthalimido-butyl)ester werden analog zu Beispiel 15 a) nach 18-stündigem Kochen in 85 ml Ethanol und chromatographischer Reinigung des Rohprodukts an Kieselgel mit Dichlormethan/Methanol (95:5) als Laufmittel 6,14 g (35 %) eines gelben Öls erhalten.
$C_{28}H_{27}N_3O_8$ (533,54)
Rf ($CH_2Cl_2$/$CH_3OH$ 95:5): 0,67

b) 1,4-Dihydro-2,6-dimethyl-3-methoxycarbonyl-4-(3-nitrophenyl)-5-(4-aminobutoxy)carbonyl-pyridin

Erhalten analog zu Beispiel 1 b) aus 5,0 g (9.4 mmol) 1,4-Dihydro-2,6-dimethyl-3-methoxycarbonyl-4-(3-nitrophenyl)-5-(4-phthalimidobutoxy)carbonyl-pyridin und 1,4 ml (28 mmol) Hydrazinhydrat. 3,6 g (95 %) gelbes Öl, das ohne Reinigung weiter umgesetzt wird.

$C_{20}H_{25}N_3O_6$ (403,44)

Rf ($CH_2Cl_2/CH_3OH$ 80:20): 0,3

c) N[1]-Benzoyl-N[2]-[4-[1,4-dihydro-2,6-dimethyl-3-methoxycarbonyl-4-(3-nitrophenyl)-pyridin-5-carboxy]butyl]-thioharnstoff

Hergestellt analog zu Beispiel 1 c) aus 3,60 g (9 mmol) 1,4-Dihydro-2,6-dimethyl-3-methoxycarbonyl-4-(3-nitrophenyl)-5-(4-aminobutoxy) carbonyl-pyridin und 1,45 g (9 mmol) Benzoylisothiocyanat. Säulenchromatographie an Kieselgel mit Dichlormethan/Methanol (97:3) als Laufmittel ergibt 2,71 g (51 %) eines orangegelben, zähen Öls.

$C_{28}H_{30}N_4O_7S$ (566,44)

Rf ($CH_2Cl_2/CH_3OH$ 99:1): 0,58

d) S-Methyl-N-[4-[1,4-dihydro-2,6-dimethyl-3-methoxycarbonyl-4-(3-nitrophenyl)-pyridin-5-carboxy]butyl]-isothiuronium-jodid

Erhalten als hellgelber, amorpher Feststoff aus 2,0 g (3,5 mmol) N[1]-Benzoyl-N[2]-[4-[1,4-dihydro-2,6-dimethyl-3-methoxycarbonyl-4-(3-nitrophenyl)-pyridin-5-carboxy]butyl]-thioharnstoff entsprechend zu Beispiel 1 d). Ausbeute 1,8 g (84 %).

$C_{22}H_{29}JN_4O_6S$ (604,46)

Rf ($CH_2Cl_2/CH_3OH$ 90:10): 0,55

e) N[1]-[4-[1,4-Dihydro-2,6-dimethyl-3-methoxycarbonyl-4-(3-nitrophenyl)-pyridin-5-carboxy]butyl]-N[2]-[3-(1H-imidazol-4-yl)propyl]-guanidin-hydrojodid

1,00 g (1,65 mmol) S-Methyl-N-[4-[1,4-dihydro-2,6-dimethyl-3-methoxycarbonyl-4-(3-nitrophenyl)-pyridin-5-carboxy]butyl]-isothiuronium-jodid und 0,21 g (1,65 mmol) 3-(1H-Imidazol-4-yl)propylamin werden in 15 ml Acetonitril 3 Std. gekocht. Das Reaktionsgemisch wird analog zu Beispiel 1 e) aufgearbeitet und ergibt 0,18 g (16 %) eines hellgelben, amorphen Feststoffs.

$C_{27}H_{36}JN_7O_6$ (681,53)

Rf ($CH_3COOC_2H_5/CH_3OH/NH_4Cl/NH_3$-Puffer 70:28:2): 0,3

| | | | | |
|---|---|---|---|---|
| $^1$H-NMR-Daten: $\delta$ = | 1,4 - 2,1 | (m) | 6 H, | |
| ($CD_3OD$, TMS als | 2,3 | (s) | 3 H, | |
| interner Standard) | 2,35 | (s) | 3 H, | |
| | 2,7 | (t) | 2 H, | |
| | 3,1 - 3,4 | (m) | 4 H, | |
| | 3,6 | (s) | 3 H, | |
| | 3,9 - 4,2 | (m) | 2 H, | |
| | 4,9 | (breit) | 6 H, | austauschbar mit $D_2O$, |
| | 5,1 | (s) | 1 H, | |
| | 6,9 | (s) | 1 H, | |
| | 7,4 - 8,2 | (m) | 5 H ppm. | |

Beispiel 17

$N^1$-[5-[1,4-Dihydro-2,6-dimethyl-3-methoxycarbonyl-4-(2-nitrophenyl)-pyridin-5-carboxy]pentyl]-$N^2$-[3-(1H-imidazol-4-yl)propyl]-guanidin-hydrojodid

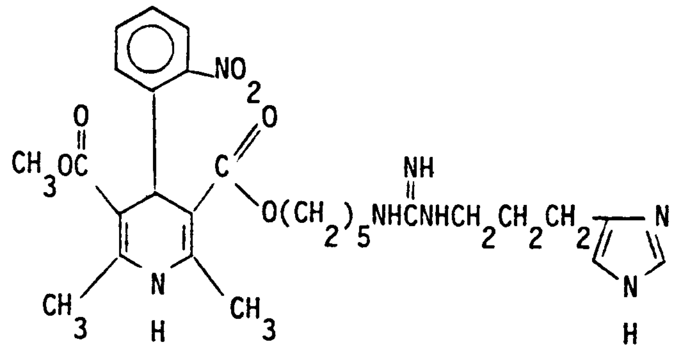

x HJ

Hergestellt analog zu Beispiel 1 e) aus 1,00 g (1,62 mmol) S-Methyl-N-[5-[1,4-dihydro-2,6-dimethyl-3-methoxycarbonyl-4-(2-nitrophenyl)-pyridin-5-carboxy]pentyl]-isothiuronium-jodid und 0,20 g (1,62 mmol) 3-(1H-Imidazol -4-yl)propylamin. 0,28 g (25 %) hellgelber, amorpher Feststoff.

$C_{28}H_{38}JN_7O_6$ (695,56)

Rf ($CH_3COOC_2H_5/CH_3OH/NH_4Cl/NH_3$-Puffer 50:47,5:2,5): 0,50

| | | | | |
|---|---|---|---|---|
| $^1$H-NMR-Daten: | $\delta$ = | 1,2 - 2,2 | (m) | 8 H, |
| ($CD_3OD$, TMS als | | 2,3 | (s) | 3 H, |
| interner Standard) | | 2,4 | (s) | 3 H, |
| | | 2,7 | (t) | 2 H, |
| | | 3,1 - 3,4 | (m) | 4 H, |
| | | 3,6 | (s) | 3 H, |
| | | 3,9 - 4,3 | (m) | 2 H, |
| | | 4,9 | (breit) | 6 H, austauschbar mit $D_2O$, |
| | | 5,8 | (s) | 1 H, |
| | | 7,0 | (s) | 1 H, |
| | | 7,3 - 7,9 | (m) | 5 H ppm. |

Beispiel 18

$N^1$-[5-[1,4-Dihydro-2,6-dimethyl-3-methoxycarbonyl-4-(3-nitrophenyl)-pyridin-5-carboxy]pentyl]-$N^2$-[3-(1H-imidazol-4-yl)propyl]-guanidin-hydrojodid

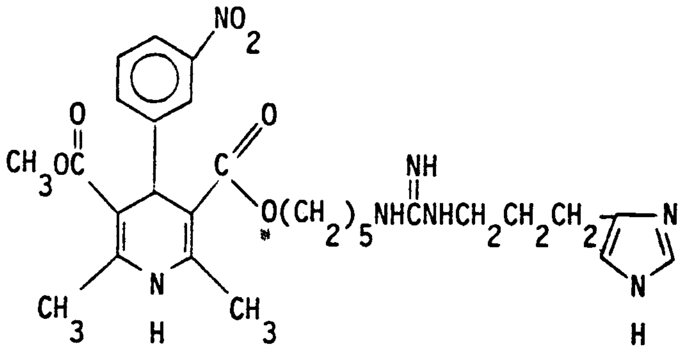

x HJ

Hergestellt analog zu Beispiel 1 e) aus 1,00 g (1,62 mmol) S-Methyl-N-[5-[1,4-dihydro-2,6-dimethyl-3-methoxycarbonyl-4-(3-nitrophenyl)-pyridin -5-carboxy]pentyl]-isothiuronium-jodid und 0,20 g (1,62 mmol) 3-(1H-Imidazol-4-yl)propylamin. 0,32 g (28 %) hellgelber, amorpher Feststoff.

$C_{28}H_{38}JN_7O_6$ (695,56)

Rf ($CH_3COOC_2H_5/CH_3OH/NH_4Cl/NH_3$-Puffer 50:47,5:2,5): 0,48

| | | | | |
|---|---|---|---|---|
| $^1$H-NMR-Daten: | δ = | 1,2 - 2,1 | (m) | 8 H, |
| ($CD_3OD$, TMS als | | 2,35 | (s) | 3 H, |
| interner Standard) | | 2,4 | (s) | 3 H, |
| | | 2,7 | (t) | 2 H, |
| | | 3,1 - 3,4 | (m) | 4 H, |
| | | 3,7 | (s) | 3 H, |
| | | 4,0 - 4,2 | (m) | 2 H, |
| | | 4,9 | (breit) | 6 H, austauschbar mit $D_2O$ |
| | | 5,15 | (s) | 1 H, |
| | | 6,95 | (s) | 1 H, |
| | | 7,4 - 8,2 | (m) | 5 H, ppm. |

Beispiel 19

N[1]-[6-[1,4-Dihydro-2,6-dimethyl-3-methoxycarbonyl-4-(2,3-dichlorphenyl)-pyridin-5-carboxy]hexyl]-N[2]-[3-(1H-imidazol-4-yl)propyl]-guanidin-hydrojodid

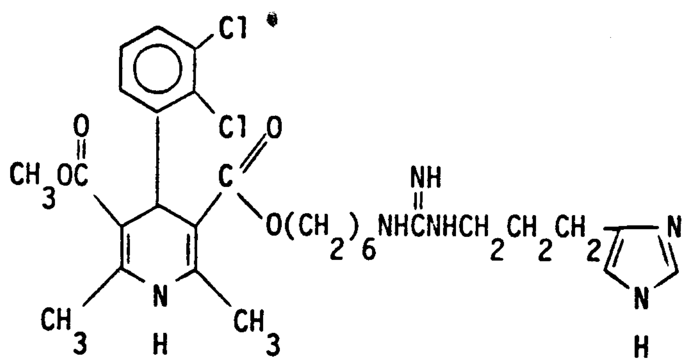

x HJ

a) 1,4-Dihydro-2,6-dimethyl-3-methoxycarbonyl-4-(2,3-dichlorphenyl)-5-(6-phthalimido-hexyloxy)carbonyl-pyridin

Aus 6,62 g (24 mmol) 2-(2,3-Dichlorbenzyliden)-acetessigsäure-methylester und 8,00 g (24 mmol) 3-Amino-crotonsäure-(6-phthalimidohexyl)ester erhält man analog zu Beispiel 1 a) 14,15 g (quant.) eines rötlichgelben Öls, das als Rohprodukt weiter umgesetzt wird.

$C_{30}H_{30}Cl_2N_2O_6$ (585,48)

Rf ($CH_2Cl_2/CH_3OH$ 98:2): 0,53

b) 1,4-Dihydro-2,6-dimethyl-3-methoxycarbonyl-4-(2,3-dichlorphenyl)-5-(6-aminohexyloxy)carbonyl-pyridin

Hergestellt analog zu Beispiel 1 b) aus 11,0 g (19 mmol) 1,4-Dihydro-2,6-dimethyl-3-methoxycarbonyl-4-(2,3-dichlorphenyl)-5-(6-phthalimido-hexyloxy)carbonyl-pyridin und 2,8 ml (56 mmol) Hydrazinhydrat. Das Rohprodukt wird ohne Reinigung weiter umgesetzt.

$C_{22}H_{28}Cl_2N_2O_4$ (455,38)

Rf ($CH_2Cl_2/CH_3OH$ 80:20): 0,17

c) $N^1$-Benzoyl-$N^2$-[6-[1,4-dihydro-2,6-dimethyl-3-methoxycarbonyl-4-(2,3-dichlorphenyl)-pyridin-5-carboxy]-hexyl]-thioharnstoff

Hergestellt analog zu Beispiel 1 c) aus 8,0 g (17,6 mmol) 1,4-Dihydro-2,6-dimethyl-3-methoxycarbonyl-4-(2,3-dichlorphenyl)-5-(6-aminohexyloxy)carbonyl-pyridin und 2,9 g (17,6 mmol) Benzoylisothiocyanat. 6,9 g (63 %) gelber, amorpher Feststoff nach Säulenchromatographie an Kieselgel mit Dichlormethan/Methanol (98:2) als Laufmittel.

$C_{30}H_{33}Cl_2N_3O_5S$ (618,58)

Rf ($CH_2Cl_2/CH_3OH$ 98:2): 0,40

d) S-Methyl-N-[6-[1,4-dihydro-2,6-dimethyl-3-methoxycarbonyl-4-(2,3-dichlorphenyl)-pyridin-5-carboxy]-hexyl]-isothiuronium-jodid

Hergestellt analog zu Beispiel 1 d) aus 5,0 g (8,1 mmol) $N^1$-Benzoyl-$N^2$-[6-[1,4-dihydro-2,6-dimethyl-3-methoxycarbonyl-4-(2,3-dichlorphenyl)-pyridin-5-carboxy]hexyl]-thioharnstoff. 3,7 g (86 %) gelber, amorpher Feststoff.

$C_{24}H_{32}Cl_2JN_3O_4S$ (656,41)

Rf ($CH_2Cl_2/CH_3OH$ 90:10): 0,33

e) $N^1$-[6-[1,4-Dihydro-2,6-dimethyl-3-methoxycarbonyl-4-(2,3-dichlorphenyl)-pyridin-5-carboxy]hexyl]-$N^2$-[3-(1H-imidazol-4-yl)propyl]-guanidin-hydrojodid

Hergestellt analog zu Beispiel 1 e) aus 1,50 g (2,8 mmol) S-Methyl-N-[6-[1,4-dihydro-2,6-dimethyl-3-methoxycarbonyl-4-(2,3-dichlorphenyl)-pyridin-5-carboxy]hexyl]-isothiuronium-jodid und 0,35 g (2,8 mmol) 3-(1H-Imidazol-4-yl)propylamin. 0.63 g (30 %) hellgelber, amorpher Feststoff.

$C_{29}H_{39}Cl_2JN_6O_4$ (733,48)
Rf ($CH_3COOC_2H_5/CH_3OH/NH_4Cl/NH_3$-Puffer 60:38:2): 0,42

| | | | |
|---|---|---|---|
| $^1$H-NMR-Daten: $\delta$ = | 1,1 - 1,8 | (m) | 8 H, |
| ($CD_3OD$, TMS als | 1,8 - 2,1 | (quin) | 3 H, |
| interner Standard) | 2,3 | (s) | 3 H, |
| | 2,35 | (s) | 3 H, |
| | 2,7 | (t) | 2 H, |
| | 3,1 - 3,4 | (m) | 4 H, |
| | 3,6 | (s) | 3 H, |
| | 3,9 - 4,2 | (m) | 2 H, |
| | 4,9 | (breit) | 6 H, austauschbar mit $D_2O$, |
| | 5,5 | (s) | 1 H, |
| | 6,95 | (s) | 1 H, |
| | 7,1 - 7,5 | (m) | 3 H, |
| | 7,75 | (s) | 1 H ppm. |

**Beispiel 20**

N[1]-[6-[1,4-Dihydro-2,6-dimethyl-3-isobutoxycarbonyl-4-(3-nitrophenyl)-pyridin-5-carboxy]hexyl]-N[2]-[3-(1H-imidazol-4-yl)propyl]-guanidin-hydrojodid

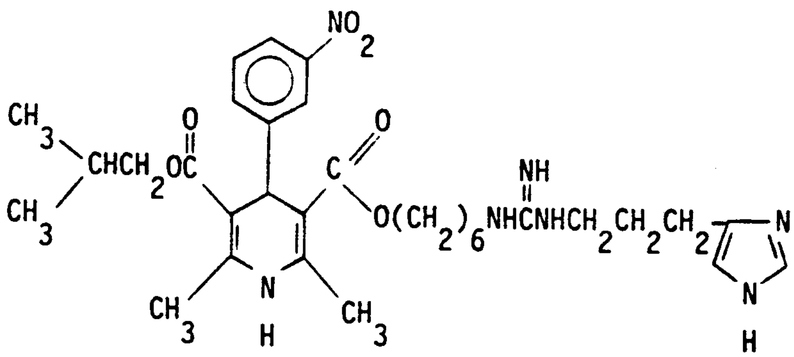

x HJ

a) 1,4-Dihydro-2,6-dimethyl-3-isobutoxycarbonyl-4-(3-nitrophenyl)-5-(6-phthalimido-hexyloxy)carbonyl-pyridin

Hergestellt analog zu Beispiel 1 a) aus 7.0 g (24 mmol) 2-(3-Nitrobenzyliden)-acetessigsäureisobutylester und 8,0 g (24 mmol) 3-Amino-crotonsäure-(6-phthalimidohexyl)ester. 14,6 g (quant.) orangegelbes Öl, das ohne Reinigung weiter umgesetzt wird.

$C_{33}H_{37}N_3O_8$ (603,67)
Rf ($CH_2Cl_2/CH_3OH$ 98:2): 0,34

b) 1,4-Dihydro-2,6-dimethyl-3-isobutoxycarbonyl-4-(3-nitrophenyl)-5-(6-aminohexyloxy)carbonyl-pyridin

Hergestellt analog zu Beispiel 1 b) aus 13,28 g (22 mmol) 1,4-Dihydro-2,6-dimethyl-3-isobutoxycarbonyl-4-(3-nitrophenyl)-5-(6-phthalimido-hexyloxy)carbonyl-pyridin und 3,3 ml (66 mmol) Hydrazinhydrat. 9,02 g (86 %) gelbes Öl.

$C_{25}H_{35}N_3O_6$ (473,57)

Rf ($CH_2Cl_2/CH_3OH$ 80:20): 0,16

c) N[1]-Benzoyl-N[2]-[6-[1,4-dihydro-2,6-dimethyl-3-isobutoxycarbonyl-4-(3-nitrophenyl)-pyridin-5-carboxy]-hexyl]-thioharnstoff

Hergestellt analog zu Beispiel 1 c) aus 9,0 g (19 mmol) 1,4-Dihydro-2,6-dimethyl-3-isobutoxycarbonyl-4-(3-nitrophenyl)-5-(6-aminohexyloxy) carbonyl-pyridin und 3,1 g (19 mmol) Benzoylisothiocyanat. Nach chromatographischer Reinigung an Kieselgel mit Dichlormethan/Methanol (98:2) als Laufmittel 5,7 g (47 %) gelber, amorpher Feststoff.

$C_{33}H_{40}N_4O_7S$ (636,77)

Rf ($CH_2Cl_2/CH_3OH$ 95:5): 0,76

d) S-Methyl-N-[6-[1,4-dihydro-2,6-dimethyl-3-isobutoxycarbonyl-4-(3-nitrophenyl)-pyridin-5-carboxy]hexyl]-isothiuronium-jodid

Hergestellt analog zu Beispiel 1 d) aus 3,0 g (4,7 mmol) N[1]-Benzoyl-N[2]-[6-[1,4-dihydro-2,6-dimethyl-3-isobutoxycarbonyl-4-(3-nitrophenyl)-pyridin-5-carboxy]hexyl]-thioharnstoff. 2,6 g (81 %) gelber, amorpher Feststoff.

$C_{27}H_{29}JN_4O_6S$ (674,60)

Rf ($CH_2Cl_2/CH_3OH$ 80:20): 0,66

e) N[1]-[6-[1,4-Dihydro-2,6-dimethyl-3-isobutoxycarbonyl-4-(3-nitrophenyl)-pyridin-5-carboxy]hexyl]-N[2]-[3-(1H-imidazol-4-yl)propyl]-guanidin-hydrojodid

Hergestellt analog zu Beispiel 1 e) aus 1,50 g (2,2 mmol) S-Methyl-N-[6-[1,4-dihydro-2,6-dimethyl-3-isobutoxycarbonyl-4-(3-nitrophenyl)-pyridin-5-carboxy]hexyl]-isothiuronium-jodid und 0,28 g (2,2 mmol) 3-(1H-Imidazol-4-yl)propylamin. 0,71 g (42 %) hellgelber, amorpher Feststoff.

$C_{32}H_{46}JN_7O_6$ (751,66)

Rf ($CH_3COOC_2H_5/CH_3OH/NH_4Cl/NH_3$-Puffer 50:47,5:2,5):0,64

| | | | |
|---|---|---|---|
| $^1$H-NMR-Daten: $\delta$ = | 0,8 - 1,0 | (2d) | 6 H, |
| ($CD_3OD$, TMS als | 1,2 - 2,1 | (m) | 3 H, |
| interner Standard) | 2,4 | (s) | 6 H, |
| | 2,7 | (t) | 2 H, |
| | 3,1 - 3,4 | (m) | 4 H, |
| | 3,85 | (d) | 2 H, |
| | 4,1 | (t) | 2 H, |
| | 4,8 | (breit) | 6 H, austauschbar mit $D_2O$, |
| | 5,2 | (s) | 1 H, |
| | 6,95 | (s) | 1 H, |
| | 7,4 - 8,3 | (m) | 5 H ppm. |

Beispiel 21

$N^1$-[8-[1,4-Dihydro-2,6-dimethyl-3-methoxycarbonyl-4-(3-nitrophenyl)-pyridin-5-carboxy]octyl]-$N^2$-[3-(1H-imidazol-4-yl)propyl]-guanidin-hydrojodid

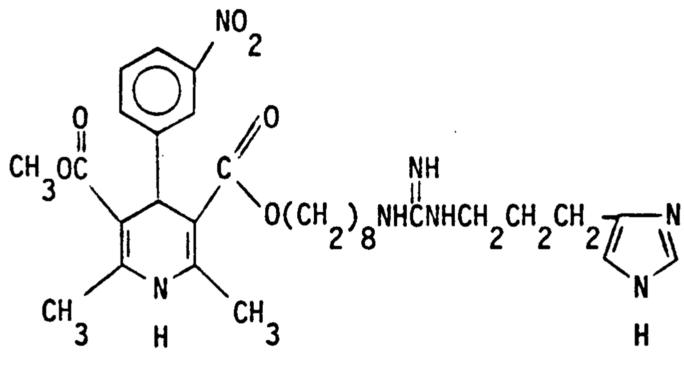

x HJ

a) 1,4-Dihydro-2,6-dimethyl-3-methoxycarbonyl-4-(3-nitrophenyl)-5-(8-phthalimido-octyloxy)carbonyl-pyridin

6,68 g (13,6 mmol) 2-(3-Nitrobenzyliden)-acetessigsäure-(8-phthalimidooctyl)ester und 1,57 g (13,6 mmol) 3-Aminocrotonsäremethylester werden in 70 ml Isopropanol 5 Std. unter Rückfluß gekocht. Das nach Abdampfen des Lösungsmittels i. Vak. erhaltene Öl wird an Kieselgel mit Dichlormethan/Methanol (97:3) als Laufmittel chromatographiert. Man erhält 6,75 g (85 %) eines gelben Öls.

$C_{32}H_{35}N_3O_8$ (589,64)

Rf ($CH_2Cl_2/CH_3OH$ 95:5): 0,5

b) 1,4-Dihydro-2,6-dimethyl-3-methoxycarbonyl-4-(3-nitrophenyl)-5-(8-aminooctyloxy)carbonyl-pyridin

Hergestellt analog zu Beispiel 1 b) aus 6,75 g (11,5 mmol) 1,4-Dihydro-2,6-dimethyl-3-methoxycarbonyl-4-(3-nitrophenyl)-5-(8-phthalimido-octyloxy)carbonyl-pyridin und 1,7 ml (34,5 mmol) Hydrazinhydrat. Mit tert.-Butyl-methylether 4,13 g (78 %) gelbe Kristalle vom Schmp. 129 - 131 ° C.

$C_{24}H_{33}N_3O_6$ (459,54)

Rf ($CH_2Cl_2/CH_3OH$ 70:30): 0,15

c) $N^1$-Benzoyl-$N^2$-[8-[1,4-dihydro-2,6-dimethyl-3-methoxycarbonyl-4-(3-nitrophenyl)-pyridin-5-carboxy]octyl]-thioharnstoff

Hergestellt analog zu Beispiel 1 c) aus 3,1 g (6,8 mmol) 1,4-Dihydro-2,6-dimethyl-3-methoxycarbonyl-4-(3-nitrophenyl)-5-(8-aminooctyloxy) carbonyl-pyridin und 1,1 g (6,8 mmol) Benzoylisothiocyanat. Chromatographische Reinigung an Kieselgel mit Dichlormethan/Methanol (99:1) als Laufmittel ergibt 3,7 g (87 %) eines gelben Öls.

$C_{32}H_{38}N_4O_7S$ (622,74)

Rf ($CH_2Cl_2/CH_3OH$ 98:2): 0,3

d) S-Methyl-N-[8-[1,4-dihydro-2,6-dimethyl-3-methoxycarbonyl-4-(3-nitrophenyl)-pyridin-5-carboxy]octyl]-isothiuronium-jodid

Hergestellt analog zu Beispiel 1 d) aus 3,7 g (5,9 mmol) $N^1$-Benzoyl-$N^2$-[8-[1,4-dihydro-2,6-dimethyl-3-methoxycarbonyl -4-(3-nitrophenyl)-pyridin-5-carboxy]octyl]thioharnstoff. 3,5 g (90 %) gelber, amorpher Feststoff.

$C_{26}H_{37}JN_4O_6S$ (660,53)

Rf ($CH_2Cl_2/CH_3OH$ 9:1): 0,4

e) N[1]-[8-[1,4-Dihydro-2,6-dimethyl-3-methoxycarbonyl-4-(3-nitrophenyl)-pyridin-5-carboxy]octyl]-N[2]-[3-(1H-imidazol-4-yl)propyl]-guanidin-hydrojodid

Hergestellt analog zu Beispiel 1 e) aus 1,20 g (1,82 mmol) S-Methyl-N-[8-[1,4-dihydro-2,6-dimethyl-3-methoxycarbonyl-4-(3-nitrophenyl)-pyridin-5-carboxy]octyl]-isothiuronium-jodid und 0,23 g (1,82 mmol) 3-(1H-Imidazol-4-yl)propylamin. 0,45 g (33 %) gelber, amorpher Feststoff.

$C_{31}H_{44}JN_7O_6$ (737,64)

Rf ($CH_3COOC_2H_5/CH_3OH/NH_4Cl/NH_3$-Puffer 50:47,5:2,5): 0,6

| | | | |
|---|---|---|---|
| $^1$H-NMR-Daten: (CD$_3$OD, TMS als interner Standard) | $\delta$ = 1,2 - 2,1 | (m) | 14 H, |
| | 2,35 | (s) | 3 H, |
| | 2,4 | (s) | 3 H, |
| | 2,7 | (t) | 2 H, |
| | 3,1 - 3,4 | (m) | 4 H, |
| | 3,7 | (s) | 3 H, |
| | 3,9 - 4,3 | (m) | 2 H, |
| | 4,9 | (breit) | 6 H, austauschbar mit $D_2O$, |
| | 5,15 | (s) | 1 H, |
| | 6,95 | (s) | 1 H, |
| | 7,4 - 8,2 | (m) | 5 H ppm. |

Beispiel 22

N[1]-[11-[1,4-Dihydro-2,6-dimethyl-3-methoxycarbonyl-4-(3-nitrophenyl)-pyridin-5-carboxy]undecyl]-N[2]-[3-(1H-imidazol-4-yl)propyl]-guanidin-hydrojodid

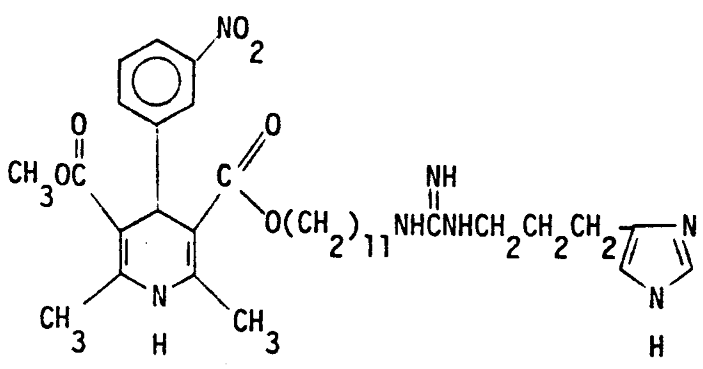

x HJ

a) 1,4-Dihydro-2,6-dimethyl-3-methoxycarbonyl-4-(3-nitrophenyl)-5-(11-phthalimido-undecyloxy)carbonyl-pyridin

Hergestellt analog zu Beispiel 1 a) aus 9,33 g (37,5 mmol) 2-(3-Nitrobenzyliden)-acetessigsäuremethyl-

ester und 15,00 g (37,5 mmol) 3-Amino-crotonsäure-(11-phthalimido-undecyl)ester. Aus Methanol 23,18 g (98 %) gelbe Kristalle vom Schmp. 130 - 132 °C.

$C_{35}H_{41}N_3O_8$ (631,73)

Rf ($CH_2Cl_2/CH_3OH$ 95:5): 0,78

b) 1,4-Dihydro-2,6-dimethyl-3-methoxycarbonyl-4-(3-nitrophenyl)-5-(11-aminoundecyloxy)carbonyl-pyridin

Hergestellt analog zu Beispiel 1 b) aus 18,95 g (30 mmol) 1,4-Dihydro-2,6-dimethyl-3-methoxycarbonyl-4-(3-nitrophenyl)-5-(11-phthalimido-undecyloxy)carbonyl-pyridin und 4,5 ml (90 mmol) Hydrazinhydrat. 15,0 g gelbes Öl, das ohne Reinigung weiter umgesetzt wird.

$C_{27}H_{39}N_3O_6$ (501,62)

Rf ($CH_3OH/NH_3$ konz. 95:5): 0,59

c) $N^1$-Benzoyl-$N^2$-[11-[1,4-dihydro-2,6-dimethyl-3-methoxycarbonyl-4-(3-nitrophenyl)-pyridin-5-carboxy]-undecyl]-thioharnstoff.

Hergestellt analog zu Beispiel 1 c) aus 10,03 g (20 mmol) 1,4-Dihydro-2,6-dimethyl-3-methoxycarbonyl-4-(3-nitrophenyl)-5-(11-aminoundecyloxy)carbonyl-pyridin und 3,26 g (20 mmol) Benzoylisothiocyanat. Nach chromatographischer Reinigung an Kieselgel mit Dichlormethan/Methanol (98:2) als Laufmittel 5,20 g (39 %) zähes, gelbes Öl.

$C_{35}H_{44}N_4O_7S$ (664,82)

Rf ($CH_2Cl_2/CH_3OH$ 98:2): 0,55

d) S-Methyl-N-[11-[1,4-dihydro-2,6-dimethyl-3-methoxycarbonyl-4-(3-nitrophenyl)-pyridin-5-carboxy]-undecyl]-isothiuronium-jodid

Hergestellt analog zu Beispiel 1 d) aus 4,0 g ( 6 mmol) $N^1$-Benzoyl -$N^2$-[11-[1,4-dihydro-2,6-dimethyl-3-methoxycarbonyl-4-(3-nitrophenyl)-pyridin-5-carboxy]undecyl]-thioharnstoff. 3,8 g (90 %) gelber, amorpher Feststoff.

$C_{29}H_{43}JN_4O_6S$ (702,65)

Rf ($CH_2Cl_2/CH_3OH$ 9:1): 0,43

e) $N^1$-[11-[1,4-Dihydro-2,6-dimethyl-3-methoxycarbonyl-4-(3-nitrophenyl)-pyridin-5-carboxy]undecyl]-$N^2$-[3-(1H-imidazol-4-yl)propyl]-guanidin-hydrojodid

Hergestellt analog zu Beispiel 1 e) aus 1,50 g (2,13 mmol) S-Methyl-N-[11-[1,4-dihydro-2,6-dimethyl-3-methoxycarbonyl-4-(3-nitrophenyl)-pyridin-5-carboxy]undecyl]-isothiuronium-jodid und 0,27 g (2,13 mmol) 3-(1H-Imidazol-4-yl)propylamin. 0,55 g (33 %) gelber, amorpher Feststoff.

$C_{34}H_{50}JN_7O_6$ (779,72)

Rf ($CH_3COOC_2H_5/CH_3OH/NH_4Cl/NH_3$-Puffer 60:38:2): 0,33

| | | | |
|---|---|---|---|
| $^{1}$H-NMR-Daten: | δ = | 1,2 - 2,1 | (m) 20 H, |
| ($CD_3OD$, TMS als | | 2,35 | (s) 3 H, |
| interner Standard) | | 2,4 | (s) 3 H, |
| | | 2,7 | (t) 2 H, |
| | | 3,1 - 3,4 | (m) 4 H, |
| | | 3,7 | (s) 3 H, |
| | | 3,9 - 4,2 | (m) 2 H, |
| | | 4,9 | (breit) 6 H, austauschbar mit $D_2O$, |
| | | 5,15 | (s) 1 H, |
| | | 6,95 | (s) 1 H, |
| | | 7,4 - 8,3 | (m) 5 H ppm. |

Beispiel 23

$N^1$-[2-[1,4-Dihydro-2,6-dimethyl-3-methoxycarbonyl-4-(benzofurazan-4-yl)-pyridin-5-carboxy]ethyl]-$N^2$-[3-(1H-imidazol-4-yl)propyl]-guanidin-hydrojodid

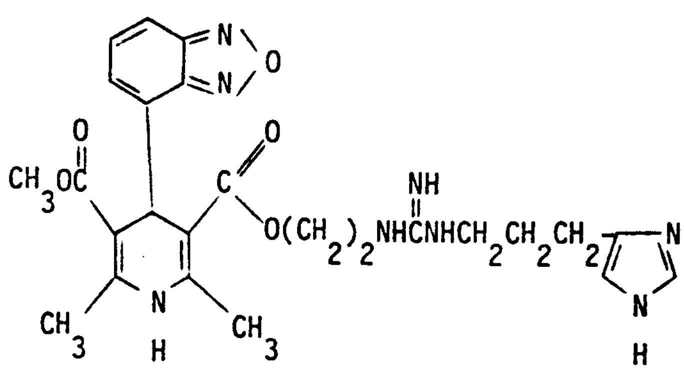

x HJ

a) 1,4-Dihydro-2,6-dimethyl-3-methoxycarbonyl-4-(benzofurazan-4-yl)-5-(2-phthalimido-ethoxy)carbonyl-pyridin

6,3 g (15,5 mmol) 2-(Benzofurazan-4-yl)-acetessigsäure-(2-phthalimido-ethyl)ester und 1,8 g (15,5 mmol) 3-Aminocrotonsäuremethylester werden in 50 ml Isopropanol 2 Std. gekocht. Der nach Abkühlen auf Raumtemperatur ausgefallene Niederschlag wird abgesaugt und aus Ethanol umkristallisiert. 5,0 g (64 %) gelbe Kristalle vom Schmp. 208 - 209° C.

$C_{26}H_{22}N_4O_7$ (502,48)

Rf ($CH_2Cl_2/CH_3OH$ 95:5): 0,7

b) 1,4-Dihydro-2,6-dimethyl-3-methoxycarbonyl-4-(benzofurazan-4-yl)-5-(2-aminoethoxy)carbonyl-pyridin

Hergestellt analog zu Beispiel 1 b) aus 5,0 g (10 mmol) 1,4-Dihydro-2,6-dimethyl-3-methoxycarbonyl-4-(benzofurazan-4-yl)-5-(2-phthalimido-ethoxy)carbonyl-pyridin und 1,5 ml (30 mmol) Hydrazinhydrat.

3,7 g (quant.) orangegelbes Öl, das ohne Reinigung weiter umgesetzt wird.

$C_{18}H_{20}N_4O_5$ (372,38)

Rf ($CH_2Cl_2/CH_3OH$ 80:20): 0,35

c) N[1]-Benzoyl-N[2]-[2-[1,4-dihydro-2,6-dimethyl-3-methoxycarbonyl-4-(benzofurazan-4-yl)-pyridin-5-carboxy]-ethyl]-thioharnstoff

Hergestellt analog zu Beispiel 1 c) aus 2,9 g (7,8 mmol) 1,4-Dihydro-2,6-dimethyl-3-methoxycarbonyl-4-(benzofurazan-4-yl)-5-(2-aminoethoxy) carbonyl-pyridin und 1,3 g (7,8 mmol) Benzoylisothiocyanat. 2,4 g (58 %) gelber amorpher Feststoff nach Chromatographie an Kieselgel mit Dichlormethan/Methanol (99:1) als Laufmittel.

$C_{26}H_{25}N_5O_6S$ (535,58)

Rf ($CH_2Cl_2/CH_3OH$ 99:1): 0,4

d) S-Methyl-N-[2-[1,4-dihydro-2,6-dimethyl-3-methoxycarbonyl-4-(benzofurazan-4-yl)-pyridin-5-carboxy]-ethyl]-isothiuronium-jodid

Hergestellt analog zu Beispiel 1 d) aus 2,24 g (4,2 mmol) N[1]-Benzoyl-N[2]-[2-[1,4-dihydro-2,6-dimethyl-3-methoxycarbonyl-4-(benzofurazan-4-yl)-pyridin-5-carboxy]ethyl]-thioharnstoff. 2,20 g (91 %) gelber amorpher Feststoff.

$C_{20}H_{24}JN_5O_5S$ (573,41)

Rf ($CH_2Cl_2/CH_3OH$ 9:1): 0,4

e) N[1]-[2-[1,4-Dihydro-2,6-dimethyl-3-methoxycarbonyl-4-(benzofurazan-4-yl)-pyridin-5-carboxy]ethyl]-N[2]-[3-(1H-imidazol-4-yl)propyl]-guanidin-hydrojodid

Hergestellt analog zu Beispiel 1 e) aus 1,2 g (2,1 mmol) S-Methyl-N-[2-[1,4-dihydro-2,6-dimethyl-3-methoxycarbonyl-4-(benzofurazan-4-yl)-pyridin-5-carboxy]ethyl]-isothiuronium-jodid und 0,26 g (2,1 mmol) 3-(1H-Imidazol-4-yl)propylamin. 0,35 g (26 %) hellgelber, amorpher Feststoff.

$C_{25}H_{31}JN_8O_5$ (650,48)

Rf ($CH_3COOC_2H_5/CH_3OH/NH_4Cl/NH_3$-Puffer 50:47,5:2,5): 0,65

| | | | | |
|---|---|---|---|---|
| $^1$H-NMR-Daten: | $\delta$ = | 1,8 - 2,2 | (m) | 2 H, |
| ($CD_3OD$, TMS als | | 2,4 | (s) | 6 H, |
| interner Standard) | | 2,7 | (t) | 2 H, |
| | | 3,2 - 3,7 | (m) | 4 H, |
| | | 3,65 | (s) | 3 H, |
| | | 4,0 - 4,5 | (m) | 2 H, |
| | | 4,9 | (breit) | 6 H, austauschbar mit $D_2O$, |
| | | 5,65 | (s) | 1 H, |
| | | 6,95 | (s) | 1 H, |
| | | 7,3 - 7,9 | (m) | 4 H, ppm. |

Beispiel 24

8-[1,4-Dihydro-2,6-dimethyl-3-methoxycarbonyl-4-(3-nitrophenyl)-pyridin-5-carboxy]-N-[3-(1H-imidazol-4-yl)-propyl]-caprylamidin-hydrochlorid

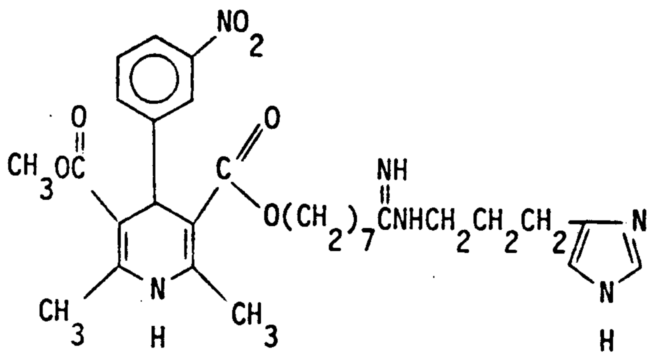

x HCl

a) 1,4-Dihydro-2,6-dimethyl-3-methoxycarbonyl-4-(3-nitrophenyl)-5-(7-cyanheptyloxy)carbonyl-pyridin

6,7 g (26,8 mmol) 2-(3-Nitrobenzyliden)-acetessigsäuremethylester und 6,0 g (26,8 mmol) 3-Aminocrotonsäure-(7-cyanheptyl)ester werden in 50 ml Isopropanol 12 Std. gekocht. Der ausgefallene Niederschlag wird abgesaugt und an Kieselgel mit Dichlormethan/Methanol (98:2) chromatographiert. die Hauptfraktion ergibt nach Eindampfen i. Vak, und Umkristallisieren des Rückstands aus tert.-Butyl-methylether 7,9 g (65%) gelbe Kristalle vom Schmp. 151 - 152° C.

$C_{24}H_{29}N_3O_6$ (455,51)

Rf ($CH_2Cl_2$/$CH_3OH$ 98:2): 0,4

b) 8-[1,4-Dihydro-2,6-dimethyl-3-methoxycarbonyl-4-(3-nitrophenyl)-pyridin-5-carboxy]-caprylimidsäuremethylester-dihydrochlorid

Hergestellt analog zu Beispiel 12 b) aus 2,0 g (4,4 mmol) 1,4-Dihydro-2,6-dimethyl-3-methoxycarbonyl-4-(3-nitrophenyl)-5-(7-cyanheptyloxy) carbonyl-pyridin. 2,5 g gelber, hygroskopischer Schaum.

$C_{25}H_{35}Cl_2N_3O_7$ (560,47)

Rf ($CH_2Cl_2$/$CH_3OH$/$N(C_2H_5)_3$ 90:10:1): 0,6

c) 8-[1,4-Dihydro-2,6-dimethyl-3-methoxycarbonyl-4-(3-nitrophenyl)-pyridin-5-carboxy]-N-[3-(1H-imidazol-4-yl)propyl]-caprylamidin-hydrochlorid

Hergestellt analog zu Beispiel 12 c) aus 1,50 g (2,7 mmol) 8-[ 1,4-Dihydro-2,6-dimethyl-3-methoxycarbonyl-4-(3-nitrophenyl)-pyridin-5-carboxy]-caprylimidsäure-methylester-hydrochlorid und 0,38 g (3,0 mmol) 3-(1H-Imidazol-4-yl)propylamin. 0,63 g (38 %) hellgelber, amorpher Feststoff.

$C_{30}H_{41}ClN_6O_6$ (617,15)

Rf ($CH_3COOC_2H_5$/$CH_3OH$/$NH_4Cl$/$NH_3$-Puffer 50:47,5:2,5): 0,65

| | | | | |
|---|---|---|---|---|
| $^1$H-NMR-Daten: | δ = | 1,1 - 2,2 | (m) | 12 H, |
| ($CD_3OD$, TMS als | | 2,35 | (s) | 3 H, |
| interner Standard) | | 2,4 | (s) | 3 H, |
| | | 2,4 - 2,9 | (m) | 4 H, |
| | | 3,2 - 3,5 | (t) | 2 H, |
| | | 3,7 | (s) | 3 H, |
| | | 3,9 - 4,2 | (m) | 2 H, |
| | | 5,0 | (breit) | 5 H, austauschbar mit $D_2O$, |
| | | 5,15 | (s) | 1 H, |
| | | 6,95 | (s) | 1 H, |
| | | 7,4 - 8,3 | (m) | 5 H, ppm. |

**Patentansprüche**
**Patentansprüche für folgende Vertragsstaaten : BE, CH, DE, FR, GB, IT, LI, LU, NL, SE**

**1.** 1,4-Dihydropyridinderivate der allgemeinen Formel I

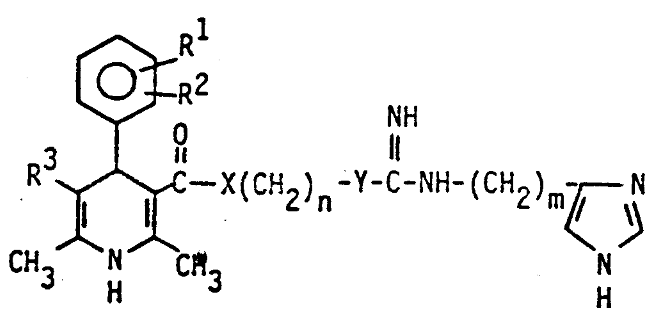

(I)

in der $R^1$ und $R^2$ unabhängig voneinander für ein Wasserstoffatom, eine gegebenenfalls durch Halogen substituierte Methylgruppe, ein Halogenatom, eine Nitrogruppe oder eine Trifluormethylgruppe stehen oder zusammen für einen 1,2,5-Oxadiazolrest stehen,
$R^3$ eine Nitrogruppe oder den Rest

$$-\overset{O}{\overset{\|}{C}}-OR^4$$

bedeutet, worin $R^4$ eine gerad-oder verzweigtkettige, gegebenenfalls durch eine $C_1$-$C_4$-Alkoxygruppe substituierte $C_1$-$C_4$-Alkylgruppe ist,
X für ein Sauerstoffatom oder die Gruppe NH steht,
Y für die Gruppe NH oder eine Bindung steht,
n eine ganze Zahl von 1 bis 16 bedeutet und
m den Wert 2 oder 3 hat,
sowie die physiologisch annehmbaren Salze davon.

**2.** 1,4-Dihydropyridinderivate nach Anspruch 1, dadurch **gekennzeichnet,** daß $R^1$ und $R^2$ unabhängig voneinander für ein Wasserstoffatom, eine Methylgruppe, die gegebenenfalls durch ein Halogenatom

substituiert ist, ein Halogenatom, eine Nitrogruppe oder eine Trifluormethylgruppe stehen, wobei die Substituenten $R^1$ und $R^2$ in 2- und/oder 3-Stellung des Phenylrings gebunden sind, oder $R^1$ und $R^2$ zusammen für einen 1,2,5-Oxadiazolrest stehen, der in Positionen 3 und 4 an den Phenylrest in den Positionen 2 und 3 ankondensiert ist,
$R^3$ für eine Nitrogruppe oder den Rest

$$-\overset{\overset{O}{\|}}{C}-OR^4$$

steht, worin $R^4$ eine gerad- oder verzweigtkettige $C_1$-$C_4$-Alkylgruppe bedeutet, die gegebenenfalls durch eine $C_1$-$C_4$-Alkoxygruppe substituiert ist,
X für ein Sauerstoffatom oder die Gruppe NH steht,
Y für die Gruppe NH oder eine Bindung steht,
n eine ganze Zahl von 1 bis 16 ist und
m den Wert 3 hat,
sowie die physiologisch annehmbaren Salze davon.

**3.** 1,4-Dihydropyridinderivate nach Anspruch 1, dadurch **gekennzeichnet,** daß $R^1$ und $R^2$ unabhängig voneinander für ein Wasserstoffatom, ein Chloratom oder eine Nitrogruppe stehen, wobei die Substituenten $R^1$ und $R^2$ in 2- und/oder 3-Stellung des Phenylrings gebunden sind,
$R^3$ den Rest

$$-\overset{\overset{O}{\|}}{C}-OR^4$$

darstellt, worin $R^4$ für eine gerad- oder verzweigtkettige $C_1$-$C_4$-Alkylgruppe, die gegebenenfalls durch eine $C_1$-$C_4$-Alkoxygruppe substituiert ist, steht,
X für ein Sauerstoffatom oder die Gruppe NH steht,
Y für die Gruppe NH oder eine Bindung steht,
n eine ganze Zahl von 2 bis 6 bedeutet und
m den Wert 3 hat,
sowie die physiologisch annehmbaren Salze davon.

**4.** 1,4-Dihydropyridinderivate nach Anspruch 1, dadurch **gekennzeichnet,** daß $R^1$ für ein Wasserstoffatom steht, $R^2$ für eine in 2- oder 3-Position des Phenylrings gebundene Nitrogruppe steht,
$R^3$ für den Rest

$$-\overset{\overset{O}{\|}}{C}-OR^4$$

steht, worin $R^4$ eine gerad- oder verzweigtkettige $C_1$-$C_4$-Alkylgruppe ist, die gegebenenfalls durch eine $C_1$-$C_4$-Alkoxygruppe substituiert ist,
X für ein Sauerstoffatom steht,
Y für die Gruppe NH steht,
n eine ganze Zahl von 1 bis 16 bedeutet und
m den Wert 3 hat,
sowie die physiologisch annehmbaren Salze davon.

**5.** Verfahren zur Herstellung der 1,4-Dihydropyridinderivate nach den Ansprüche 1 bis 4 sowie der physiologisch annehmbaren Salze davon, dadurch **gekennzeichnet,** daß man
(a) zur Herstellung von Verbindungen der allgemeinen Formel I, bei denen $R^1$, $R^2$, $R^3$, X, m und n wie in Anspruch 1 definiert sind und Y für NH steht

a1) ein Isothiuroniumsalz der allgemeinen Formel II

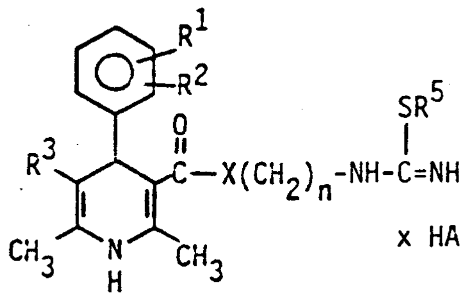

(II)

in der $R^1$, $R^2$, R, X und n wie in Anspruch 1 definiert sind, $R^5$ für einen gegebenenfalls substituierten Alkylrest und A für ein Halogenatom oder die Gruppe $-OSO_2OR^5$ stehen, mit einem m-(1H-Imidazol-4-yl)-alkylamin der Formel III

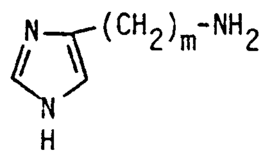

(III)

worin m den Wert 2 oder 3 hat, umsetzt oder
daß man
a2) ein Amin der allgemeinen Formel IV

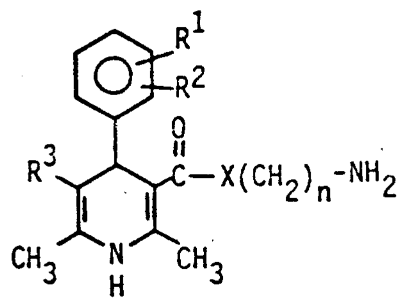

(IV)

worin $R^1$, $R^2$, $R^3$, X und n wie in Anspruch 1 definiert sind, mit einer Verbindung der allgemeinen Formel V

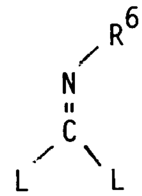

(V)

in der $R^6$ für eine Cyangruppe oder einen Benzoylrest steht und L eine Abgangsgruppe, wie $-SR^7$ oder $-OR^7$, bedeutet, wobei $R^7$ einen Alkyl- oder Arylrest darstellt, zu einer Zwischenverbindung der allgemeinen Formel VI

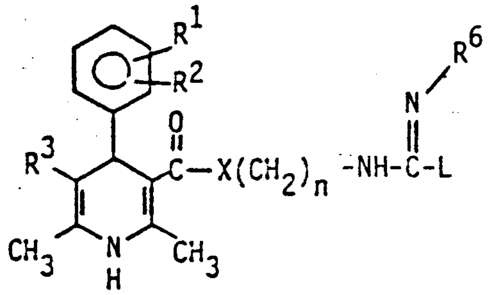

(VI)

worin $R^1$, $R^2$, $R^3$, $R^6$, X, L und n wie oben definiert sind, umsetzt, die erhaltene Zwischenverbindung der Formel VI mit einem m-(1H-Imidazol-4-yl)-alkylamin der Formel III

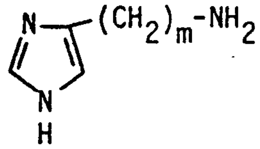

(III)

worin m den Wert 2 oder 3 hat, zu einer Verbindung der allgemeinen Formel VII

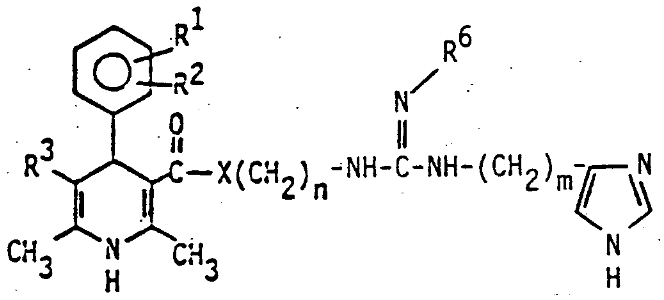

(VII)

worin $R^1$, $R^2$, $R^3$, $R^6$, X, m und n wie oben definiert sind, umsetzt und die Verbindung der allgemeinen Formel VII durch anschließende säurekatalysierte Hydrolyse zu einer Verbindung der allgemeinen Formel I umsetzt oder
daß man
a3) ein Amin der allgemeinen Formel IV

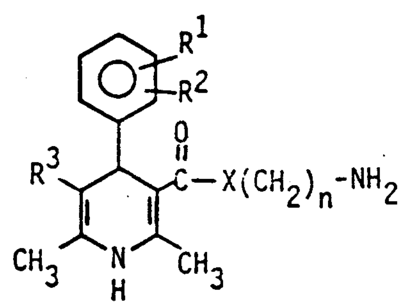

(IV)

worin $R^1$, $R^2$, $R^3$, X und n wie in Anspruch 1 definiert sind, mit einer Verbindung der allgemeinen Formel VIII

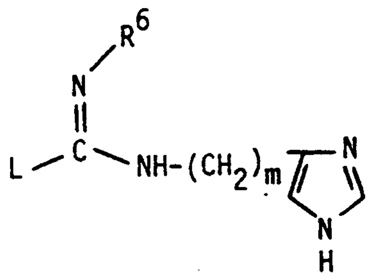

(VIII)

worin $R^6$ und L die oben angegebenen Bedeutungen haben und m den Wert 2 oder 3 hat, zu einer Verbindung der allgemeinen Formel VII

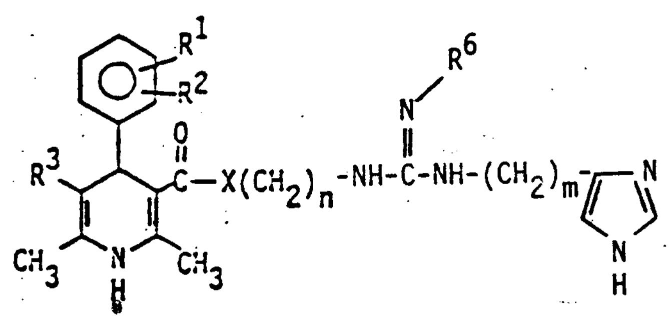

(VII)

worin $R^1$, $R^2$, $R^3$, $R^6$, X, m und n wie oben definiert sind, umsetzt und die erhaltene Verbindung der allgemeinen Formel VII zu einer Verbindung der allgemeinen Formel I hydrolysiert oder
daß man
a4) ein Amin der allgemeinen Formel IV, worin $R^1$, $R^2$, $R^3$, X und n wie in Anspruch 1 definiert sind, mit einer Verbindung der allgemeinen Formel IX

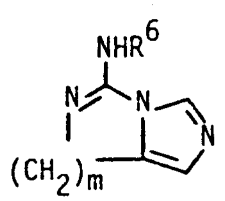

(IX)

worin $R^6$ und m wie oben definiert sind, zu einer Zwischenverbindung der allgemeinen Formel VII

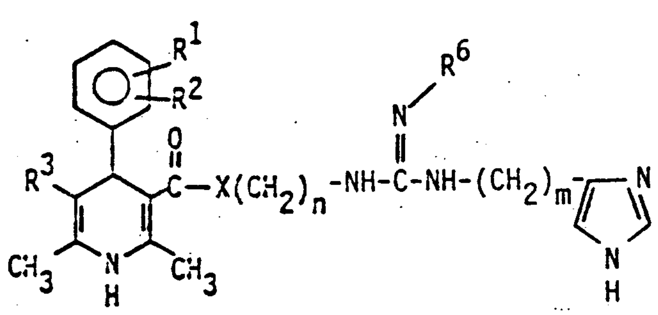

(VII)

worin $R^1$, $R^2$, $R^3$, $R^6$, X, m und n wie oben definiert sind, umsetzt und die so erhaltene Zwischenverbindung der Formel VII zu einer Verbindung der allgemeinen Formel I hydrolysiert oder
daß man

(b) zur Herstellung von Verbindungen der allgemeinen Formel I, bei denen $R^1$, $R^2$, $R^3$, X, m und n wie in Anspruch 1 definiert sind und Y eine Bindung bedeutet, einen Imidoester der allgemeinen Formel X

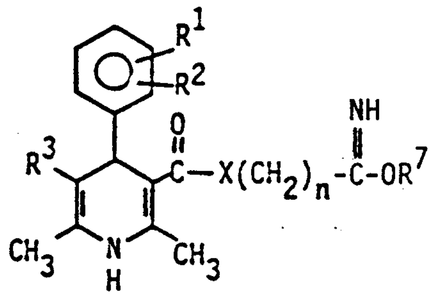

(X)

worin $R^1$, $R^2$, $R^3$, X und n wie in Anspruch 1 definiert sind und $R^7$ für einen $C_1$-$C_4$-Alkylrest steht, mit einem m-(1H-Imidazol-4-yl)-alkylamin der Formel III

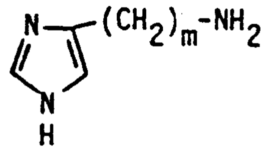

(III)

worin m den Wert 2 oder 3 hat, umsetzt
und gegebenenfalls die gemäß (a) oder (b) erhaltene Verbindung der allgemeinen Formel I in ihr physiologisch annehmbares Salz umwandelt.

6. Arzneimittel, dadurch **gekennzeichnet,** daß es eine Verbindung nach einem der Ansprüche 1 bis 4 zusammen mit einem inerten, pharmazeutisch annehmbaren Träger oder Verdünnungsmittel enthält.

**Patentansprüche für folgende Vertragsstaaten : AT, ES, GR**

1. Verfahren zur Herstellung von 1,4-Dihydropyridinderivaten der allgemeinen Formel I

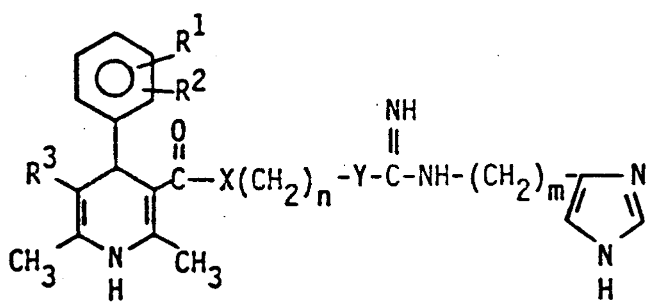

(I)

in der $R^1$ und $R^2$ unabhängig voneinander für ein Wasserstoffatom, eine gegebenenfalls durch Halogen substituierte Methylgruppe, ein Halogenatom, eine Nitrogruppe oder eine Trifluormethylgruppe stehen oder zusammen für einen 1,2,5-Oxadiazolrest stehen,
$R^3$ eine Nitrogruppe oder den Rest

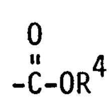

bedeutet, worin $R^4$ eine gerad-oder verzweigtkettige, gegebenenfalls durch eine $C_1$-$C_4$-Alkoxygruppe

substituierte $C_1$-$C_4$-Alkylgruppe ist,
X für ein Sauerstoffatom oder die Gruppe NH steht,
Y für die Gruppe NH oder eine Bindung steht,
n eine ganze Zahl von 1 bis 16 bedeutet und
m den Wert 2 oder 3 hat,
sowie der physiologisch annehmbaren Salze davon, dadurch **gekennzeichnet,** daß man

(a) zur Herstellung von Verbindungen der allgemeinen Formel I, bei denen $R^1$, $R^2$, $R^3$, X, m und n die oben angegebenen Bedeutungen haben und Y für NH steht

a1) ein Isothiuroniumsalz der allgemeinen Formel II

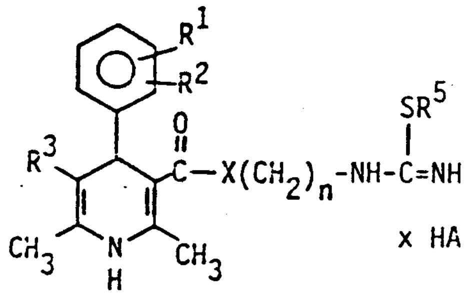

(II)

in der $R^1$, $R^2$, $R^3$, X und n die oben angegebenen Bedeutungen haben, $R^5$ für einen gegebenenfalls substituierten Alkylrest und A für ein Halogenatom oder die Gruppe $-OSO_2OR^5$ stehen, mit einem m-(1H-Imidazol-4yl)-alkylamin der Formel III

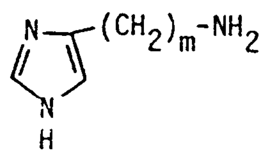

(III)

worin m den Wert 2 oder 3 hat, umsetzt oder
daß man

a2) ein Amin der allgemeinen Formel IV

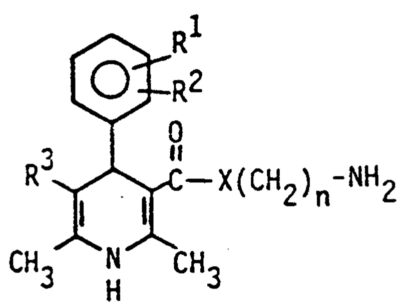

(IV)

worin $R^1$, $R^2$, $R^3$, X und n die oben angegebenen Bedeutungen haben, mit einer Verbindung der allgemeinen Formel V

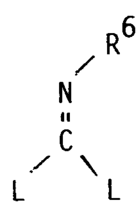

(V)

in der $R^6$ für eine Cyangruppe oder einen Benzoylrest steht und L eine Abgangsgruppe, wie $-SR^7$ oder $-OR^7$, bedeutet, wobei $R^7$ einen Alkyl- oder Arylrest darstellt, zu einer Zwischenverbindung der allgemeinen Formel VI

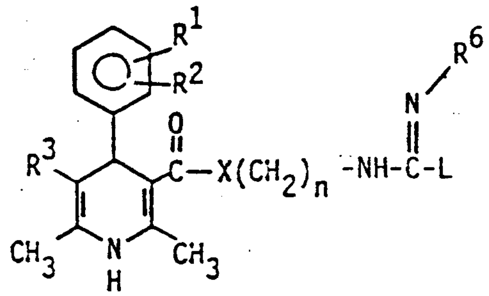

(VI)

worin $R^1$, $R^2$, $R^3$, $R^6$, X, L und n wie oben definiert sind, umsetzt, die erhaltene Zwischenverbindung der Formel VI mit einem m-(1H-Imidazol-4-yl)-alkylamin der Formel III

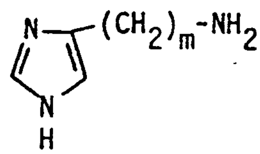

(III)

worin m den Wert 2 oder 3 hat, zu einer Verbindung der allgemeinen Formel VII

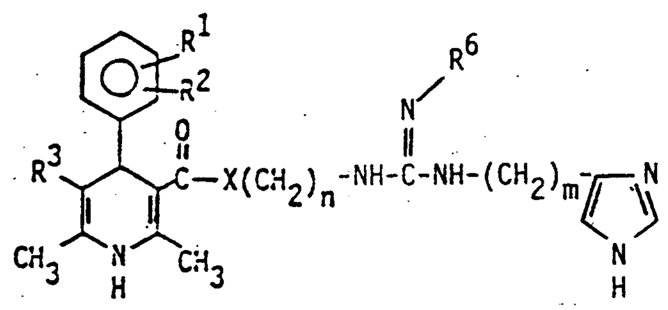

(VII)

worin $R^1$, $R^2$, $R^3$, $R^6$, X, m und n wie oben definiert sind, umsetzt und die Verbindung der allgemeinen Formel VII durch anschließende säurekatalysierte Hydrolyse zu einer Verbindung der allgemeinen Formel I umsetzt oder
daß man
a3) ein Amin der allgemeinen Formel IV

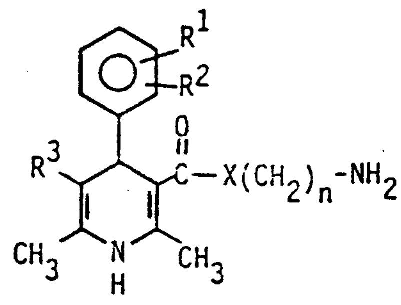

(IV)

worin $R^1$, $R^2$, $R^3$, X und n die oben angegebenen Bedeutungen haben, mit einer Verbindung der allgemeinen Formel VIII

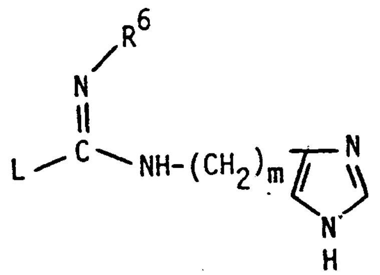

(VIII)

worin $R^6$ und L die oben angegebenen Bedeutungen haben und m den Wert 2 oder 3 hat, zu einer Verbindung der allgemeinen Formel VII

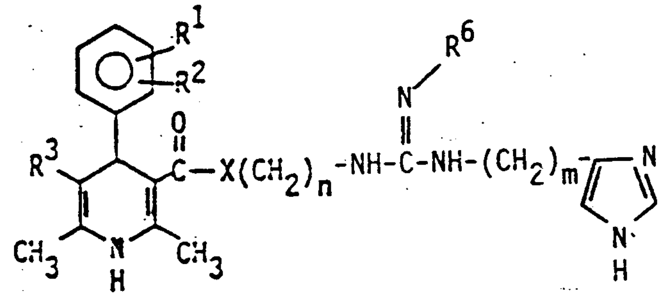

(VII)

worin $R^1$, $R^2$, $R^3$, $R^6$, X, m und n wie oben definiert sind, umsetzt und die erhaltene Verbindung der allgemeinen Formel VII zu einer Verbindung der allgemeinen Formel I hydrolysiert oder
daß man
a4) ein Amin der allgemeinen Formel IV, worin $R^1$, $R^2$, $R^3$, X und n die oben angegebenen Bedeutungen haben, mit einer Verbindung der allgemeinen Formel IX

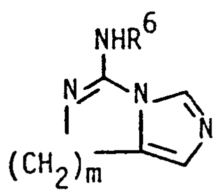

(IX)

worin $R^6$ und m wie oben definiert sind, zu einer Zwischenverbindung der allgemeinen Formel VII

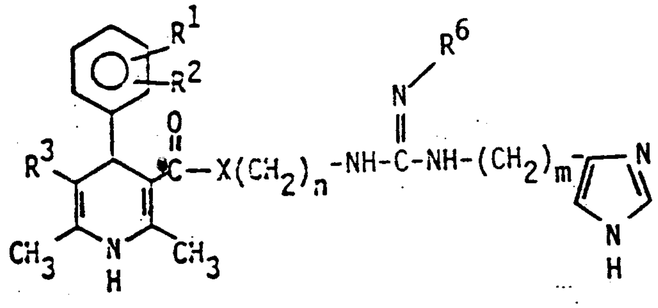

(VII)

worin $R^1$, $R^2$, $R^3$, $R^6$, X, m und n wie oben definiert sind, umsetzt und die so erhaltene Zwischenverbindung der Formel VII zu einer Verbindung der allgemeinen Formel I hydrolysiert oder
daß man

(b) zur Herstellung von Verbindungen der allgemeinen Formel I, bei denen $R^1$, $R^2$, $R^3$, X, m und n die oben angegebenen Bedeutungen haben und Y eine Bindung bedeutet, einen Imidoester der allgemeinen Formel X

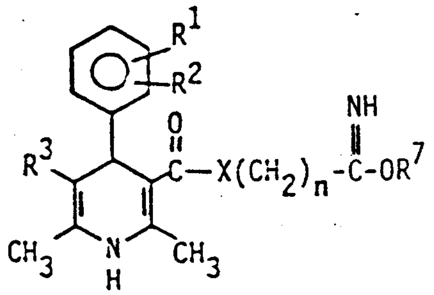

(X)

worin $R^1$, $R^2$, $R^3$, X und n die oben angegebenen Bedeutungen haben und $R^7$ für einen $C_1$-$C_4$-Alkylrest steht, mit einem m-(1H-Imidazol-4-yl)-alkylamin der Formel III

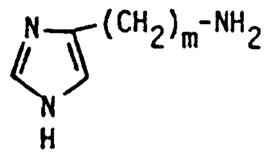

(III)

worin m den Wert 2 oder 3 hat, umsetzt
und gegebenenfalls die gemäß (a) oder (b) erhaltene Verbindung der allgemeinen Formel I in ihr physiologisch annehmbares Salz umwandelt.

**2.** Verfahren nach Anspruch 1 zur Herstellung von Verbindungen, die dadurch **gekennzeichnet** sind, daß $R^1$ und $R^2$ unabhängig voneinander für ein Wasserstoffatom, eine Methylgruppe, die gegebenenfalls durch ein Halogenatom substituiert ist, ein Halogenatom, eine Nitrogruppe oder eine Trifluormethylgruppe stehen, wobei die Substituenten $R^1$ und $R^2$ in 2- und/oder 3-Stellung des Phenylrings gebunden sind, oder $R^1$ und $R^2$ zusammen für einen 1,2,5-Oxadiazolrest stehen, der in Positionen 3 und 4 an den Phenylrest in den Positionen 2 und 3 ankondensiert ist,
$R^3$ für eine Nitrogruppe oder den Rest

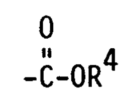

steht, worin $R^4$ eine gerad- oder verzweigtkettige $C_1$-$C_4$-Alkylgruppe bedeutet, die gegebenenfalls durch eine $C_1$-$C_4$-Alkoxygruppe substituiert ist,
X für ein Sauerstoffatom oder die Gruppe NH steht,
Y für die Gruppe NH oder eine Bindung steht,
n eine ganze Zahl von 1 bis 16 ist und
m den Wert 3 hat,
sowie die physiologisch annehmbaren Salze davon.

**3.** Verfahren nach Anspruch 1 zur Herstellung von Verbindungen, die dadurch **gekennzeichnet** sind, daß $R^1$ und $R^2$ unabhängig voneinander für ein Wasserstoffatom, ein Chloratom oder eine Nitrogruppe stehen, wobei die Substituenten $R^1$ und $R^2$ in 2- und/oder 3-Stellung des Phenylrings gebunden sind,
$R^3$ den Rest

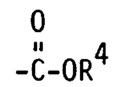

darstellt, worin $R^4$ für eine gerad- oder verzweigtkettige $C_1$-$C_4$-Alkylgruppe, die gegebenenfalls durch eine $C_1$-$C_4$-Rlkoxygruppe substituiert ist, steht,
X für ein Sauerstoffatom oder die Gruppe NH steht,
Y für die Gruppe NH oder eine Bindung steht,
n eine ganze Zahl von 2 bis 6 bedeutet und
m den Wert 3 hat,
sowie die physiologisch annehmbaren Salze davon.

**4.** Verfahren nach Anspruch 1 zur Herstellung von Verbindungen, die dadurch **gekennzeichnet** sind, daß $R^1$ für ein Wasserstoffatom steht, $R^2$ für eine in 2- oder 3-Position des Phenylrings gebundene Nitrogruppe steht,
$R^3$ für den Rest

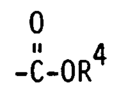

steht, worin $R^4$ eine gerad- oder verzweigtkettige $C_1$-$C_4$-Alkylgruppe ist, die gegebenenfalls durch eine $C_1$-$C_4$-Alkoxygruppe substituiert ist,
X für ein Sauerstoffatom steht,
Y für die Gruppe NH steht,
n eine ganze Zahl von 1 bis 16 bedeutet und
m den Wert 3 hat,
sowie die physiologisch annehmbaren Salze davon.

**Claims**
**Claims for the following Contracting States : BE, CH, DE, FR, GB, IT, LI, LU, NL, SE**

**1.** 1,4-dihydropyridine derivatives having the general formula I:

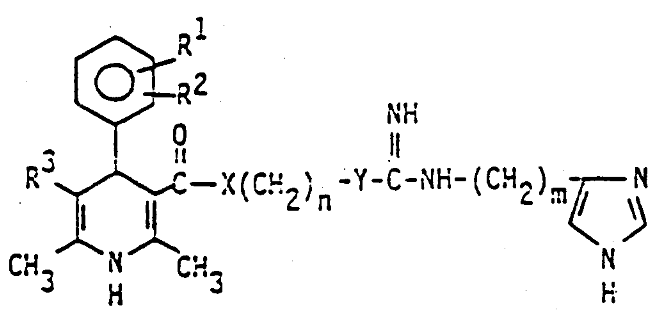

(I)

in which $R^1$ and $R^2$ independently denote a hydrogen atom, a methyl group optionally substituted by halogen, a halogen atom, a nitro group or a trifluoromethyl group or together stand for a 1,2,5-oxadiazole radical,
$R^3$ denotes a nitro group or a radical

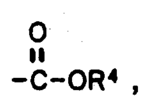

in which $R^4$ is a straight or branched-chain $C_1$-$C_4$ alkyl group, optionally substituted by a $C_1$-$C_4$ alkoxy group,
X stands for an oxygen atom or the group NH,
Y stands for the group NH or a bond,
n is an integer from 1 to 16 and
m has the value 2 or 3,
and physiologically acceptable salts thereof.

**2.** 1,4-dihydropyridine derivatives according to claim 1, characterised in that $R^1$ and $R^2$ independently stand for a hydrogen atom, a methyl group optionally substituted by a halogen atom, a halogen atom, a nitro group or a trifluoromethyl group, the substituents $R^1$ and $R^2$ being bonded in the 2 and/or 3 position of the phenyl ring, or $R^1$ and $R^2$ together stand for a 1,2,5-oxadiazole radical condensed in positions 3 and 4 on to the phenyl radical in positions 2 and 3,
$R^3$ stands for a nitro group or a radical

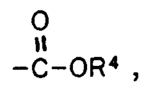

in which $R^4$ denotes a straight or branched-chain $C_1$-$C_4$-alkyl group, optionally substituted by a $C_1$-$C_4$ alkoxy group,
X stands for a hydrogen atom or the group NH,
Y stands for the group NH or a bond,
n is an integer from 1 to 16, and
m is equal to 3,
and physiologically acceptable salts thereof.

**3.** 1,4-dihydropyridine derivatives according to claim 1, characterised in that $R^1$ and $R^2$ independently stand for a hydrogen atom, a chlorine atom or a nitro group, the substituents $R^1$ and $R^2$ being bonded in the 2 and/or 3 position of the phenyl ring,
$R^3$ denotes the radical

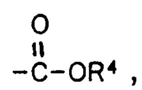

in which $R^4$ stands for a straight or branched-chain $C_1$-$C_4$ alkyl group, optionally substituted by a $C_1$-$C_4$ alkoxy group,
X stands for an oxygen atom or the group NH,
Y stands for the group NH or a bond,
n is an integer from 2 to 6 and
m is equal to 3,
and physiologically acceptable salts thereof.

**4.** 1,4-dihydropyridine derivatives according to claim 1, characterised in that $R^1$ stands for a hydrogen atom, $R^2$ for a nitro group bonded in the 2 or 3 position of the phenyl ring, $R^3$ stands for the radical

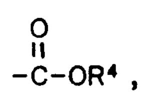

in which $R^4$ denotes a straight or branched-chain $C_1$-$C_4$-alkyl group optionally substituted by a $C_1$-$C_4$ alkoxy group,
X stands for an oxygen atom,
Y stands for the NH group,

n is an integer from 1 to 16 and
m is 3,
and physiologically acceptable salts thereof.

**5.** A method of preparing the 1,4-dihydropyridine derivatives according to claims 1 to 4 and the physiologically acceptable salts thereof, characterised in that

(a) in order to obtain compounds having the general formula I in which $R^1$, $R^2$, $R^3$, X, m and n are defined as in claim 1 and Y stands for NH

a1) an isothiuronium salt having the general formula II

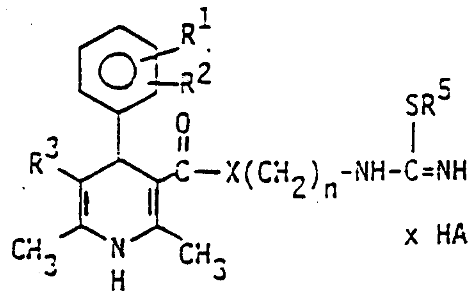

(II)

in which $R^1$, $R^2$, $R^3$, X and n are defined as in claim 1, $R^5$ stands for an optionally substituted alkyl radical and A for a halogen atom or the group $-OSO_2OR^5$, is reacted with an m-(1H-imidazol-4-yl)-alkyl amine having the formula III

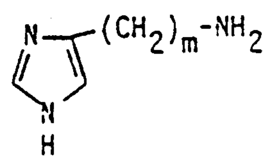

(III)

where m is equal to 2 or 3, or

a2) an amine having the general formula IV

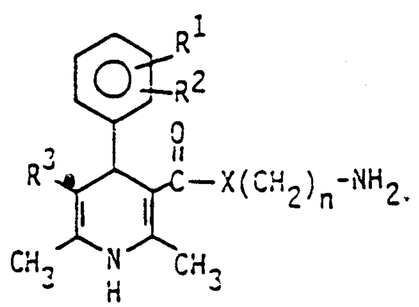

(IV)

where $R^1$, $R^2$, $R^3$, X and n are as defined in claim 1, is reacted with a compound having the general formula V

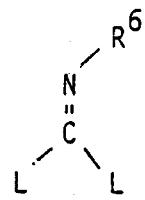

(V)

in which $R^6$ stands for a cyano group or a benzoyl radical and L denotes a starting group such as $-SR^7$ or $-OR^7$, where $R^7$ is an alkyl or aryl radical, is converted into an intermediate compound having the general formula VI

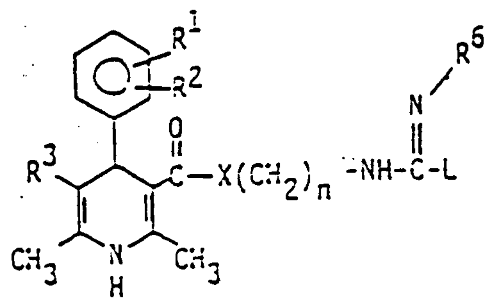

(VI)

where $R^1$, $R^2$, $R^3$, $R^6$, X, L and n are as defined hereinbefore, the resulting formula VI intermediate is reacted with an m-(1H-Imidazol-4-yl)-alkyl amine having the formula III

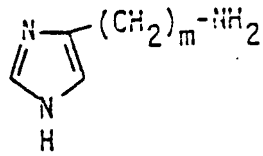

(III)

in which m is 2 or 3, to obtain a compound having the general formula VII

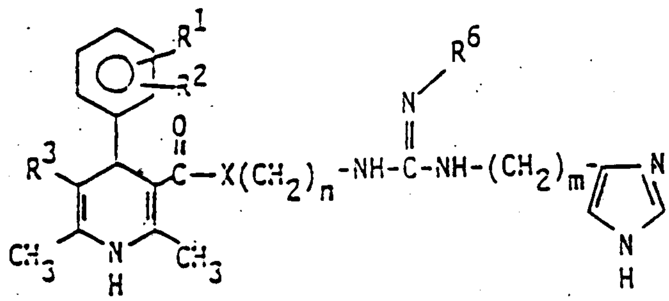

(VII)

where $R^1$, $R^2$, $R^3$, $R^6$, X, m and n are defined as hereinbefore, and the compound having the general formula VII is then reacted by acid-catalysed hydrolysis to form a compound having the general formula I or

a3) an amine having the general formula IV

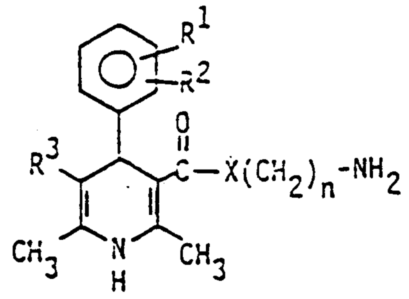

(IV)

where $R^1$, $R^2$, $R^3$, X and n are as defined in claim 1, is reacted with a compound having the general formula VIII

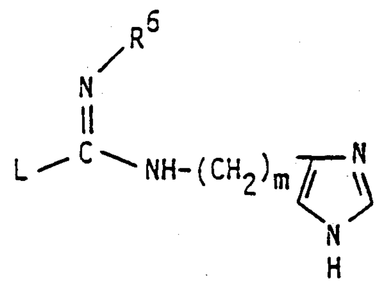

(VIII)

where $R^6$ and L have the meanings given previously and m is equal to 2 or 3, to obtain a compound having the general formula VII

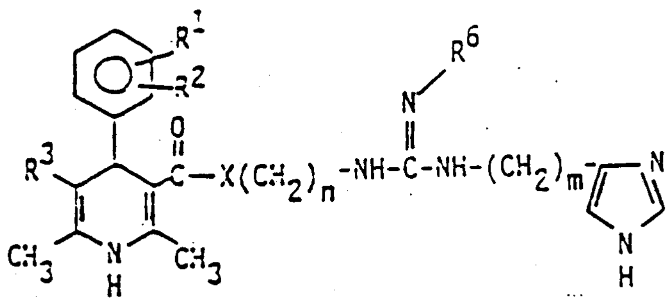

(VII)

where $R^1$, $R^2$, $R^3$ ,$R^6$, X, m and n are as defined hereinbefore, and the resulting compound having the general formula VII is hydrolysed to obtain a compound having the general formula I or
a4) an amine having the general formula IV, where $R^1$, $R^2$, $R^3$, X and n are as defined in claim 1, is reacted with a compound having the general formula IX

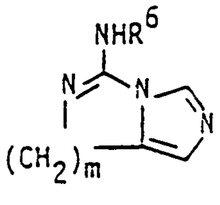

(IX)

where $R^6$ and m are as defined hereinbefore, to obtain an intermediate compound having the general formula VII

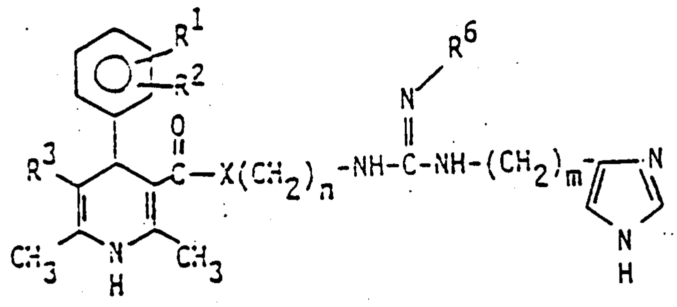

(VII)

where $R^1$, $R^2$, $R^3$, $R^6$, X, m and n are as defined hereinbefore, and the resulting formula VII intermediate compound is hydrolysed to a compound having the general formula I or

(b) in order to prepare compounds having the general formula I in which $R^1$, $R^2$, $R^3$, X, m and n are as defined as in claim 1 and Y denotes a bond, an imido ester having the general formula X

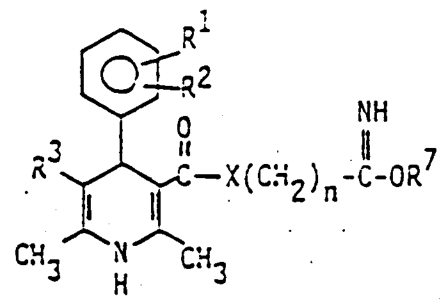

(X)

where $R^1$, $R^2$, $R^3$, X and n are as defined in claim 1 and $R^7$ stands for a $C_1$-$C_4$ alkyl radical, is reacted with an m-(1H-Imidazol-4-yl)-alkyl amine having the formula III

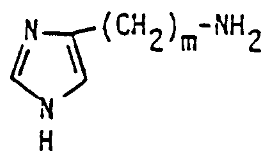

(III)

where m is equal to 2 or 3, and optionally the compound having the general formula I and obtained as per (a) or (b) is converted into its physiologically acceptable salt.

**6.** A drug characterised in that it contains a compound according to any of claims 1 to 4, together with an inert pharmaceutically acceptable excipient or diluent.

**Claims for the following Contracting States : AT, ES, GR**

**1.** A method of preparing 1,4-dihydropyridine derivatives having the general formula I:

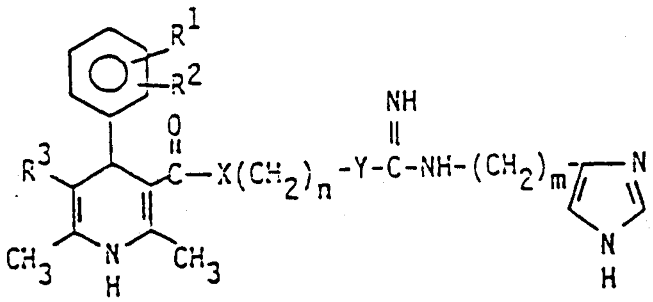

(I)

in which $R^1$ and $R^2$ independently denote a hydrogen atom, a methyl group optionally substituted by halogen, a halogen atom, a nitro group or a trifluoromethyl group or together stand for a 1,2,5-oxadiazole radical,
$R^3$ denotes a nitro group or a radical

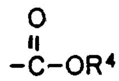 ,

in which $R^4$ is a straight or branched-chain $C_1$-$C_4$ alkyl group, optionally substituted by a $C_1$-$C_4$ alkoxy group,
X stands for an oxygen atom or the group NH,
Y stands for the group NH or a bond,
n is an integer from 1 to 16 and
m has the value 2 or 3,
and physiologically acceptable salts thereof,
characterised in that

(a) in order to obtain compounds having the general formula I in which $R^1$, $R^2$, $R^3$, X, m and n are defined as in claim 1 and Y stands for NH

a1) an isothiuronium salt having the general formula II

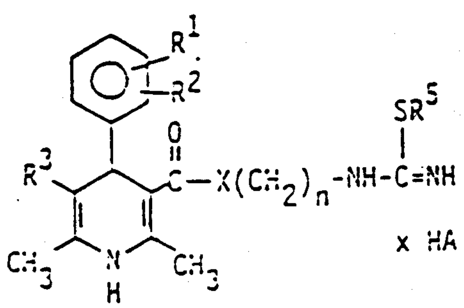

(II)

in which $R^1$, $R^2$, $R^3$, X and n have the meanings given hereinbefore, $R^5$ stands for an optionally substituted alkyl radical and A for a halogen atom or the group $-OSO_2OR^5$, is reacted with an m-(1H-imidazol-4-yl)-alkyl amine having the formula III

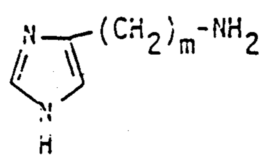

(III)

where m is equal to 2 or 3, or

a2) an amine having the general formula IV

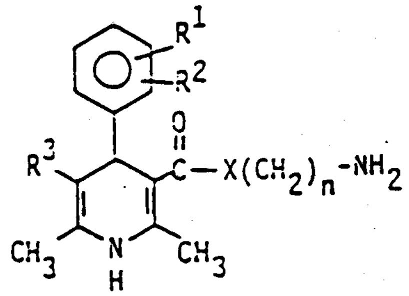

(IV)

where $R^1$, $R^2$, $R^3$, X and n have the meanings given hereinbefore, is reacted with a compound having the general formula V

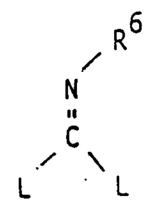

(V)

in which $R^6$ stands for a cyano group or a benzoyl radical and L denotes a starting group such as $-SR^7$ or $-OR^7$, where $R^7$ is an alkyl or aryl radical, is converted into an intermediate compound having the general formula VI

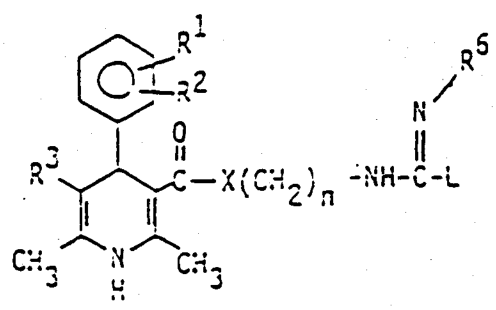

(VI)

where $R^1$, $R^2$, $R^3$, $R^6$, X, L and n are as defined hereinbefore, the resulting formula VI intermediate is reacted with an m-(1H-imidazol-4-yl)-alkyl amine having the formula III

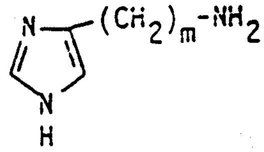

(III)

in which m is 2 or 3, to obtain a compound having the general formula VII

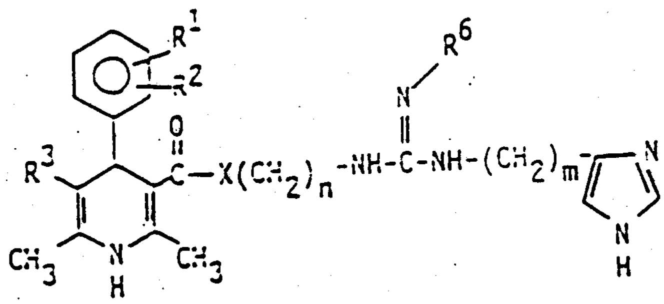

(VII)

where $R^1$, $R^2$, $R^3$, $R^6$, X, m and n are defined as hereinbefore, and the compound having the general formula VII is then reacted by acid-catalysed hydrolysis to form a compound having the general formula I or

a3) an amine having the general formula IV

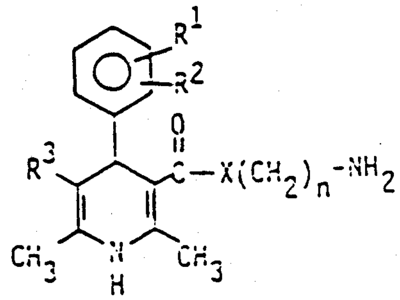

(IV)

where $R^1$, $R^2$, $R^3$, X and n have the meanings given hereinbefore, is reacted with a compound having the general formula VIII

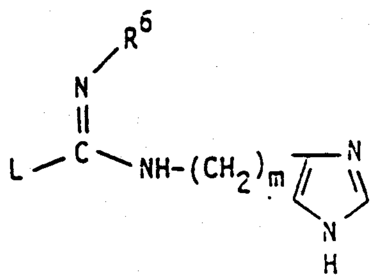

(VIII)

where $R^6$ and L have the meanings given previously and m is equal to 2 or 3, to obtain a compound having the general formula VII

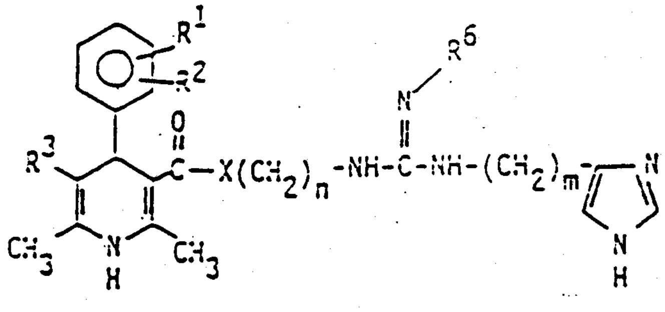

(VII)

where $R^1$, $R^2$, $R^3$, $R^6$, X, m and n have the meanings given hereinbefore, and the resulting compound having the general formula VII is hydrolysed to obtain a compound having the general formula I or

a4) an amine having the general formula IV, where $R^1$, $R^2$, $R^3$, X and n have the meanings given hereinbefore, is reacted with a compound having the general formula IX

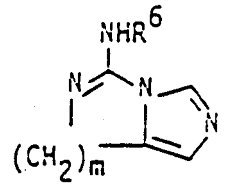

(IX)

where $R^6$ and m are as defined hereinbefore, to obtain an intermediate having the general formula VII

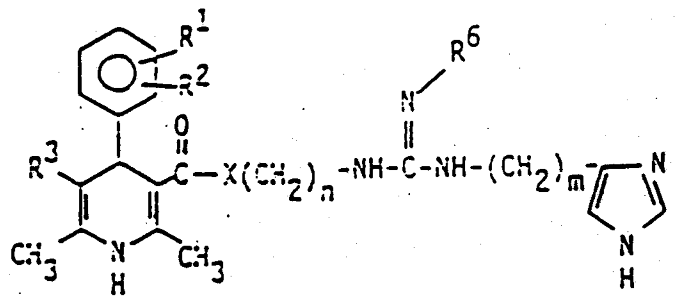

(VII)

where $R^1$, $R^2$, $R^3$, $R^6$, X, m and n are as defined hereinbefore, and the resulting formula VII intermediate compound is hydrolysed to a compound having the general formula I or

(b) in order to prepare compounds having the general formula I in which $R^1$, $R^2$, $R^3$, X, m and n have the meanings given hereinbefore, and Y denotes a bond, an imido ester having the general formula X

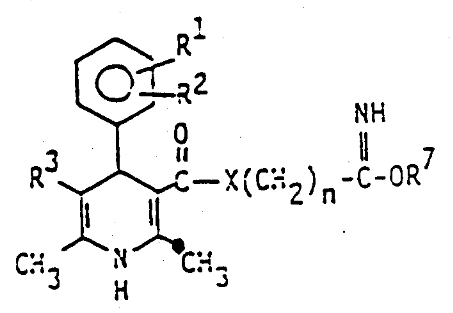

(X)

where $R^1$, $R^2$, $R^3$, X and n are as defined in claim 1 and $R^7$ stands for a $C_1$-$C_4$ alkyl radical, is reacted with an m-(1H-Imidazol-4-yl)-alkyl amine having the formula III

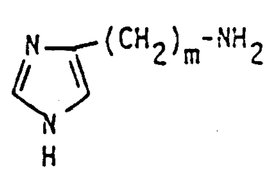

(III)

where m is equal to 2 or 3, and optionally the compound having the general formula I and obtained

as per (a) or (b) is converted into its physiologically acceptable salt.

**2.** A method according to claim 1 for preparing compounds, characterised in that
$R^1$ and $R^2$ independently stand for a hydrogen atom, a methyl group optionally substituted by a halogen atom, a halogen atom, a nitro group or a trifluoromethyl group, the substituents $R^1$ and $R^2$ being bonded in the 2 and/or 3 position of the phenyl ring, or $R^1$ and $R^2$ together stand for a 1,2,5-oxadiazole radical which is condensed in positions 3 and 4 on to the phenyl radical in positions 2 and 3,
$R^3$ stands for a nitro group or a radical

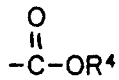 ,

in which $R^4$ denotes a straight or branched-chain $C_1$-$C_4$-alkyl group, optionally substituted by a $C_1$-$C_4$ alkoxy group,
X stands for a hydrogen atom or the group NH,
Y stands for the group NH or a bond,
n is an integer from 1 to 16, and
m is equal to 3,
and physiologically acceptable salts thereof.

**3.** A method according to claim 1 for preparing compounds, characterised in that $R^1$ and $R^2$ independently stand for a hydrogen atom, a chlorine atom or a nitro group, the substituents $R^1$ and $R^2$ being bonded in the 2 and/or 3 position of the phenyl ring,
$R^3$ denotes the radical

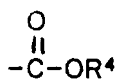 ,

in which $R^4$ stands for a straight or branched-chain $C_1$-$C_4$ alkyl group, optionally substituted by a $C_1$-$C_4$ alkoxy group,
X stands for an oxygen atom or the group NH,
Y stands for the group NH or a bond,
n is an integer from 2 to 6 and
m is equal to 3,
and physiologically acceptable salts thereof.

**4.** A method according to claim 1 for preparing compounds, characterised in that $R^1$ stands for a hydrogen atom, $R^2$ for a nitro group bonded in the 2 or 3 position of the phenyl ring, $R^3$ stands for the radical

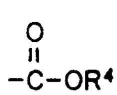 ,

in which $R^4$ denotes a straight or branched-chain $C_1$-$C_4$-alkyl group optionally substituted by a $C_1$-$C_4$ alkoxy group,
X stands for an oxygen atom,
Y stands for the NH group,
n is an integer from 1 to 16 and
m is 3,
and physiologically acceptable salts thereof.

**Revendications**

**Revendications pour les Etats contractants suivants : BE, CH, DE, FR, GB, IT, LI, LU, NL, SE**

**1.** Dérivés de 1,4-dihydropyridine de formule générale I

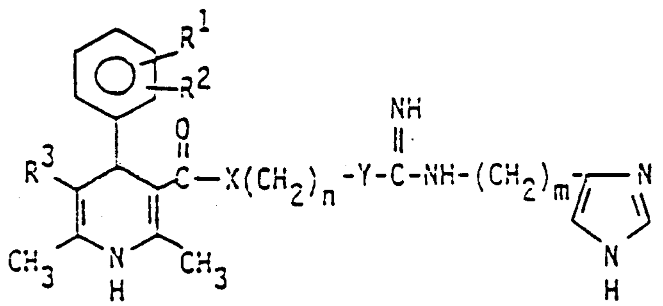

(I)

dans laquelle $R^1$ et $R^2$ représentent indépendamment l'un de l'autre un atome d'hydrogène, un groupe méthyle éventuellement halogéno substitué, un atome d'halogène, un groupe nitro ou un groupe trifluorométhyle ou bien $R^1$ et $R^2$ pris ensemble représentent un reste 1,2,5-oxadiazole,
$R^3$ représente un groupe nitro ou le reste

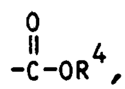

dans lequel $R^4$ est un groupe alkyle à chaîne droite ou ramifiée en $C_1$-$C_4$, éventuellement substitué par un groupe alcoxy en $C_1$-$C_4$,
X représente un atome d'oxygène ou le groupe NH,
Y représente le groupe NH ou une liaison,
n représente un nombre entier de 1 à 16 et
m est égal à 2 ou 3,
et leurs sels physiologiquement acceptables.

**2.** Dérivés de 1,4-dihydropyridine selon la revendication 1, caractérisés en ce que $R^1$ et $R^2$ représentent indépendamment l'un de l'autre un atome d'hydrogène, un groupe méthyle, qui est éventuellement substitué par un atome d'halogène, un atome d'halogène, un groupe nitro ou un groupe trifluorométhyle, les substituants $R^1$ et $R^2$ étant fixés en position 2 et/ou en position 3 du noyau phényle, ou bien $R^1$ et $R^2$ pris ensemble représentent un reste 1,2,5-oxadiazole, qui est condensé par ses positions 3 et 4 au reste phényle dans les positions 2 et 3,
$R^3$ représente un groupe nitro ou le reste

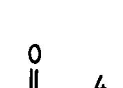

dans lequel $R^4$ représente un groupe alkyle à chaîne droite ou ramifiée en $C_1$-$C_4$, qui est éventuellement substitué par un groupe alcoxy en $C_1$-$C_4$,
X représente un atome d'oxygène ou le groupe NH,
Y représente le groupe NH ou une liaison,
n est un entier de 1 à 16 et
m est égal à 3,
et leurs sels physiologiquement acceptables.

**3.** Dérivés de 1,4-dihydropyridine selon la revendication 1, caractérisés en ce que $R^1$ et $R^2$ représentent

indépendamment l'un de l'autre un atome d'hydrogène, un atome de chlore ou un groupe nitro, les substituants $R^1$ et $R^2$ étant reliés en position 2 et/ou en position 3 du noyau phényle,
$R^3$ représente le reste

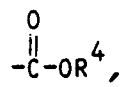

dans lequel $R^4$ représente un groupe alkyle à chaîne droite ou ramifiée en $C_1$-$C_4$ qui est éventuellement substitué par un groupe alcoxy en $C_1$-$C_4$,
X représente un atome d'oxygène ou le groupe NH,
Y représente le groupe NH ou une liaison,
n représente un nombre entier de 2 à 6 et
m est égal à 3,
et leurs sels physiologiquement acceptables.

**4.** Dérivés de 1,4-dihydropyridine selon la revendication 1, caractérisés en ce que $R^1$ représente un atome d'hydrogène, $R^2$ représente un groupe nitro relié en position 2 ou 3 du noyau phényle, $R^3$ représente le reste

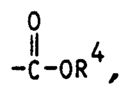

dans lequel $R^4$ représente un groupe alkyle à chaîne droite ou ramifiée en $C_1$-$C_4$ qui est éventuellement substitué par un groupe alcoxy en $C_1$-$C_4$,
X représente un atome d'oxygène,
Y représente le groupe NH,
n représente un nombre entier de 1 à 16 et
m est égal à 3,
et leurs sels physiologiquement acceptables.

**5.** Procédé pour la fabrication des dérivés de 1,4-dihydropyridine selon les revendications 1 à 4 et de leurs sels physiologiquement acceptables, caractérisé en ce que
(a) pour la fabrication de composés de formule I, dans lesquels $R^1$, $R^2$, $R^3$, X, m et n sont définis comme à la revendication 1 et Y représente NH,
a1) on fait réagir un sel d'isothiuronium de formule générale II

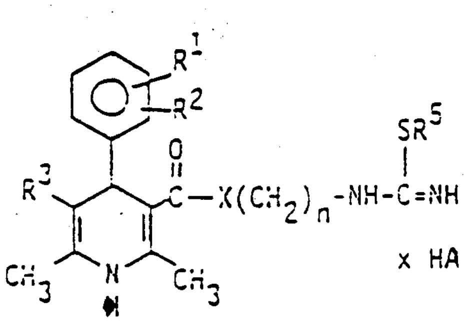

(II)

dans laquelle $R^1$, $R^2$, $R^3$, X et n sont définis comme à la revendication 1, $R^5$ représente un reste alkyle éventuellement substitué et A représente un atome d'halogène ou le groupe $-OSO_2OR^5$, avec une m-(1H-imidazole-4-yl)-alkylamine de formule III

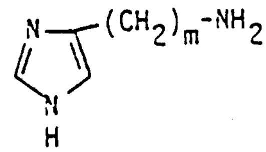

(III)

dans laquelle m est égal à 2 ou 3, ou bien en ce que a2) on fait réagir une amine de formule générale IV

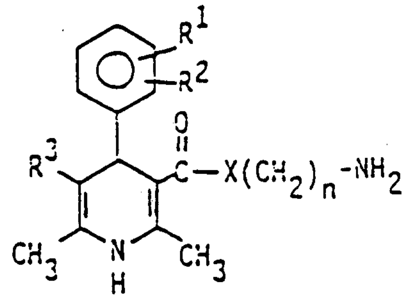

(IV)

dans laquelle $R^1$, $R^2$, $R^3$, X et n sont définis comme à la revendication 1, avec un composé de formule générale V

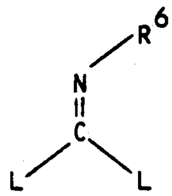

(V)

dans laquelle $R^6$ représente un groupe cyano ou un reste benzoyle et L représente un groupe éliminable tel que $-SR^7$ ou $-OR^7$, dans lesquels $R^7$ représente un reste alkyle ou un reste aryle, en un composé intermédiaire de formule générale VI

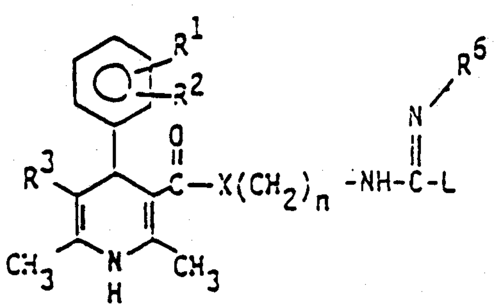

(VI)

dans laquelle $R^1$, $R^2$, $R^3$, $R^6$, X, L et n sont définis comme ci-dessus,
on fait réagir le composé intermédiaire de formule VI obtenu avec une m-(1H-imidazole-4-yl)-alkylamine de formule III

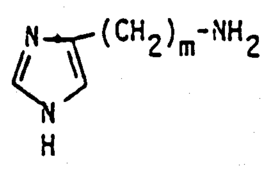

(III)

dans laquelle m est égal à 2 ou 3, en un composé de formule générale VII

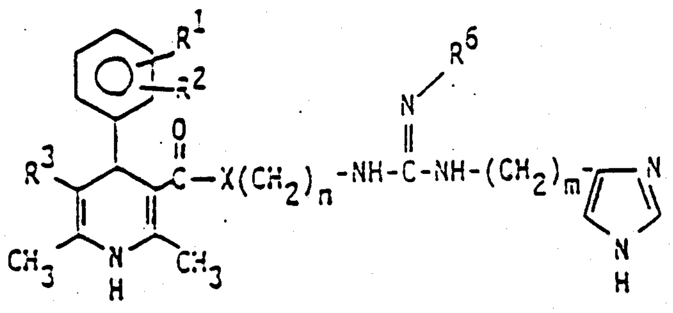

(VII)

dans laquelle $R^1$, $R^2$, $R^3$, $R^6$, X, m et n sont définis comme ci-dessus, et on fait ensuite réagir le composé de formule générale VII par hydrolyse par catalyse acide en un composé de formule générale I, ou bien

en ce que

a3) on fait réagir une amine de formule générale IV

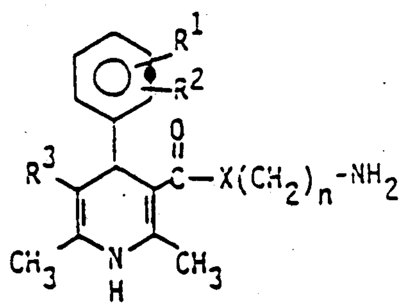

(IV)

dans laquelle $R^1$, $R^2$, $R^3$, X et n sont définis comme à la revendication 1 avec un composé de formule générale VIII

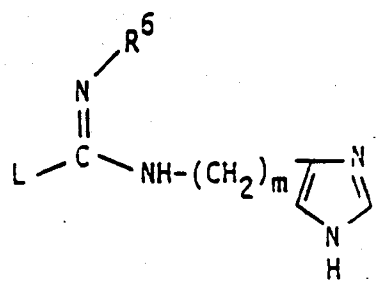

(VIII)

dans laquelle $R^6$ et L ont les significations indiquées ci-dessus et m est égal à 2 ou 3, en un composé de formule générale VII

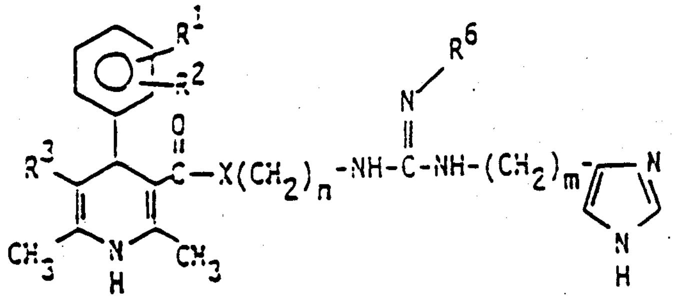 (VII)

dans laquelle $R^1$, $R^2$, $R^3$, $R^6$, X, m et n sont définis comme ci-dessus et on hydrolyse le composé de formule générale VII obtenu en un composé de formule générale I,ou bien
en ce que
a4) on fait réagir une amine de formule générale IV dans laquelle $R^1$, $R^2$, $R^3$, X et n sont définis comme à la revendication 1 avec un composé de formule générale IX

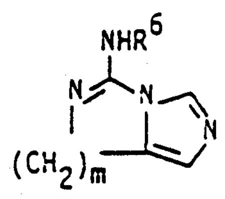 (IX)

dans laquelle $R^6$ et m sont définis comme ci-dessus en un composé intermédiaire de formule générale VII

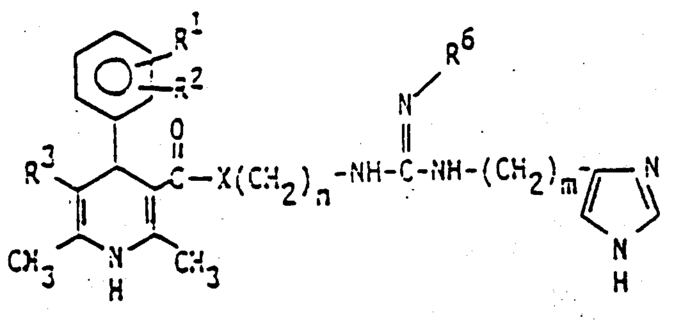 (VII)

dans laquelle $R^1$, $R^2$, $R^3$, $R^6$, X, m et n sont définis comme ci-dessus et on hydrolyse le composé intermédiaire de formule VII obtenu en un composé de formule générale I, ou bien
en ce que
(b) pour la fabrication de composés de formule générale I dans lesquels $R^1$, $R^2$, $R^3$, X, m et n sont définis comme à la revendication 1 et Y représente une liaison, on fait réagir un imidoester de formule générale X

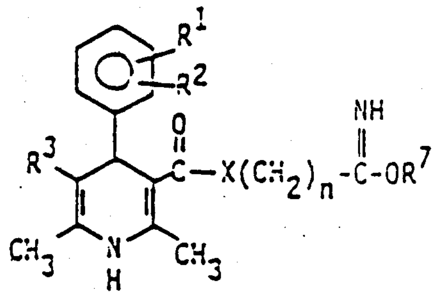

(X)

dans laquelle $R^1$, $R^2$, $R^3$, X et n sont définis comme à la revendication 1 et $R^7$ représente un reste alkyle en $C_1$-$C_4$ avec une m-(1H-imidazole-4-yl)-alkylamine de formule III

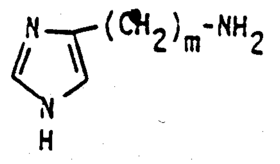

(III)

dans laquelle a est égal à 2 ou 3,

et on transforme éventuellement le composé de formule générale I obtenu selon (a) ou (b) en son sel physiologiquement acceptable.

**6.** Médicament, caractérisé en ce qu'il contient un composé selon une des revendications 1 à 4 avec un support ou diluant inerte pharmaceutiquement acceptable.

**Revendications pour les Etats contractants suivants : AT, ES, GR**

**1.** Procédé pour la fabrication de dérivés de 1,4-dihydropyridine de formule générale I

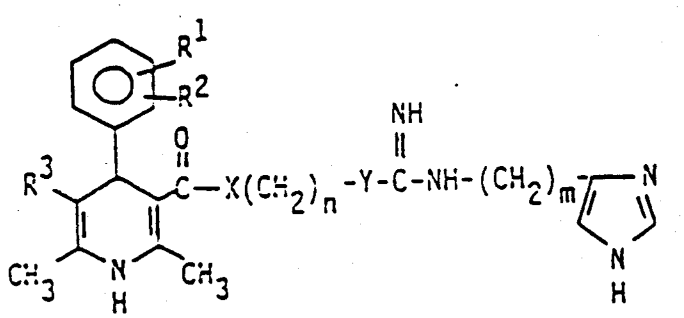

(I)

dans laquelle $R^1$ et $R^2$ représentent indépendamment l'un de l'autre un atome d'hydrogène, un groupe méthyle éventuellement halogéno substitué, un atome d'halogène, un groupe nitro ou un groupe trifluorométhyle ou bien $R^1$ et $R^2$ pris ensemble représentent un reste 1,2,5-oxadiazole,

$R^3$ représente un groupe nitro ou le reste

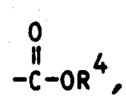

dans lequel $R^4$ est un groupe alkyle à chaîne droite ou ramifiée en $C_1$-$C_4$, éventuellement substitué par

un groupe alcoxy en $C_1$-$C_4$,
X représente un atome d'oxygène ou le groupe NH,
Y représente le groupe NH ou une liaison,
n représente un nombre entier de 1 à 16 et
m est égal à 2 ou 3,
et leurs sels pysiologiquement acceptables, caractérisés en ce que

(a) pour la fabrication de composés de formule I, dans lesquels $R^1$, $R^2$, $R^3$, X, m et n sont définis comme à la revendication 1 et Y représente NH,

a1) on fait réagir un sel d'isothiuronium de formule générale II

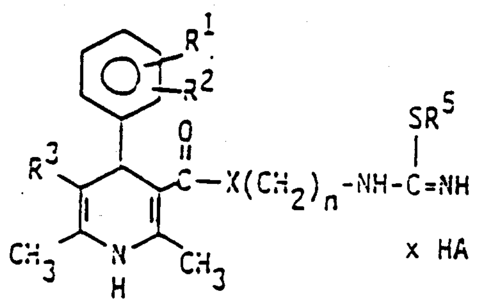

(II)

dans laquelle $R^1$, $R^2$, $R^3$, X et n sont définis comme à la revendication 1, $R^5$ représente un reste alkyle éventuellement substitué et A représente un atome d'halogène ou le groupe $-OSO_2OR^5$, avec une m-(1H-imidazole-4-yl)-alkylamine de formule III

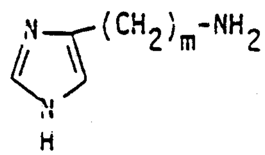

(III)

dans laquelle m est égal à 2 ou 3, ou bien en ce que

a2) on fait réagir une amine de formule générale IV

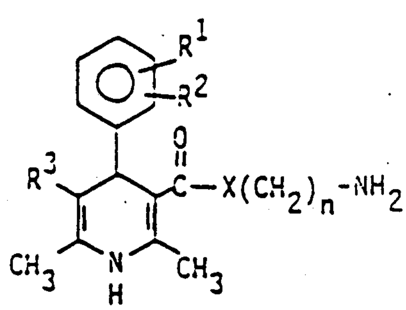

(IV)

dans laquelle $R^1$, $R^2$, $R^3$, X et n sont définis comme à la revendication 1, avec un composé de formule générale V

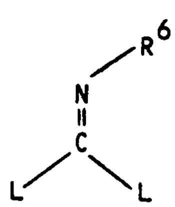

(V)

dans laquelle $R^6$ représente un groupe cyano ou un reste benzoyle et L représente un groupe éliminable tel que $-SR^7$ ou $-OR^7$, dans lesquels $R^7$ représente un reste alkyle ou un reste aryle, en un composé intermédiaire de formule générale VI

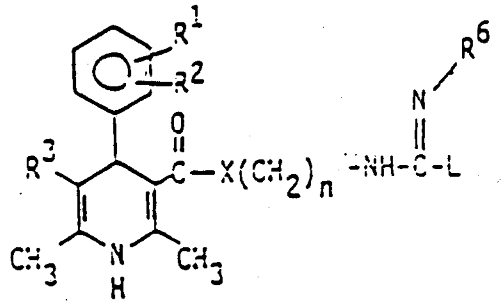

(VI)

dans laquelle $R^1$, $R^2$, $R^3$, $R^6$, X, L et n sont définis comme ci-dessus,
on fait réagir le composé intermédiaire de formule VI obtenu avec une m-(1H-imidazole-4-yl)-alkylamine de formule III

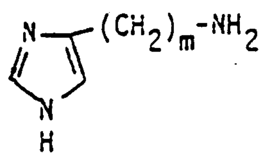

(III)

dans laquelle m est égal à 2 ou 3, en un composé de formule générale VII

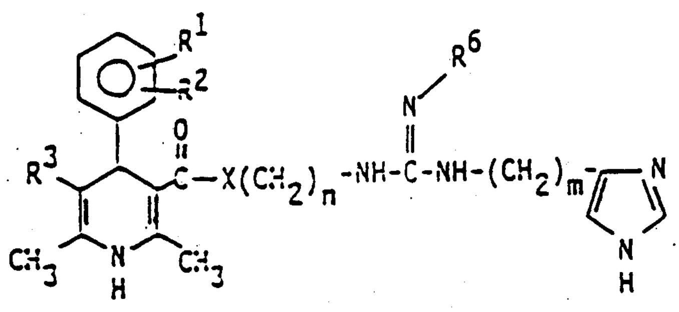

(VII)

dans laquelle $R^1$, $R^2$, $R^3$, $R^6$, X, m et n sont définis comme ci-dessus, et on fait ensuite réagir le composé de formule générale VII par hydrolyse par catalyse acide en un composé de formule générale I, ou bien
en ce que
a3) on fait réagir une amine de formule générale IV

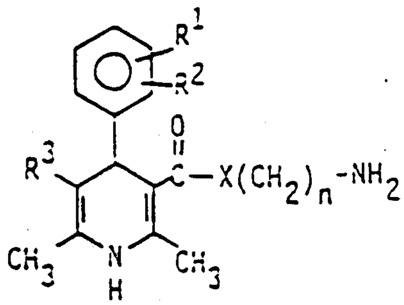

(IV)

dans laquelle $R^1$, $R^2$, $R^3$, X et n sont définis comme à la revendication 1 avec un composé de formule générale VIII

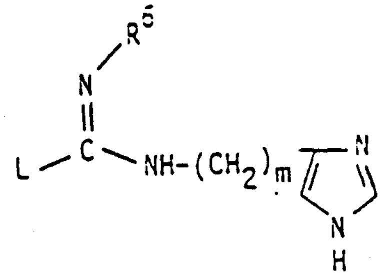

(VIII)

dans laquelle $R^6$ et L ont les significations indiquées ci-dessus et m est égal à 2 ou 3, en un composé de formule générale VII

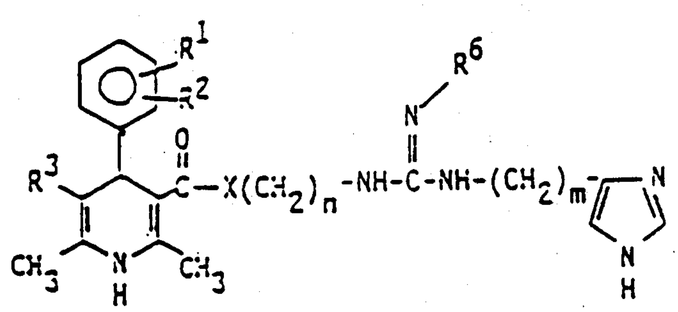

(VII)

dans laquelle $R^1$, $R^2$, $R^3$, $R^6$, X, m et n sont définis comme ci-dessus et on hydrolyse le composé de formule générale VII obtenu en un composé de formule générale I, ou bien
en ce que
a4) on fait réagir une amine de formule générale IV dans laquelle $R^1$, $R^2$, $R^3$, X et n sont définis comme à la revendication 1 avec un composé de formule générale IX

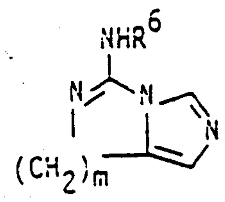

(IX)

dans laquelle $R^6$ et m sont définis comme ci-dessus en un composé intermédiaire de formule générale VII

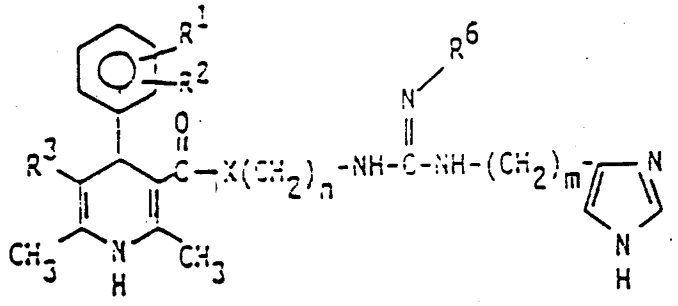

(VII)

dans laquelle $R^1$, $R^2$, $R^3$, $R^6$, X, m et n sont définis comme ci-dessus et on hydrolyse le composé intermédiaire de formule VII obtenu en un composé de formule générale I, ou bien
en ce que
(b) pour la fabrication de composés de formule générale I dans lesquels $R^1$, $R^2$, $R^3$, X, m et n sont définis comme à la revendication 1 et Y représente une liaison, on fait réagir un imidoester de formule générale X

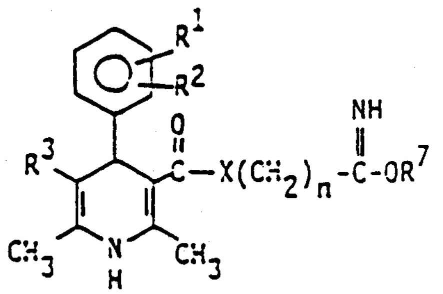

(X)

dans laquelle $R^1$, $R^2$, $R^3$, X et n sont définis comme à la revendication 1 et $R^7$ représente un reste alkyle en $C_1$-$C_4$ avec une m-(1H-imidazole-4-yl)-alkylamine de formule III

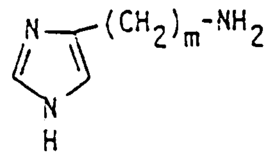

(III)

dans laquelle m est égal à 2 ou 3,
et on transforme éventuellement le composé de formule générale I obtenu selon (a) ou (b) en son sel physiologiquement acceptable.

2. Procédé selon la revendication 1 pour la fabrication de composés qui sont caractérisés en ce que $R^1$ et $R^2$ représentent indépendamment l'un de l'autre un atome d'hydrogène, un groupe méthyle, qui est éventuellement substitué par un atome d'halogène, un atome d'halogène, un groupe nitro ou un groupe trifluorométhyle, les substituants $R^1$ et $R^2$ étant fixés en position 2 et/ou en position 3 du noyau phényle, ou bien $R^1$ et $R^2$ pris ensemble représentent un reste 1,2,5-oxadiazole, qui est condensé par ses positions 3 et 4 au reste phényle dans les positions 2 et 3,
$R^3$ représente un groupe nitro ou le reste

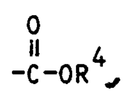

dans lequel $R^4$ représente un groupe alkyle à chaîne droite ou ramifiée en $C_1$-$C_4$, qui est éventuellement substitué par un groupe alcoxy en $C_1$-$C_4$,
X représente un atome d'oxygène ou le groupe NH,
Y représente le groupe NH ou une liaison,
n est un entier de 1 à 16 et
m est égal à 3,
et leurs sels physiologiquement acceptables.

3. Procédé selon la revendication 1 pour la fabrication de composés qui sont caractérisés en ce que $R^1$ et $R^2$ représentent indépendamment l'un de l'autre un atome d'hydrogène, un atome de chlore ou un groupe nitro, les substituants $R^1$ et $R^2$ étant reliés en position 2 et/ou en position 3 du noyau phényle,
$R^3$ représente le reste

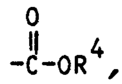

dans lequel $R^4$ représente un groupe alkyle à chaîne droite ou ramifiée en $C_1$-$C_4$ qui est éventuellement substitué par un groupe alcoxy en $C_1$-$C_4$,
X représente un atome d'oxygène ou le groupe NH,
Y représente le groupe NH ou une liaison,
n représente un nombre entier de 2 à 6 et
m est égal à 3,
et leurs sels physiologiquement acceptables.

**4.** Procédé selon la revendication 1 pour la fabrication de composés qui sont caractérisés en ce que $R^1$ représente un atome d'hydrogène, $R^2$ représente un groupe nitro relié en position 2 ou 3 du noyau phényle, $R^3$ représente le reste

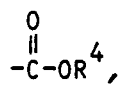

dans lequel $R^4$ représente un groupe alkyle à chaîne droite ou ramifiée en $C_1$-$C_4$ qui est éventuellement substitué par un groupe alcoxy en $C_1$-$C_4$,
X représente un atome d'oxygène,
Y représente le groupe NH,
n représente un nombre entier de 1 à 16 et
m est égal à 3,
et leurs sels physiologiquement acceptables.